(19) Europäisches Patentamt
European Patent Office
Office européen des brevets

(11) EP 2 456 783 B1

(12) **EUROPÄISCHE PATENTSCHRIFT**

(45) Veröffentlichungstag und Bekanntmachung des Hinweises auf die Patenterteilung:
**19.08.2015 Patentblatt 2015/34**

(21) Anmeldenummer: **10732868.4**

(22) Anmeldetag: **08.07.2010**

(51) Int Cl.:
***C07J 21/00*** $^{(2006.01)}$ ***C07J 41/00*** $^{(2006.01)}$
***C07J 43/00*** $^{(2006.01)}$ ***C07J 1/00*** $^{(2006.01)}$

(86) Internationale Anmeldenummer:
**PCT/EP2010/004156**

(87) Internationale Veröffentlichungsnummer:
**WO 2011/009533 (27.01.2011 Gazette 2011/04)**

(54) **17-HYDROXY-17-PENTAFLUORETHYL-ESTRA-4,9(10) -DIEN-11-ETHINYLPHENYL-DERIVATE, VERFAHREN ZU DEREN HERSTELLUNG UND DEREN VERWENDUNG ZUR BEHANDLUNG VON KRANKHEITEN**

17-HYDROXY-17-PENTAFLUORETHYL-ESTRA-4,9(10)-DIEN-11-ETHINYLPHENYL DERIVATIVES, METHODS FOR THE PRODUCTION THEREOF AND USE THEREOF FOR TREATING DISEASES

DÉRIVÉS 17-HYDROXY-17-PENTAFLUOROÉTHYL-ESTRA-4,9(10)-DIÈNE-11-ÉTHINYLPHÉNYLE, PROCÉDÉS DE PRÉPARATION ET UTILISATION POUR LE TRAITEMENT DE MALADIES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **21.07.2009 DE 102009034526**

(43) Veröffentlichungstag der Anmeldung:
**30.05.2012 Patentblatt 2012/22**

(73) Patentinhaber: **Bayer Intellectual Property GmbH 40789 Monheim (DE)**

(72) Erfinder:
- **KLAR, Ulrich 13503 Berlin (DE)**
- **SCHWEDE, Wolfgang 16548 Glienicke (DE)**
- **MÖLLER, Carsten 10115 Berlin (DE)**
- **ROTGERI, Andrea 13503 Berlin (DE)**
- **BONE, Wilhelm 13467 Berlin (DE)**

(74) Vertreter: **BIP Patents c/o Bayer Intellectual Property GmbH Alfred-Nobel-Straße 10 40789 Monheim am Rhein (DE)**

(56) Entgegenhaltungen:
**WO-A1-98/34947 DE-A1-102006 054 535**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 456 783 B1

**Beschreibung**

Die Erfindung betrifft den in den Anspüchen beschriebenen Gegenstand, d.h. neue 17-Hydroxy-13-methyl-17-pentafluorethyl-11-ethinylphenyl-dodecahydro-cyclopenta[a]phenanthren-3-on-Derivate mit Progesteron antagonisierender Wirkung, Verfahren zu deren Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten. Verbindungen mit antagonistischer Wirkung am Progesteronrezeptor (kompetitive Progesteronrezeptorantagonisten) sind erstmals 1982 bekannt geworden (RU 486; EP 057115) und seither intensiv untersucht und beschrieben worden.

Progesteronrezeptorantagonisten mit fluorierter 17$\alpha$-Seitenkette wurden von U. Fuhrmann et al., J. Med. Chem. 43, 5010 - 5016 (2000) beschrieben und sind in der WO 98/34947 beansprucht. Die dort beschriebenen Verbindungen mit fluorierter 17$\alpha$-Seitenkette weisen im allgemeinen eine sehr starke antagonistische Aktivität am Progesteronrezeptor auf. Sehr potente und daher in WO 98/34947 bevorzugte Verbindungen sind 11$\beta$-(4-Acetylphenyl)-20,20,21,21,21-pentafluor-17-hydroxy-19-nor-17$\alpha$-pregna-4,9-dien-3-on, 11$\beta$-(4-Acetylphenyl)-20,20,21,21,21-pentafluor-17-hydroxy-19-nor-17$\alpha$-pregna-4-en-3-on und 6'-Acetyl-9,11$\beta$-dihydro-17$\beta$-hydroxy-17$\alpha$-(1,1,2,2,2-pentafluorethyl)-4'H-naphth[3',2',1':10,9,11]ester-4-en-3-on. Diese Verbindungen werden in vivo in erheblichem Maße in verschiedene Metabolite überführt, die zum Teil starke, zum Teil geringere pharmakologische Aktivität aufweisen. Der Metabolismus tritt überwiegend am 4-Substituenten des 11 $\beta$-Phenylrestes auf.

In WO 2008/058767 werden Verbindungen beschrieben, die zumindest zum Teil Metabolite der in WO 98/34947 beschriebenen Verbindungen sind.

Aufgabe der vorliegenden Erfindung ist es, hoch potente kompetitive Prögesteronrezeptorantagonisten zur Verfügung zu stellen und damit alternative Behandlungsmöglichkeiten gynäkologischer Erkrankungen zu schaffen.

Es wurde gefunden, dass die erfindungsgemäßen Verbindungen besonders geeignet sind, um diese Aufgabe zu lösen.

Die vorliegende Erfindung betrifft 17-Hydroxy-17-pentafluorethyl-estra-4,9(10)-dien-11-methylenoxy-alkylenaryl-Derivate mit der allgemeinen chemischen Formel I:

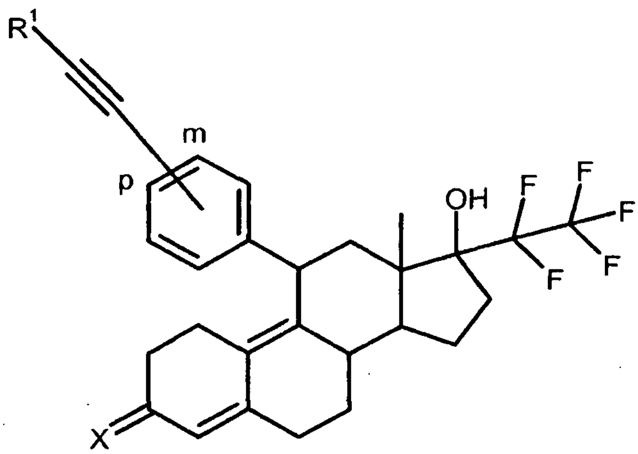

(I)

worin

- $R^1$ über eine C-C-Dreifachbindung in Position m oder p am Phenylring angeknüpft ist und für einen Rest $-(CH{=}CH)_n-R^2$, $-(CH_2)_q-R^3$ oder $-CH{=}NOR^4$ steht,
- $R^2$ Wasserstoff oder eine Aryl-, $C_1$-$C_{10}$-Alkyl-, $-CO_2R^6$ oder -CN Gruppe ist,
- $R^3$ Wasserstoff, $NH_2$, $N_3$ oder eine $-NHCONHR^4$, $-OCONHR^4$, $-OR^5$ Gruppe ist,
- $R^4$ ausgewählt ist aus der Gruppe, umfassend Wasserstoff, $C_1$-$C_{10}$-Alkyl, $C_2$-$C_{10}$-Alkenyl, Aryl, $C_7$-$C_{20}$-Aralkyl, $(CH_2)_S-R^6$, $CH_2-CO-OR^6$,

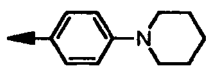,

worin

$R^5$ ausgewählt ist aus der Gruppe, umfassend Wasserstoff, $C_7$-$C_{20}$-Aralkyl, $CH_2CO_2R^6$, $CH_2CN$, $CH_2CH_2OH$,

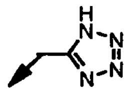 , 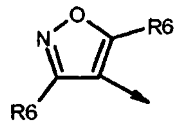

n für 0 bis 2,
q für 1 oder 2,
s für 1 oder 2,
$R^6$ für Wasserstoff, $C_1$-$C_{10}$-Alkyl, Aryl, $C_7$-$C_{20}$-Aralkyl,
X für Sauerstoff, $NOR^6$ oder eine $NNHSO_2R^6$ Gruppe, mit $R^6$ in der angegebenen Bedeutung, steht.

Der Pfeil beispielsweise in der

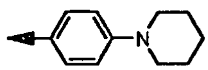 oder 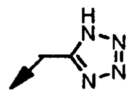

Gruppe, kennzeichnet die Stelle an der die Bindung des jeweiligen Restes an die benachbarte Gruppe erfolgt.

Im Rahmen der vorliegenden Erfindung haben die Substituenten, soweit nicht anders spezifiziert, die folgende Bedeutung:

Unter Alkvl in $R^2$, $R^4$ und $R^6$ sowie in anderen Fällen sind gerad- oder verzweigtkettige Alkylgruppen mit der angegebenen Anzahl an Kohlenstoffatomen oder gegebenenfalls 1-10 Kohlenstoffatomen zu verstehen, wie beispielsweise Methyl, Ethyl, Propyl, Isopropyl, Butyl, Isobutyl, tert.-Butyl, Pentyl, Isopentyl, Neopentyl, Hexyl, Heptyl, Octyl, Nonyl oder Decyl. Die Alkylgruppen $R^2$, $R^4$ und R6 können ferner perhalogeniert, bevorzugt perfluoriert oder durch 1-5 Halogenatome, Hydroxygruppen, $C_1$-$C_4$-Alkoxygruppen, $C_6$-$C_{12}$-Arylgruppen (die wiederum durch 1-3 Halogenatome substituiert sein können) substituiert sein. Insbesondere kann Alkyl daher auch für Hydroxymethylen (HO-$CH_2$), Hydroxyethylen (HO-$C_2H_4$), Hydroxypropylen (HO-$C_3H_6$) und Hydroxybutylen (HO-$C_4H_8$) sowie deren Isomere stehen.

Unter Alkenyl in $R^4$ sind gerad- oder verzweigtkettige Alkenylgruppen mit 2-10 Kohlenstoffatomen zu verstehen, wie beispielsweise Vinyl, Propenyl, Butenyl, Pentenyl, Isobutenyl oder Isopentenyl.

Die Alkenylgruppe $R^4$ kann durch 1-5 Halogenatome, Hydroxygruppen, $C_1$-$C_3$-Alkoxygruppen oder $C_6$-$C_{12}$-Arylgruppen (die wiederum durch 1-3 Halogenatome substituiert sein können) substituiert sein.

Unter Aryl in $R^2$, R4 und $R^6$ sowie in anderen Fällen sind aromatische, mono- oder bicyclische Reste zu verstehen, wie zum Beispiel Phenyl oder Naphthyl die einfach oder mehrfach mit Halogen, OH, $SO_2$-Alkyl, SO-Alkyl und S-Alkyl, O-Alkyl, $CO_2H$, $CO_2$-Alkyl, $NH_2$, $NO_2$, $N_3$, CN, $C_1$-$C_{10}$-Alkyl, $C_1$-$C_{10}$-Acyl oder $C_1$-$C_{10}$-Acyloxy-Gruppen, substituiert sein können. Soweit ansonsten Aryl als Substituent an Alkyl, Alkenyl oder Alkinyl erwähnt wird, handelt es sich insbesondere um Arylgruppen mit 5-12 Ringatomen.

Unter Aralk in $R^4$, $R^5$ und $R^6$ sind Aralkylgruppen zu verstehen. Aralkyl steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl, Furylmethyl, Thienylethyl oder Pyridylpropyl in Betracht. Die Ringe können einfach oder mehrfach durch Halogen, OH, O-Alkyl, $CO_2H$, $CO_2$-Alkyl $NH_2$, NH($C_1$-$C_{10}$-Alkyl), N($C_1$-$C_{10}$-Alkyl)$_2$, $NO_2$, $N_3$, CN, $C_1$-$C_{20}$-Alkyl, $C_1$-$C_{10}$-Perfluor-Alkyl, $C_1$-$C_{20}$-Acyl oder $C_1$-$C_{20}$-Acyloxy-Gruppen substituiert sein.

Als Heteroatome kommen Schwefel, Sauerstoff oder Stickstoff in Frage, wobei Stickstoff bevorzugt ist. Als Beispiel sei der Pyridylpropyl-Rest genannt.

Soweit Alkoxy (O-Alkyl) erwähnt wird, handelt es sich um Alkoxygruppen mit 1-4 Kohlenstoffatomen. Alkoxy kann insbesondere Methoxy, Ethoxy und Propoxy sein.

Soweit Acyl (CO=Alkyl) erwähnt wird, handelt es sich um Acylgruppen mit 1-20 Kohlenstoffatomen. Acyl kann insbesondere Formyl, Acetyl, Propionyl und Butyryl sein.

Soweit Acyloxy (O-CO-Alkyl) erwähnt wird, handelt es sich um Acyloxygruppen mit 1-20 Kohlenstoffatomen. Acyloxy kann insbesondere Formyloxy, Acetyloxy, Propionyloxy und Butyryloxy sein.

Halogen bedeutet Fluor, Chlor oder Brom. Unter diesen sind Fluor oder Chlor bevorzugt.

Erfindungsgemäß bevorzugt sind Derivate bei denen die Gruppe X ein Säuerstoffatom ist.

Bevorzugt sind weiterhin Verbindungen bei denen die Gruppe

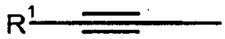

in para- Position an den Phenylring geknüpft ist.

Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.

Jeder der genannten Substituenten am Steroid-Grundgerüst kann sowohl in einer α- als auch in einer β-Stellung vorliegen. Außerdem können auch die Substituenten am Steroid-Grundgerüst, die eine Doppelbindung enthalten und in denen die Doppelbindung an jedem Atom mindestens einen Substituenten, der nicht Wasserstoff ist, trägt, sowohl E- als auch Z-konfiguriert vorliegen.

Sofern die erfindungsgemäßen Verbindungen in tautomeren Formen vorkommen können, umfasst die vorliegenden Erfindung sämtliche tautomere Formen.

Die erfindungsgemäßen Verbindungen der allgemeinen Formel I können auch als Solvate, Hydrate und Salze vorliegen, wobei auch unterschiedliche Kristallmodifikationen, sowie α-, β- oder γ-Cyclodextrinclathrate oder mit Liposomen verkapselten Verbindungen der Formel I umfasst sind.

Als Solvate werden im Rahmen der Erfindung solche Formen der erfindungsgemäßen Verbindungen bezeichnet, welche in festem oder flüssigem Zustand eine Adduktbildüng mit Lösungsmittelmolekülen zeigen. Liegen die Derivate der Formel I als Solvat vor, kann das enthaltene Solvens in einem stöchiometrischen oder auch nicht-stöchiometrischen Verhältnis vorliegen. Bei stöchiometrischen Solvaten, spricht man auch von Hemi-, (Semi-), Mono-, Sesqui-, Di-, Tri-, Tetra-, Penta-, usw. Solvaten.

Hydrate sind eine spezielle Form der Solvate, bei denen die Koordination mit Wasser erfolgt.

Erfindungsgemäß bevorzugt sind unter den Solvaten solche mit Wasser. Diese werden auch als Hydrate bezeichnet.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen-wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure oder Benzoesäure.

Physiologisch unbedenkliche Salze der erfindungsgemäßen Verbindungen umfassen-wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B: Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methyl-aminomethan oder 1-Amino-2,3,4-butantriol.

Es wurde gefunden, dass die erfindungsgemäßen Verbindungen der Formel (I) überraschenderweise eine gute, das Progesteron antagonisierende Wirkung aufweisen, ohne die Nachteile der Verbindungen des Standes der Technik zu haben.

So zeigen die meisten der erfindungsgemäßen Verbindungen, eine überraschend hohe metabolische Stabilität in vitro in Lebermikrosomen von Ratte und Mensch. Bei den in vivo in der Ratte getesteten Verbindungen wurde zum Teil eine niedrige Clearance und eine überraschend lange Halbwertzeit in vivo in der Ratte gefunden. Gleichzeitig ist es gelungen, die Wasserlöslichkeit für einige Verbindungen deutlich zu verbessern (*siehe Beispiel 61).*

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden.

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/ Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84-tägige Gabe des Progesteronrezeptorantagonisten gefolgt von der 14-tägigen Gabe des Gestagens.

Zur Behandlung von Tumor-Erkrankungen können z.B. die folgenden Wirkstoffe/Wirkstoffklassen entweder gleichzeitig oder equentiell verabreicht werden: SERMs, SERDs, Antiöstrogene, Aromataseinhibitoren, Kinaseinhibitoren, Angiogeneseinhibitoren und/oder Cytostatika.

Zur Behandlung von mit der Menopause assoziierten Beschwerden kommt eine gleichzeitige oder sequentielle Verabreichung der erfindungsemäßen Verbindungen z.B. mit SERMs, SERDs und Östrogenen in Betracht.

SERMs (Selective Estrogen Receptor Modulators) sind erfindungsgemäß solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen. Selektive Estrogenrezeptordestabilisatoren (SERD) sind Arzneistoffe, die den Estrogenrezeptor vollständig antagonisieren und zu einem Abbau des Rezeptors führen.

Antiöstrogene sind Verbindungen die den Estrogenrezeptor vollständig antagonisieren, beispielsweise Fulvestrant.

Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.

Kinaseinhibitoren hemmen Enzyme, die einen Phosphatrest von ATP auf andere Substrate, dort insbesondere auf Hydroxygruppen, übertragen, z.B. Sorafenib (Nexavar) oder Imatinib (Gleevec).

Angiogenesehemmer, z.B. Avastin, reduzieren bzw. blockieren die Gefäßneubildung und damit die Durchblutung eines Tumors.

Zytostatika, z.B. cis-Platin, Taxol, Taxotere, Sagopilon, Ixabepilon sind natürliche oder synthetische Substanzen, die Tumorzellen in die Apoptose treiben.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die Wirksamkeit der erfindungsgemäßen Verbindungen als Progesteronrezeptorantagonist wurde in vitro in Transaktivierungstests und in vivo an der Ratte (Terminierung der frühen Schwangerschaft) gezeigt.

Erfindungsgemäß besonders bevorzugt sind die nachstehend genannten Verbindungen deren Herstellung in den Beispielen beschrieben ist:

- ➢ (8S,11R,13S,14S,17S)-17-Hydroxy-11-[4-(3-hydroxy-prop-1-in-1-yl)-phenyl]-13-methyl-17-pentafluorethyl-1,2,6,7,8,11,12,13, 14,15,16,17-dodecahydrocyclopenta[a]phenanthren-3-on (Beispiel 1)

- ➢ [4-((8S,11R,13S,14S,17S)-17-Hydroxy-13-methyl-3-oxo-17-pentafluorethyl-2,3,6,7,8,11,12,13,14,15,16,17-dodecahydro-*1H*-cyclopenta[a]phenanthren-11-yl)-phenyl]-propinsäure (Beispiel 2)

- ➢ [4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-propinsäure-methylester (Beispiel 3)

- ➢ (E)-5-[4-((8S,11R,13S,14S,17S)-17-Hydroxy-13-methyl-3-oxo-17-pentafluorethyl-2,3,6,7,8,11,12,13,14,15,16,17-dodecahydro1*H*-cyclopenta[*a*]phenanthren-11-yl)-phenyl]-pent-2-en-4-innitril (Beispiel 4)

- ➢ (Z)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril (Beispiel 5)

- ➢ (E)-5-[4-((8S,11 R,13S,14S, 17S)-17-Hydroxy-13-methyl-3-oxo-17-pentafluorethyl-2,3,6,7,8,11,12,13,14,15,16,

17-dodecahydro-1*H*-cyclopenta[*a*]phenanthren-11-yl)-phenyl]pent-2-en-4-n-säureethylester (Beispiel 6)

➢ 7-(2E/Z,4E/Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-hepta-2,4-dien-6-inylsäureethylester (Beispiel 7)

➢ {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-essigsäure*tert*-butylester (Beispiel 8)

➢ {3-[4-((8S,11R,13S,14S,17S)-17-Hydroxy-13-methyl-3-oxo-1,7-pentafluorethyl-2,3,6,7,8,11,12,13,14,15,16,17-dodecahydro-1*H*-cyclopenta[*a*]phenanthren-11-yl)-phenyl]-prop-2-inyloxy}-essigsäuremethylester (Beispiel 9)

➢ {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-essigsäure (Beispiel 10)

➢ (8S,11R,13S,14S,17S)-17-Hydroxy-11-{4-[3-(2-hydroxy-ethoxy)-prop-1-in-1-y1]-phenyl}-13-methyl-17-pentafluorethyl-1,2,6,7,8,11,12,13,14,15,16,17-dodecahydro-cyclopenta[*a*] phenanthren-3-on (Beispiel 11)

➢ 3-{(E/Z)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäuremethylester (Beispiel 12)

➢ 3-{(E)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäure (Beispiel 13A)

➢ 3-{(Z)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäure (Beispiel 13B)

➢ {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-acetonitril (Beispiel 14)

➢ 4-[4-((8S, 11 R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester (Beispiel 15)

➢ 4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure (Beispiel 16)

➢ 3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester (Beispiel 17)

➢ (8S,11R,13S,14S,17S)-17-Hydroxy-13-methyl-17-pentafluorethyl-11-{4-[3-(1*H*-tetrazol-5-ylmethoxy)-prop-1-in-1-yl]-phenyl}-1,2,6,7,8,11,12,13,14,15,16,17-dodecahydro-cyclopenta[a]phenanthren-3-on (Beispiel 18)

➢ 4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure (Beispiel 19)

➢ 3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-phenylethinyl]-benzoesäure (Beispiel 20)

➢ (8S,11R,13S,14S,17S)-17-Hydroxy-11-[4-(3-hydroxy-3-methyl-but-1-inyl)-phenyl]-13-methyl-17-pentafluorethyl-1,2,6,7,8,11,12,13,14,15,16,17-dodecahydro-cyclopenta[*a*]phenanthren-3-on (Beispiele 21)

➢ (8S,11R,13S,14S,17S)-11-(4-Ethtnyl-phenyl)-17-hydroxy-13-methyl-17-pentafluorethyl-1,2,6,7,8,11,12,13,14,15,16,17-dodecahydro-cyclopenta[*a*]phenanthren-3-on (Beispiel 22)

➢ (8S,11R,13S,14S,17S)-11-[4-(3-Azido-prop-1-in-1-yl)-phenyl]-17-hydroxy-13-methyl-17-pentafluorethyl-1,2,6,7,8,11,12,13,14,15,16,17-dodecahydro-cyclopenta[a]phenanthren-3-on (Beispiel 23)

➢ (E)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyt]-propinal *O*-benzyl-oxim (Bespiel 24A)

➢ und (Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-benzyl-oxim (Beispiel 24B)

➢ (E)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-ethyl-oxim (Beispiel 25A)

➢ (Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-ethyl-oxim (Beispiel 25B)

➢ [4-((8S,11R,13S,14S,17S)-17-Hydroxy-13-methyl-3-oxo-17-pentafluorethyl-2,3,6,7,8,11,12,13,14,1,5,16,17-dodecahydro-1*H*-cyclopenta[*a*]phenanthren-11-yl)-phenyl]-propinal *O*-isobutyl-oxim (Beispiel 26)

➢ [4-((8S,11R,13S,14S,17S)-17-Hydroxy-13-methyl-3-oxo-17-pentafluorethyl-2,3,6,7,8,11,12,13,14,15,16,17-dodecahydro-1*H*-cydopenta[*a*]phenanthren-11-yl)-phehyl]-propinal *O*-(3,4-dichlor-benzyl)-oxim (Beispiel 27)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-isopropyl-harnstoff (Beispiel 29)

➢ 3-(3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-propionsäureethylester (Beispiel 30)

➢ 1-Ethyl-3-{3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff (Beispiel 31)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-methoxy-phenyl)-harnstoff (Beispiel 32)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-*tert*-butyl-phenyl)-harnstoff (Beispiel 33)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[*a*]phenanthren-11-yl)-phenyl]-prop-2-inyl}-3-phenyl-harnstoff (Beispiel 34)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[*a*]phenanthren-11-yl)-phenyl]-prop-2-inyl}-3-(4-chlor-phenyl)-harnstoff (Beispiel 35)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1 H-cyclopenta[a]phenanthren-11-yl)-phenyl]-prop-2-inyl}-3-(4-fluor-phenyl)-harnstoff (Beispiel 36)

➢ 4-(3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-benzoesäureethylester (Beispiel 37)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-methyl-phenyl)-harnstoff (Beispiel 38)

➢ 1-Benzyl-3-{3-[4-((8S,11R,13S,14S,17S)-17-Hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-prop-2-inyl}-harnstoff (Beispiel 39)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-*tert*-butyl-harnstoff (Beispiel 40)

➢ 1-Allyl-3-{3-[4-((8S,11R,13S, 14S, 17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-prop-2-inyl}-harnstoff (Beispiel 41)

➢ (3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-essigsäureethylester (Beispiel 42)

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-piperidin-1-yl-phenyl)-harnstoff (Beispiel 43)

➢ Allyl-carbaminsäure 3-[4-((8S,11R,13S,14S,17S)-17-hydroxy-13-methyl-3-oxo-17-pentafluorethyl-2,3,6, 7,8,11,12,13,14,15,16,17-dodecahydro-1*H*-cyclopenta[*a*]phenanthren-11-yl)-phenyl]-prop-2-inyl ester (Beispiel 44)

➢ Ethyl-carbaminsäure 3-[4-((8S,11R,13S,14S,17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-prop-2-inyl ester (Beispiel 45)

➢ Phenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 46)

➢ 4-Methylphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*] phenanthren-11-yl)-phenyl]-prop-2-inyl ester (Beispiel 47)

➢ 4-Fluorphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 48)

➢ Isopropyl-carbaminsäure 3-[4-((8S,11R,13S,14S,17S)-17-hydroxy-13-methyl-3-oxo-17-pentafluorethyl-2,3,6,7,8,11,12, 13,14,15,16,17-dodecahydro-1*H*-cyclopenta[*a*]phenanthren-11-yl)phenyl]-prop-2-inyl ester (Beispiel 49)

➢ Benzyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 50)

➢ (8S,11R,13S,14S,17S)-17-Hydroxy-11-[4-(3-methanesulfonyl-phenylethinyl)-phenyl]-13-methyl-17-pentafluorethyl-1,2,6,7,8,11,12,13,14,15,16,17-dodecahydro-cyclopenta[*a*]phenanthren-3-on (Beispiel 51)

➢ 4-Methoxyphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*] phenanthren-11-yl)-phenyl]-prop-2-inyl ester (Beispiel 52)

➢ 4-{3-[4-((8S,11R,13S,14S,17S)-17-Hydroxy-13-methyl-3-oxo-17-pentafluorethy)-2,3,6,7,8,11,12,13,14,15,16,17-dodecahydro-1*H*-cyclopenta[*a*]phenanthren-11-yl)-phenyl]-prop-2-inyloxycarbonylamino}-benzoesäureethylester (Beispiel 53)

➢ (4-Piperidin-1-yl-phenyl)-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 54)

➢ 4-Chlorphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 55)

➢ (8S,11R,13S,14S,17S)-17-Hydroxy-11-[4-(4-methanesulfonyl-phenylethinyl)-phenyl]-13-methyl-17-pentafluorethyl-1,2,6,7,8,11,12,13,14,15,16,17-dodecahydro-cyclopenta[*a*]phenanthren-3-on (Beispiel 56)

➢ 3-Pyridyl-carbaminsäure 3-[4-((8S,11 R,13S,14S,17S)-17-hydroxy-13-methyl-3-oxo-17-pentafluorethyl-2,3,6,7, 8,11,12,13,14,15,16,17-dodecahydro-1*H*-cyclopenta[*a*]phenanthren-11-yl)-phenyl]-prop-2-inyl ester (Beispiel 57)

➢ *tert*-Butyl-carbaminsäure 3-[4-((8S,11R,13S,14S,17S)-17-hydroxy-13-methyl-3-oxo-17-pentafluorethyl-2,3,6,7, 8,11,12,13,14,15,16,17-dodecahydro-1*H*-cyclopenta[*a*]phenanthren-11-yl)-phenyl]-prop-2-inyl ester (Beispiel 58)

➢ 4-*tert*-Butylphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester (Beispiel 59)

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Die Erfindung betrifft weiterhin ein Verfahren zu Herstellung der erfindungsgemäßen Derivate der Formel I. Derartige Derivate lassen sich wie in Schema 1 dargestellt, herstellen, indem man ein Phenylsulfonat der allgemeinen Formel II, worin

$R^{1'}$ eine perforiert $C_1$-$C_{10}$-Alkylgruppe, X' ein Sauerstoffatom, zwei Alkoxygruppen $OR^7$, eine $C_2$-$C_{10}$-Alkylen-$\alpha$, $\omega$-dioxygruppe, die geradkettig oder verzweigt sein kann,

$R^7$ $C_1$-$C_4$-Alkyl,

$R^8$ Wasserstoff,

$R^9$ eine Hydroxylgruppe, oder

$R^8$, $R^9$ gemeinsam eine Bindung bedeuten,

durch Palladium katalysierte Kupplungsreaktionen in eine Verbindung der allgemeinen Formel I' worin $R^1$, X', $R^8$ und $R^9$ die oben genannten Bedeutungen haben umsetzt und gegebenenfalls in $R^1$ vorhandene Funktionalitäten umwandelt und/oder weitere Folgereaktionen durchführt und gebenenfalls aus der Gruppe X' die Gruppe X in der Bedeutung von Sauerstoff freisetzt und/oder eine Doppelbindung ($R^8$, $R^9$ gemeinsam eine Bindung) durch Eliminierung von Wasser ($R^8$ = Wasserstoff, $R^9$ = Hydroxylgruppe) erzeugt und gegebenenfalls die Carbonylgruppe (X = Sauerstoff) weiter funktionalisiert (X = $NOR^6$ oder eine $NNHSO_2R^6$ Gruppe).

Schema 1

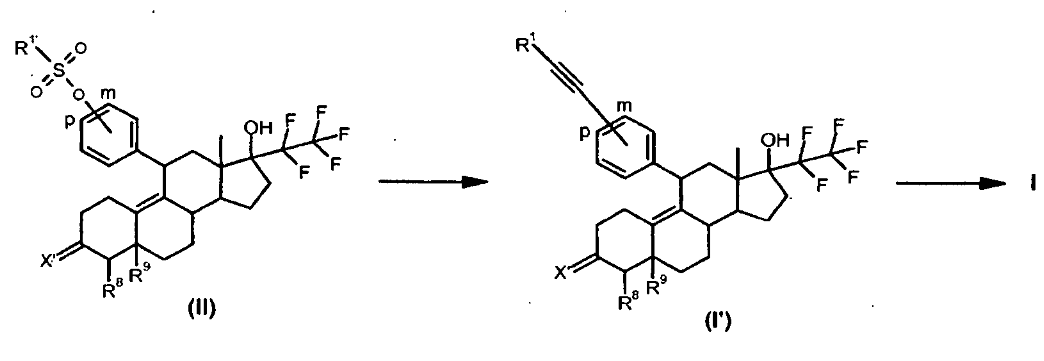

Stellen $R^8$ und $R^9$ gemeinsam eine Bindung in der Struktur der allgemeinen Formel I' dar, so steht diese stellvertretend für die drei möglichen Doppelbindungsisomeren I'-A, I'-B und I'-C, die während der beschriebenen Umsetzungen in unterschiedlichsten Verhältnissen zueinander entstehen können.

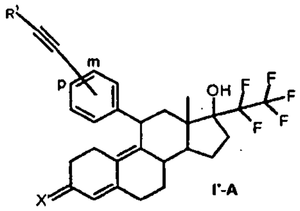
I'-A

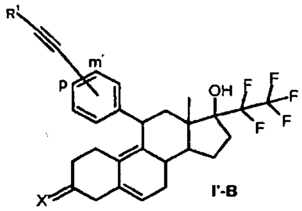
I'-B

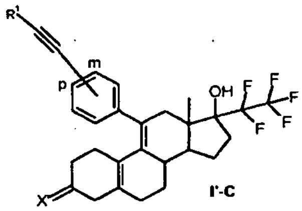
I'-C

Einige mögliche Umsetzungssequenzen zu Verbindungen der allgemeinen Formel I' sind exemplarisch in Schema 2 näher ausgeführt.

Schema 2

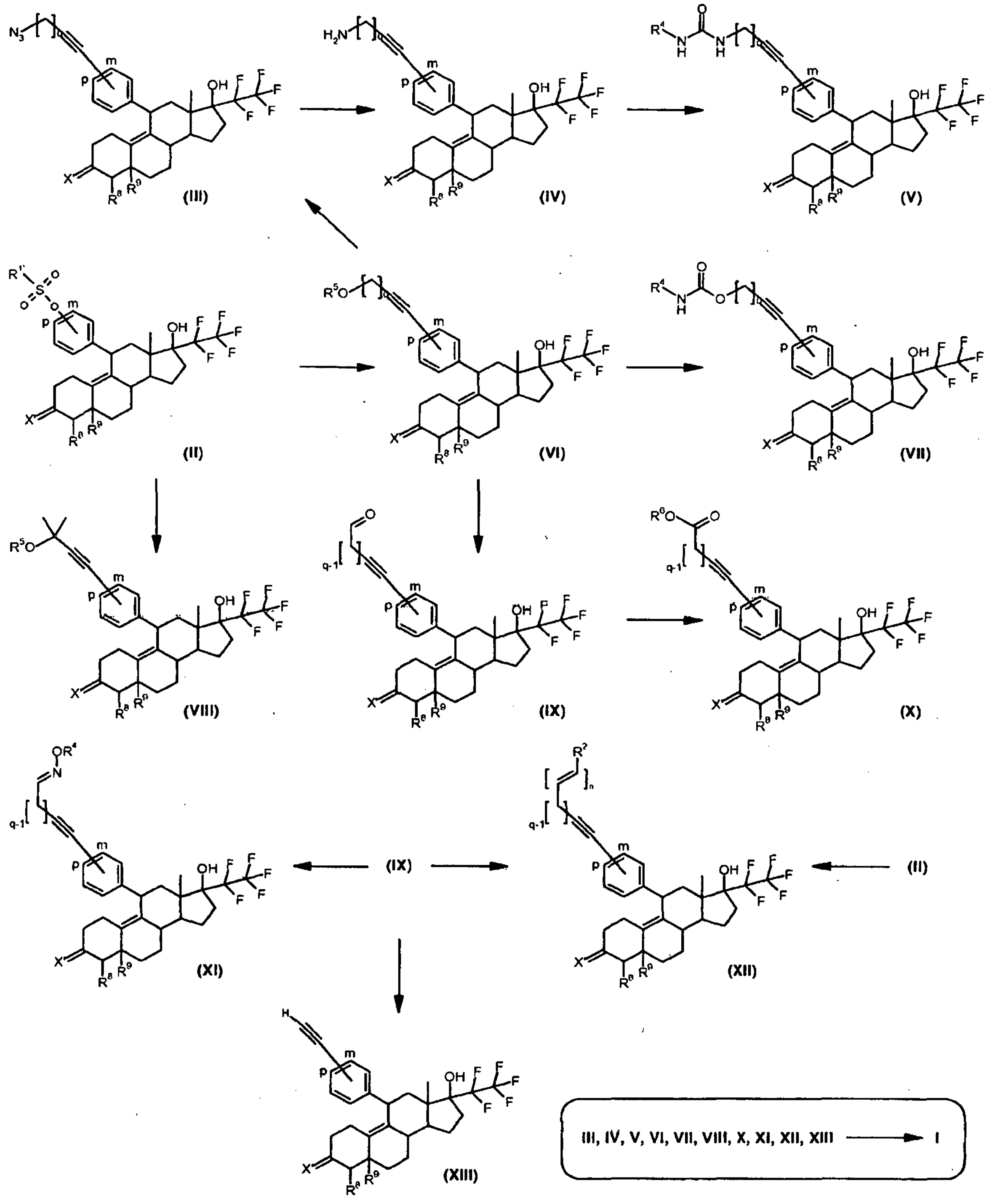


Die Umsetzung von Verbindungen der allgemeinen Formel II zu Verbindungen der allgemeinen Formeln VI, VIII oder XII erfolgt durch Palladium katalysierte Kupplungsreaktionen nach den, dem Fachmann bekannten Methoden. Typische Ausführungen finden sich in den Beispielen 1a, 15a, 17b und 19.

Die Umsetzung von Verbindungen der allgemeinen Formel VI zu Verbindungen der allgemeinen Formel VII erfolgt (a) durch Reaktion des Alkoholes mit Isocyanaten oder (b) zweistufig durch Herstellung des Chlorameisensäureesters und weitere Umsetzung mit Aminen nach den, dem Fachmann bekannten Methoden. Typische Ausführungen zu (a) finden sich in den Beispielen 44a bis 49a und zu (b) in den Beispielen 50, 52-55 und 57-59.

Die Umsetzung von Verbindungen der allgemeinen Formel IV zu Verbindungen der allgemeinen Formeln V erfolgt mit Isocyanaten erfolgt nach den, dem Fachmann bekannten Methoden. Typische Ausführungen finden sich in

den Beispielen 28-43.

Die Umsetzung von Verbindungen der allgemeinen Formel VI zu Verbindungen der allgemeinen Formeln X erfolgt (a) entweder durch direkte Oxidation oder (b) durch eine zweistufige Oxidation unter Bildung des Aldehydes der allgemeinen Formel IX und Folgeoxidation zu X nach den, dem Fachmann bekannten Methoden. Beispielsweise genannt sei die direkte Oxidation mit Jones-Reagenz. Typische Ausführungen zu (b) finden sich in den Beispielen 2a und 2.

Die Umsetzung von Verbindungen der allgemeinen Formel IX zu Verbindungen der allgemeinen'Formeln XI erfolgt nach den, dem Fachmann bekannten Methoden. Typische Ausführungen finden sich in den Beispielen 24a-27a.

Die Umsetzung von Verbindungen der allgemeinen Formel IX zu Verbindungen der allgemeinen Formeln XII erfolgt nach den, dem Fachmann bekannten Methoden. Typische Ausführungen finden sich in den Beispielen 4a, 6a, 7a und 12a.

Die Umsetzung von Verbindungen der allgemeinen Formel IX zu Verbindungen der allgemeinen Formeln XIII erfolgt nach den, dem Fachmann bekannten Methoden. Eine typische Ausführung findet sich in Beispiel 22a.

Soweit durch die beschriebenen Umsetzungen noch nicht geschehen, werden in den Verbindungen der allgemeinen Formeln III, IV, V, VI, VII, VIII, X, XI, XII, XIII gegebenenfalls vorhandene Ketale in X' gespalten und/oder, falls $R^8$ die Bedeutung Wasserstoff und $R^9$ die Bedeutung Hydroxyl besitzt, Wasser eliminiert. Eine typische Ausführung findet sich in Beispiel 1 und 28.

Soweit die Herstellung der Ausgangsverbindungen hier nicht beschrieben ist, sind diese dem Fachmann bekannt oder analog zu bekannten Verbindungen oder hier beschriebenen Verfahren herstellbar (siehe auch Beispiele 1 b bis 1 e).

Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Die erfindungsgemäßen Verbindungen können systemisch und/oder lokal wirken. Zu diesem Zweck können sie auf geeignete Weise, wie z.B. oral, intrauterin[är], intravaginal, parenteral, pulmonal, nasal, sublingual, lingual, buccal, rectal, dermal, transdermal, conjunctival, otisch oder als Implantat bzw. Stent appliziert werden. Intrauterin[är] bedeutet dabei insbesondere die Applikation mittels IUS (intrauterine system) oder IUD (intrauterine device). Die intravaginale Applikation kann u.a. mittels IVR/VRS (Intra-Vaginalring/Vaginalringsystem) erfolgen.

Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nonsilikon- und/oder Silikonpolymere, insbesondere auch siloxanebasierte Elastomere (vergl. WO 01/47490, insbesondere Seite 7, Zeile 19 - Seite 15, Zeile 15), enthalten.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen,

Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in

an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die nachfolgenden Beispiele dienen der Erläuterung der Erfindung ohne diese zu beschränken.

Beispiel 1:

**(8S,11R,13S,14S,17S)-17-Hydroxy-11-[4-(3-hydroxy-prop-1-in-1-yl)-phenyl]-13-methyl-17-pentafluorethyl-1,2,6,7,8,11,12,13,14,15,16,17-dodecahydro**cyclopenta[a]phenanthren-3-on

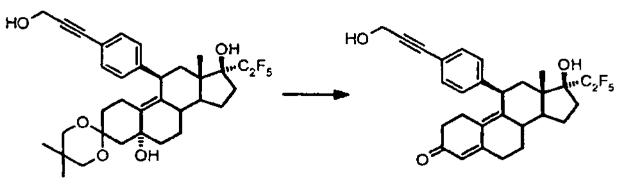

Die Lösung von 200 mg (0,32 mmol) der nach Beispiel 1 a dargestellten Verbindung in 10,4 ml Aceton versetzte man mit 480 ml einer 4N Salzsäure und rührte 20 Minuten bei 23°C. Man goss in eine gesättigte Natriumhydrogencarbonatlösung, extrahierte mehrfach mit Ethylacetat, trocknete die vereinigten organischen Extrakte über Natriumsulfat und reinigte den nach Filtration und Lösungsmittelabzug erhaltenen Rückstand durch Chromatographie. Isoliert wurden 133 mg (80%) der Titelverbindung als farbloser Schaum.

$^1$H-NMR ($CDCl_3$): $\delta$= 0,58 (3H), 1,40-1,55 (2H), 1,71-1,85 (4H), 2,02-2,63 (11 H), 2,72 (1 H), 4,43 (1 H), 4,49 (2H), 5,79 (1H), 7,13 (2H), 7,36 (2H) ppm.

Beispiel 1 a:

(5R,8S,11R,13S,14S,17S)-11-[4-(3-hydroxyprop-1-in-1-yl)phenyl]-5',5',13-trimethyl-17-(pentafluorethyl)-1,2,3,4,6,7,8,11,12,13,14,15,16,17-tetradecahydrospiro[cyclopenta[a]phenanthren-3,2'-[1,3]dioxan]-5,17-diol

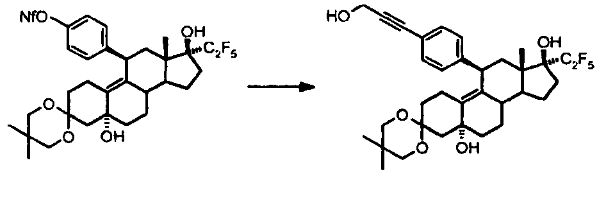

Die Lösung von 34,4 g (39,6 mmol) der nach Beispiel 1b dargestellten Verbindung in 435 ml Tetrahydrofuran versetzte man mit 19,6 ml Piperidin, 4,58 g

Tetrakis(triphenylphosphin)palladium(0), 11,7 ml Propargylalkohol und erhitzte 1 Stunde auf 80°C. Man goss in eine gesättigte Ammoniumchloridlösung, extrahierte mehrfach mit Ethylacetat, wusch die vereinigten organischen Extrakte mit gesättigter Ammoniumchlorid- und Natriumchloridlösung und trocknete über Natriumsulfat. Den nach Filtration und Lösungsmittelabzug erhaltenen Rückstand reinigte man durch Chromatographie. Isoliert wurden 4,53 g (18%) der Titelverbindung als farbloser Schaum.

Beispiel 1b:

1,1,2,2,3,3,4,4,4-Nonafluor-butan-1-sulfonsäure 4-((5R,8S,11R,13S,14S,17S)-5,17-dihydroxy-5',5',13-trimethyl-17-(pentafluorethyl)-1,2,3,4,6,7,8,11,12,13,14,15,16,17-tetradecahydro-spiro[cyclopenta[a]phenanthren-3,2'-[1,3]dioxan]-11-yl)phenyl ester

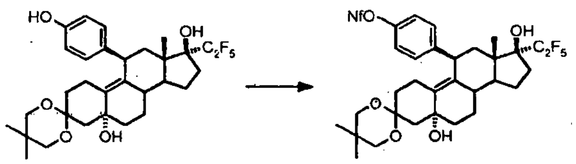

Zu einer Lösung von 25 g (43 mmol) der der nach Beispiel 1c dargestellten Verbindung in 590 ml Tetrahydrofuran wurden bei -10°C 26,4 ml einer 1,6 molaren Lösung von n-Butyllithium in Hexan addiert. Man ließ 30 Minuten bei 0°C nachrühren, versetzte mit 15,5 ml Perfluorbutan-1-sulfonsäurefluorid und ließ weitere 1,5 Stunden bei 0°C nachrühren. Man versetzte mit 10 ml Triethylamin, goß in eine gesättigte Natriumhydrogencarbonatlösung und rührte 16 Stunden bei 23°C. Man versetzte erneut mit 10 ml Triethylamin und extrahierte mehrfach mit Diethylether. Die vereinigten organischen Extrakte wusch man mit gesättigter Natriumchloridlösung, trocknete über Natriumsulfat und reinigte den nach Filtration und Lösungsmittelabzug erhaltenen Rückstand durch Chromatographie. Isoliert wurden 31,8 g (82%) der Titelverbindung als blass gelber Schaum.

Beispiel 1 c:

((5R,8S,11R,13S,14S,17S)-11-(4-Hydroxyphenyl)-5,17-dihydroxy-5',5',13-trimethy)-17-(pentafluorethyl)-1,2,3,4,6,7,8,11,12,13,14,15,16,17-tetradecahydrospiro[cyclopenta[a]phenanthren-3,2'-[1,3]dioxan]

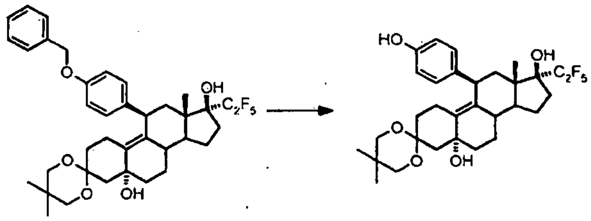

Die Lösung aus 90 g (96 mmol) der nach Beispiel 1d dargestellten Verbindung und 30,3 g Ammoniumformiat in 600 ml Methanol versetzte man mit einer Suspension aus 6,5 g Palladium auf Aktivkohle (10%ig) in 80 ml Methanol und rührte 2 Stunden bei 23°C. Man verdünnte mit Dichlormethan, filtrierte über Celite und engte ein. Der Rückstand wurde in Ethylacetat gelöst, mit Wasser und gesättigter Natriumchloridlösung gewaschen und über Natriumsulfat getrocknet. Den nach Filtration und Lösungsmittelabzug erhaltenen Rückstand reinigte man durch Kristallisation. Isoliert wurden 53,3 g (95%) der Titelverbindung als farbloser Feststoff.

Beispiel 1 d:

((5R,8S,11R,13S,14S,17S)-11-[4-(phenylmethoxy)phenyl]-5,17-dihydroxy-5',5',13-trimethyl-17-(pentafluorethyl)-1,2,3,4,6,7,8,11,12,13,14,15,16,17-tetradecahydrospiro[cyclopenta[a]phenanthren-3,2'-[1,3]dioxan]

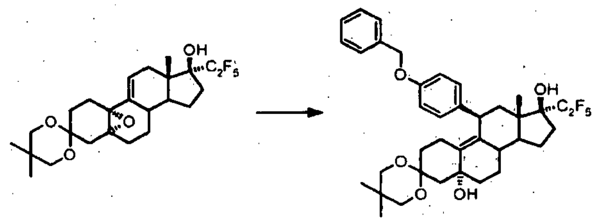

Aus 7,4 g Magnesiumspäne und einer Lösung von 80 g 1-Brom-4-(phenylmethoxy)-benzol in 267 ml Tetrahydrofuran stellte man unter Erwärmen auf 50-80°C und gegebenenfalls unter Zusatz eines Jodkristalls und/oder katalytischen Mengen 1,2-Dibromethan das Grignard-Reagenz her. Man kühlte auf 0°C, versetzte mit 1,51 g Kupfer(I)chlorid und tropfte die Lösung von 50 g (102 mmol) der nach Beispiel 1e dargestellten Verbindung in 500 ml Tetrahydrofuran zu. Man rührte noch 16 Stunden bei 23°C, goss in eine gesättigte Ammoniumchloridlösungund extrahierte mehrfach mit Ethylacetat. Die vereinigten organischen Extrakte wusch man mit gesättigter Natriumchloridlösung und trocknete über Natriumsulfat. Den nach Filtration und Lösungsmittelabzug erhaltenen Feststoff reinigte man durch Kristallisation und isolierte 47,7 g (69%) der Titelverbindung als farblosen Feststoff.

Beispiel 1e:

(5R,8S,10R,13S,14S,17S)-17-(Pentafluorethyl)-5,10-epoxy-5',5',13-trimethyl-1,2,3,4,6,7,8,12,13,14,15,16,17-tridecahydro-spiro[cyclopenta[a]phenanthren-3,2'-[1,3]dioxan]-17-ol

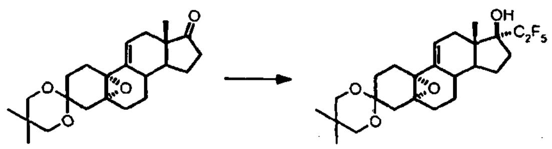

Die Lösung aus 10,0 g (27 mmol) (5'R,8'S,10'R,13'S,14'S)-5,5,13'-Trimethyl-1',2',6',7',8',12',13',14',15',16'-decahydro-17'H-spiro[1,3-dioxan-2,3'-[5,10]epoxycyclopenta[a]phenanthren]-17'-on, das man in Analogie zu dem in Tetrahedron Lett. 26, 2069-2072 (1985) beschriebenen Verfahren herstellt, in 145 ml Dichlormethan kühlte man auf-70°C, versetzte mit 11,1 ml zuvor kondensiertem Pentafluoriodethan und tropfte langsam 54 ml einer 1,5 molaren Lösung von Methyllithium-Lithiumbromid-Komplex in Diethylether zu. Nach 1 Stunde goß man in Wasser, extrahierte mit Dichtormethan, wusch mit Wasser und Natriumchloridlösung und trocknete über Natriumsulfat. Den nach Filtration und Lösungsmittelabzug isolierte man 12,9 g (98%) der Titelverbindung, die man ohne Reinigung weiter umsetzte.

**Beispiel 2:**

**[4-((8S,11R,13S,14S,17S)-17-Hydroxy-13-methyl-3-oxo-17-pentafluorethyl-2,3,6,7,8,11,12,13,14,15,16,17-dodecahydro-*1H*-cyclopenta[a]phenanthren-11-yl)-phenyl]-propinsäure**

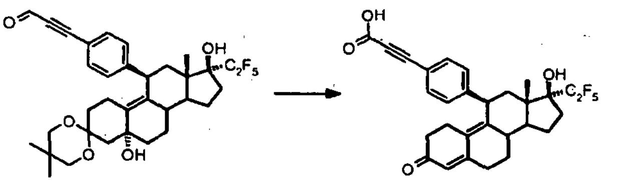

Die Lösung von 38 mg (61 µmol) der nach Beispiel 2a dargestellten Verbindung in 2 ml tert-Butanol und 1,4 ml Tetrahydrofuran versetzte man unter Eisbadkühlung mit 0,4 ml 2-Methyl-2-buten, 0,48 ml Wasser, 25,3 mg Natriumdihydrogenphosphat Monohydrate und 42,6 mg Natriumchlorit. Man ließ 1 Stunde reagieren, goss in Wasser, sättigte mit Natriumchlorid und extrahierte mehrfach mit Ethylacetat. Die vereinigten organischen Extrakte trocknete man über Natriumsulfat und reinigte den nach Filtration und Lösungsmittelabzug erhaltenen Rückstand durch Chromatographie. Isoliert wurden 7,7 mg (24%) der Titelverbindung als farbloser Schaum.

$^{1}$H-NMR ($CDCl_3$): δ= 0,56 (3H), 1,35-1,55 (2H), 1,69-2,80 (15H), 4,39 (1H), 3,31-3,93 (>1H), 5,74 (1H), 7,13 (2H), 7,35 (2H) ppm.

Beispiel 2a:

3-{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydro-spiro[cyclopenta[a]phenanthren-3,2'-[1,3]dioxan]-11-yl]phenyl}propinal

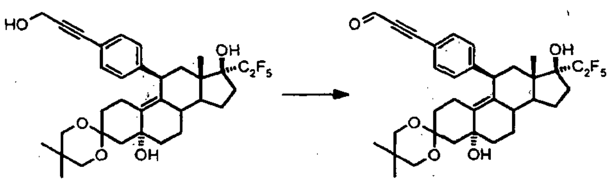

Die Lösung von 2,0 g (3,2 mmol) der nach Beispiel 1a dargestellten Verbindung in 50 ml Dichlormethan versetzte man mit wenigen Kügelchen Molekularsieb 4Å, 560 mg N-Methyl-morpholin-N-oxid, 85 mg Tetrapropylammoniumperruthenat und rührte 1,5 Stunden bei 23°C. Man reinigte durch Chromatographie und isolierte 1,44 g (72%) der Titelverbindung als farblosen Schaum.

**Beispiel 3:**

**[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinsäuremethylester**

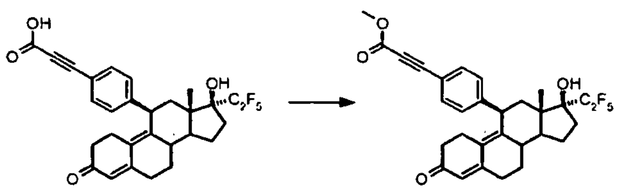

Die Lösung von 62 mg (120 µmol) der nach Beispiel 2 dargestellten Verbindung in 1 ml Tetrahydrofuran versetzte man bei 3°C mit 2,5 ml einer etherischen Lösung von Diazomethan und rührte 30 Minuten. Den nach Lösungsmittelabzug erhaltenen Rückstand reinigte man durch Chromatographie und isolierte 18 mg (28%) der Titelverbindung als farblosen Schaum.

$^{1}$H-NMR ($CDCl_3$): δ= 0,57 (3H), 1,40-1,55 (2H), 1,74-1,86 (3H), 2,07 (1H), 2,22 (1H), 2,24-2,64 (9H), 2,71 (1H), 3,84 (3H), 4,46 (1H), 5,79 (1H), 7,21 (2H), 7,50 (2H) ppm.

**Beispiel 4**

**(E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril**

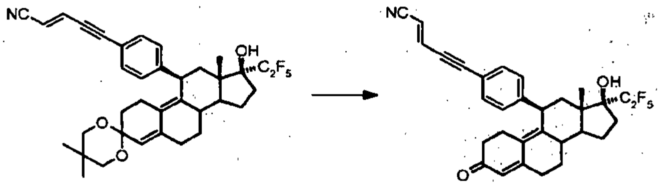

In Analogie zu Beispiel 1 setzte man 125 mg (0,2 mmol) der nach Beispiel 4a dargestellten Verbindung A um und isolierte nach Aufarbeitung und Reinigung 51 mg (47%) der Titelverbindung als farblosen Schaum.
$^1$H-NMR ($CDCl_3$): δ= 0,58 (3H), 1,40-1,56 (2H), 1,73-1,87 (3H), 2,05 (1H), 2,08 (1H), 2,19-2,65 (10H), 2,72 (1H), 4,46 (1H), 5,78 (1H), 5,80 (1H), 6,69 (1H), 7,19 (2H), 7,40 (2H) ppm.

Beispiel 4a

(2E)5-{4-[(5R,8S,11,R,13S,14S,17S)-5,17-Dihydroxy-5',5',13-trimethyl-17-(pentafluorethyl)-1,2,4,5,6,7,8,11, 12,13,14,15,16,17-tetradecahydro-spiro[cyclopenta[a]phenanthren-3,2'-[1,3]dioxan]-11-yl]phenyl}pent-2-en-4-innitril (A) und (2Z)-5-{4-[(5R,8S,11R,13S,14S,17S)-5,17-Dihydroxy-5',5',13-trimethyl-17-(pentafluorethyl)-1,2,4,5,6, 7,8,11, 12,13,14,15,16,17-tetradecahydro-spiro[cyclopenta[a]phenanthren-3,2'-[1,3]dioxan]-11-yl]phenyl}pent-2-en-4-innitril (B)

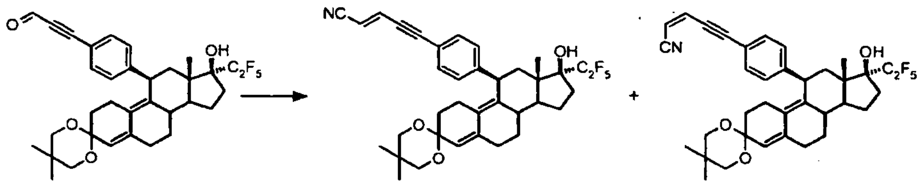

Die Lösung von 0,2 ml Cyanomethanphosphonsäure-diethylester in 5 ml Diethylether versetzte man mit 2,1 ml einer 0,6 molaren Lösung von Bis-(trimethylsilyl)-natriumamid und rührte 30 Minuten bei 23°C. Anschließend tropfte man die Lösung von 500 mg (0,83 mmol) der nach Beispiel 2a dargestellten Verbindung in 3,4 ml Diethylether zu und ließ 1 Stunde reagieren. Man goss in Wasser, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 125 mg (24%) der Titelverbindung A sowie 67 mg (13%) der Titelverbindung B, jeweils als farblosen Schaum.

**Beispiel 5**

**(Z)-5-[4-((8S,11R,13S,14S,17S)-17-Hydroxy-13,methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril**

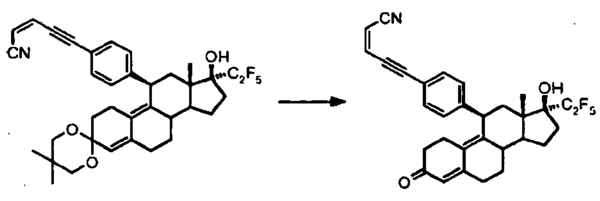

In Analogie zu Beispiel 1 setzte man 10,5 mg (17 μmol) der nach Beispiel 4a dargestellten Verbindung B um

und isolierte nach Aufarbeitung und Reinigung 6,8 mg (62%) der Titelverbindung als farblosen Schaum.

$^1$H-NMR ($CDCl_3$): δ=0,64 (3H), 1,43-1,66 (2H), 1,77-1,92 (3H), 2,06-2,18 (2H), 2,25-2,70 (9H), 2,77 (1H), 4,51 (1H), 5,70 (1H), 5,84 (1H), 6,56 (1H), 7,25 (2H), 7,52 (2H) ppm.

**Beispiel 6**

**(E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-pent-2-en-4-in-säureethylester**

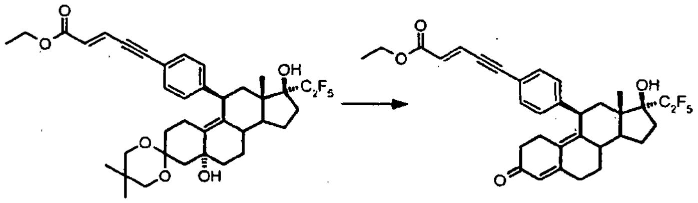

In Analogie zu Beispiel 1 setzte man 56,2 mg (81 μmol) der nach Beispiel 6a dargestellten Verbindung um und isolierte nach Aufarbeitung und Reinigung 24,9 mg (52%) der Titelverbindung als farblosen Schaum.

$^1$H-NMR ($CDCl_3$): δ= 0,63 (3H), 1,36 (3H), 1,45-1,64 (2H), 1,76-1,97 (3H), 2,06-2,20 (2H), 2,27-2,87 (10H), 4, 28 (2H), 4,50 (1H), 5,84 (1H), 6,34 (1H), 7,02 (1H) 7,22 (2H), 7,45 (2H) ppm.

Beispiel 6a

Ethyl-(2E)-5-{4-[(5R,8S,11R,13S,14S,17S)-5,17-dihydroxy-5',5',13-trimethyl-17-(pentafluorethyl)-1,2,4,5,6,7,8,11,12, 13,14,15,16,17-tetradecahydro-spiro[cyclopenta[a]phenanthren-3,2'-[1,3]dioxan]-11-yl]phenyl}pent-2-en-4-inoat

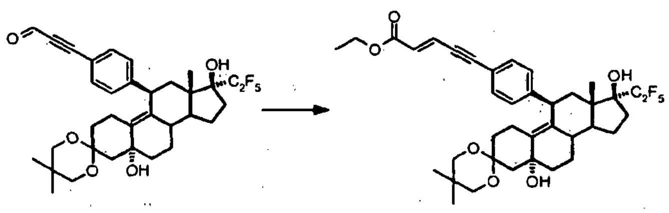

Die Lösung von 34,5 μl Triethylphosphonoacetat in 0,71 ml Tetrahydrofuran versetzte man mit 7,6 mg einer 55%igen Suspension von Natriumhydrid in Weissöl und rührte 15 Minuten bei 23°C. Anschließend tropfte man die Lösung von 70 mg (0,11 mmol) der nach Beispiel 2a dargestellten Verbindung in 1,4 ml Tetrahydrofuran zu und ließ 1 Stunde reagieren. Man versetzte mit Natriumhydrogencarbonatlösung, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 56 mg (72%) der Titelverbindung als farbloser Schaum.

**Beispiel 7**

7-(2E/Z,4E/Z)-[4-((8S,11R,13S,14S,17S)-17-Hydroxy-13-methyl-3-oxo-17-pentafluorethyl-2,3,6,7,8,11,12,13,14,15, 16,17-dodecahydro-1*H*-cyclopenta[a]phenanthren-11-yl)-phenyl]-hepta-2,4-dien-6-inylsäureethylester

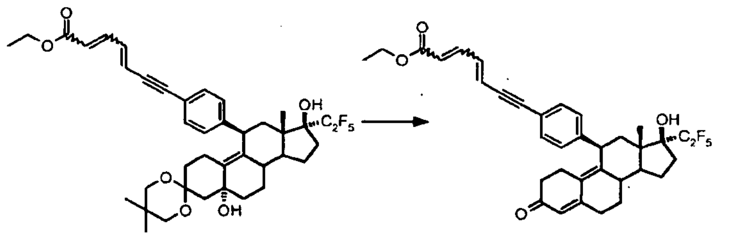

In Analogie zu Beispiel 1 setzte man 11,2 mg (16 μmol) der nach Beispiel 7a dargestellten Verbindungen um und isolierte nach Aufarbeitung und Reinigung 6,0 mg (63%) der isomeren Titelverbindungen als blass gelben Schaum.

$^1$H-NMR ($CDCl_3$): δ= 0,59 (3H), 1,31 (3H), 1,41-1,56 (2H), 1,73-1,86 (3H), 2,01-2,15 (2H), 2,19-2,65 (9H), 2, 72 (1H), 4,23 (2H), 4,45 (1H), 5,79 (1H), 5,94-6,20 (2H), 6,53+6,74 (1 H), 7,16 (2H), 7,30-7,45+7,86 (3H) ppm.

Beispiel 7a

Ethyl-(2E/Z,4E/Z)-7-{4-[(5R,8S,11R,13S,14S,17S)-5,17-dihydroxy-5',5',13-trimethyl-17-(pentafluorethyl)-1,2,4,5,6,7,8, 11,12,13,14,15,16,17-tetradecahydro-spiro[cyclopenta[a]phenanthren-3,2'-[1,3]dioxan]-11-yl]phenyl}hepta-2,4-dien-6-inoat

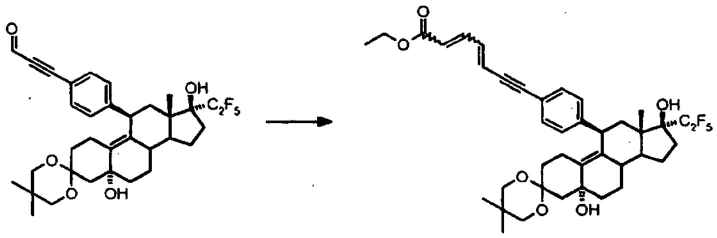

Die Lösung von 101 mg (E)-4-(Diethoxy-phosphoryl)-but-2-ensäureethylester in 2,74 ml Tetrahydrofuran versetzte mit 162 μl einer 1,6 molaren Lösung von n-Butyllithium in Hexan und rührte 45 Minuten bei 23°C. Anschließend kühlte man auf -78°C, tropfte die Lösung von 70 mg (0,11 mmol) der nach Beispiel 2a dargestellten Verbindung in 1,42 ml Tetrahydrofuran zu, entfernte das Kühlbad und ließ 1 Stunde reagieren. Man goss in eine gesättigte Ammoniumchloridlösung, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 11 mg (14%) der Titelverbindung als farbloser Schaum.

**Beispiel 8**

{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-essigsäure-*tert*-butylester

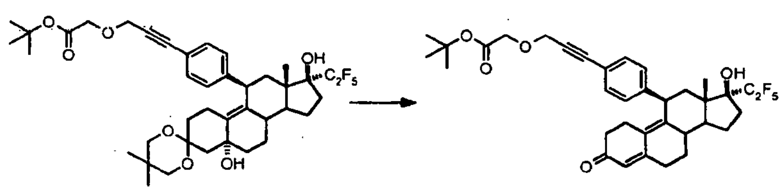

In Analogie zu Beispiel 1 setzte man 50 mg (68 μmol) der nach Beispiel 8a dargestellten Verbindung um und isolierte nach Aufarbeitung und Reinigung 31,3 mg (73%) der Titelverbindung als farblosen Schaum.

$^1$H-NMR ($CDCl_3$): δ= 0,58 (3H), 1,49 (11H), 1,74-1,86 (3H), 2,01 (1H), 2,07 (1H), 2,22-2,64 (9H), 2,72 (1H), 4, 12 (2H), 4,44 (1H), 4,51 (2H), 5,79 (1H), 7,13 (2H), 7,37 (2H) ppm.

Beispiel 8a

tert-Butyl-[(3-{4-[(5R,8S,11R,13S,14S,17S)-5,17-dihydroxy-5',5',13-trimethyl-17-(pentafluorethyl)-1,2,4,5,6,7,8,11,12,13,14,15,16,17-tetradecahydro-spiro[cyclopenta[a]phenanthren-3,2'-[1,3]dioxan]-11-yl]phenyl}prop-2-in-1-yl)oxy]acetat

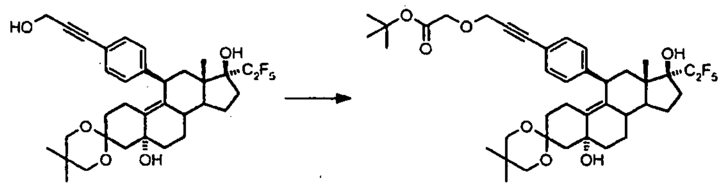

Die Lösung von 200 mg (0,32 mmol) der nach Beispiel 1 a dargestellten Verbindung in 2 ml Dichlormethan versetzte man mit 0,26 ml Bromessigsäure-tert.-butylester, 0,75 ml einer 50%igen Kaliumhydroxidlösung, 4,25 mg Tetrabutylammoniumhydrogensulfat und rührte 1 Stunde bei 23°C. Man verdünnte mit Wasser und Dichlormethan, säuerte durch Zugabe einer 4 molaren Salzsäure an und extrahierte mit Dichlormethan. Die vereinigten organischen Extrakte trocknete man über Natriumsulfat. Den nach Filtration und Lösungsmittelabzug erhaltenen Rückstand reinigte man durch Chromatographie. Isoliert wurden 206 mg (87%) der Titelverbindung als farbloser Schaum.

**Beispiel 9**

{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-essigsäuremethylester

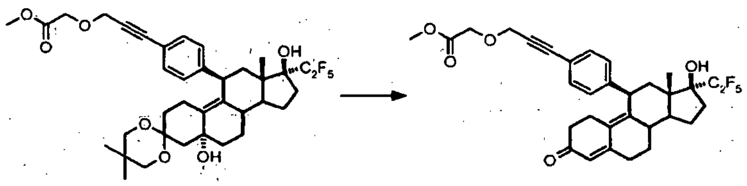

In Analogie zu Beispiel 1 setzte man 32,2 mg (46 μmol) der nach Beispiel 9a dargestellten Verbindung um und isolierte nach Aufarbeitung und Reinigung 18,7 mg (68%) der Titelverbindung als farblosen Schaum.

$^1$H-NMR ($CDCl_3$): δ= 0,58 (3H), 1,39-1,56 (2H), 1,73-1,87 (3H), 2,06 (1H), 2,20-2,64 (10H), 2,71 (1H), 3,77 (3H), 4,26 (2H), 4,43 (1H), 4,51 (2H), 5,78 (1H), 7,13 (2H), 7,36 (2H)ppm.

Beispiel 9a

Methyl-[(3-{4-[(5R,8S,11R,13S,14S,17S)-5,17-dihydroxy-5',5',13-trimethyl-17-(pentafluorethyl)-1,2,4,5,6,7,8,11,12,13,14,15,16,17-tetradecahydro-spiro[cyclopenta[a]phenanthren-3,2'-[1,3]dioxan]-11-yl]phenyl}prop-2-in-1-yl)oxy]acetat

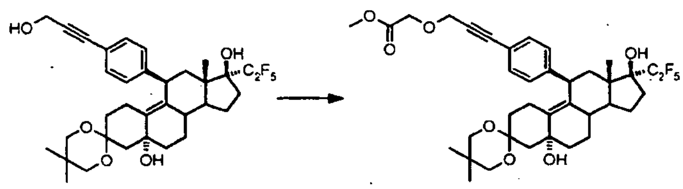

In Analogie zu Beispiel 8 setzte man 50 mg (80 μmol) der nach Beispiel 1a dargestellten Verbindung um und isolierte nach Aufarbeitung und Reinigung 32 mg (58%) der Titelverbindung als farblosen Schaum.

Beispiel 10

{3-[4-((8S,11R,13S,14S,17S)-17-Hydroxy-13-methyl-3-oxo-17-pentafluorethy)-2,3,6,7,8,11,12,13,14,15,16,17-dodecahydro-1*H*-cyclopenta[*a*]phenanthren-11-yl)-phenyl]-prop-2-inyloxy}-essigsäure

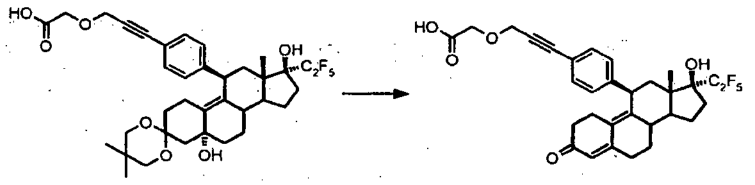

In Analogie zu Beispiel 1 setzte man 25 mg (37 μmol) der nach Beispiel 10a dargestellten Verbindung um und isolierte nach Aufarbeitung und Reinigung 3,2 mg (15%) der Titelverbindung als farblosen Schaum.

$^1$H-NMR ($CDCl_3$): δ= 0,58 (3H), 1,69-1,56 (2H), 1,73-1,87 (3H), 2,01-2,11 (2H), 2.19-2,64 (9H), 2,71 (1H), 4, 30 (2H), 4,44 (1H), 4,54 (2H), 5,80 (1H), 7,14 (2H), 7,36 (2H) ppm.

Beispiel 10a

[(3-{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydro-spiro[cyclopenta[a]phenanthren-3,2'-[1,3]dioxan]-11-yl]phenyl}prop-2-in-1-yl)oxy]essigsäure

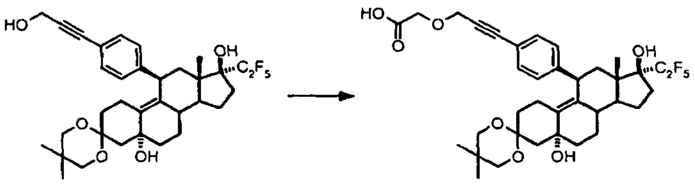

In Analogie zu Beispiel 8 setzte man 50 mg (80 μmol) der nach Beispiel 1a dargestellten Verbindung unter Verwendung von Bromessigsäuremethyester um und isolierte nach Aufarbeitung und Reinigung 45 mg (83%) der Titelverbindung als farblosen Schaum.

**Beispiel 11**

(8S,11R,13S,14S,17S)-17-Hydroxy-11-{4-[3-(2-hydroxy-ethoxy)-prop-1-in-1-yl]-phenyl}-13-methyl-17-pentafluorethyl-1,2,6,7,8,11,12,13,14,15,16,17-dodecahydro-cyclopenta[a]phenanthren-3-on

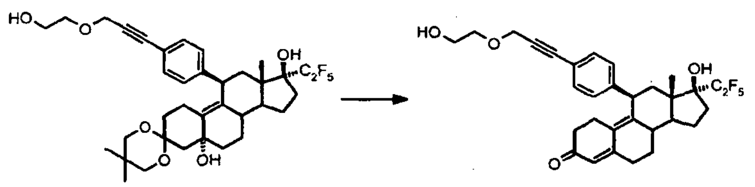

In Analogie zu Beispiel 1 setzte man 15 mg (22 μmol) der nach Beispiel 11a dargestellten Verbindung um und isolierte nach Aufarbeitung und Reinigung 8,4 mg (66%) der Titelverbindung als farbloses Öl.

$^1$H-NMR ($CDCl_3$): δ= 0,58 (3H), 1,40-1,54 (2H), 1,74-1,85 (3H), 1,93-2,12 (3H), 2,21-2,64 (9H), 2,72 (1H), 3, 71 (2H), 3,80 (2H), 4,42 (2H), 4,44 (1H), 5,79 (1H), 7,13 (2H), 7,37 (2H) ppm.

Beispiel 11 a

(5R,8S,11R,13S,14S,17S)-11-{4-[3-(2-Hydroxyethoxy)prop-1-in-1-yl]phenyl}-5',5',13-trimethyl-17-(pentafluorethyl)-1, 2,3,4,6,7,8,11,12,13,14,15,16,17-tetradecahydro-spiro[cyclopenta[a]phenanthren-3,2'-[1,3]dioxan]-5,17-diol

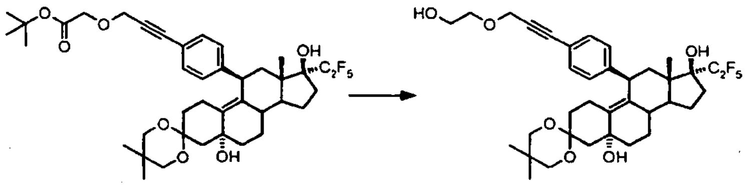

Die Lösung von 97 mg (0,13 mmol) der nach Beispiel 8a dargestellten Verbindung in 1,95 ml Toluol versetzte man bei -70°C mit 0,66 ml einer 1 molaren Lösung von Diisobutylaluminiumhydrid in Toluol. Nach 1 Stunde goss man in Wasser, säuerte durch Zugabe einer 1 molaren Salzsäure an, extrahierte mehrfach mit Ethylacetat, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 50 mg (56%) der Titelverbindung als farbloser Schaum.

**Beispiel 12**

3-{(E/Z)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäuremethylester

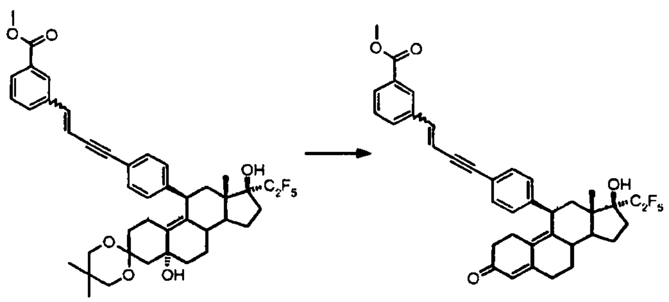

In Analogie zu Beispiel 1 setzte man 83,3 mg (0,11 mmol) eines Gemisches der nach Beispiel 12a dargestellten Verbindungen um und isolierte nach Aufarbeitung und Reinigung 46,8 mg (65%) der Titelverbindungen als blass gelben Schaum.

$^1$H-NMR ($CDCl_3$): $\delta$= 0,61 (3H), 1,40-1,56 (2H), 1,73-1,88 (3H), 2,01-2,13 (2H), 2,22-2,65 (9H), 2,73 (1H), 3, 92+3,93 (3H), 4,46 (1H), 5,79 (1H), 5,98+6,46 (1H), 6,72+7,05 (1H), 7,17 (2H), 7,36-8,05 (5H), 8,10+8,75 (1H) ppm.

Beispiel 12a

3-{(E/Z)-4-[4-((5R,8S,11R,13S,14S,17S)-5,17-Dihydroxy-5',5',13-trimethyl-17-pentafluorethyl-2,3,4,5,6,7,8,11,12,13, 14,15,16,17-tetradecahydro-1*H*-spiro[cyclopenta[*a*]phenanthren-3,2'-[1,3]dioxan]-11-yl)-phenyl]-but-1-en-3-inyl}-benzoesäuremethylester

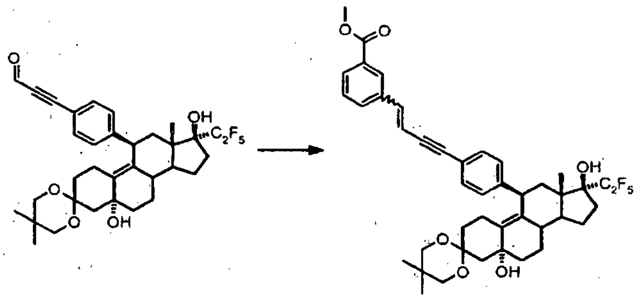

Die Lösung von 406 mg 3-Methoxycarbonyltriphenylphosphoniumbromid in 2 ml Toluol versetzte man bei 3°C mit 1,4 ml einer 0,6 molaren Lösung von Bis-(trimethylsilyl)-natriumamid in Toluol und rührte 60 Minuten bei 23°C. Anschließend tropfte man die Lösung von 100 mg (0,16 mmol) der nach Beispiel 2a dargestellten Verbindung in 2 ml Toluol zu und ließ 1 Stunde reagieren. Man goss in Wasser, extrahierte mehrfach mit Ethylacetat und trocknete die vereinigten organischen Extrakte über Natriumsulfat. Den nach Filtration und Lösungsmittelabzug erhaltenen Rückstand reinigte man durch Chromatographie. Isoliert wurden 82 mg (67%) der Titelverbindungen als farblosen Schaum.

**Beispiel 13 (A + B)**

(A) 3-{(E)-4-[4-((8S,11R,13S,14S,17S)-17-Hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-but-1-en-3-inyl}-benzoesäure und

(B) 3-{(Z)-4-[4-((8S,11R,13S,14S,17S)-17-Hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-but-1-en-3-inyl}-benzoesäure

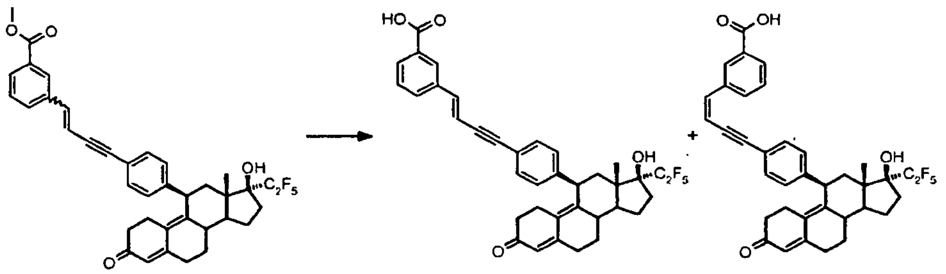

Die Lösung von 42 mg (65 μmol) der nach Beispiel 12 dargestellten Verbindung in 0,68 ml Tetrahydrofuran versetzte man mit 0,29 ml einer 5%igen wäßrigen Lithiumhydroxidlösung und rührte 16 Stunden bei 23°C. Man säuerte durch Zugabe einer 1 molaren Salzsäure an, sättigte mit Natriumchlorid, extrahierte mehrfach mit Diethylether und trocknete die vereinigten organischen Extrakte über Natriumsulfat. Den nach Filtration und Lösungsmittelabzug erhaltenen Rückstand reinigte man durch Chromatographie. Isoliert wurden jeweils 8,1 mg (20%) der Titelverbindungen als farblosen Schaum.

$^1$H-NMR ($CDCl_3$) von A: δ= 0,60 (3H), 1,42-1,54 (2H), 1,74-1,86 (3H), 2,07 (1 H), 2,24-2,65 (10H), 2,74 (1H), 4,45 (1 H), 5,81 (1H), 6,47 (1 H), 7,06 (1H), 7,16 (2H), 7,40 (2H), 7,46 (1H), 7,65 (1H), 8,03 (1H), 8,16 (1H) ppm.

$^1$H-NMR ($CDCl_3$) von B: δ= 0,51 (3H), 1,38-1,54 (2H), 1,72-1,86 (3H), 1,97-2,08 (2H), 2,16-2,63 (10H), 2,69 (1H), 4,43 (1H), 5,74 (1H), 6,01 (1H), 6,74 (1H), 7,15 (2H), 7,50 (1H), 7,54 (2H), 7,86 (1H), 8,05 (1 H), 9,14 (1H) ppm.

**Beispiel 14**

**(3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-acetonitril**

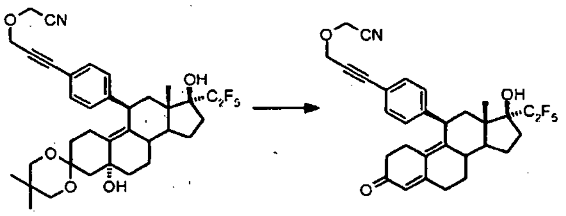

In Analogie zu Beispiel 1 setzte man 104 mg (160 μmol) der nach Beispiel 14a dargestellten Verbindung um und isolierte nach Aufarbeitung und Reinigung 81 mg (93%) der Titelverbindung als farblosen Schaum.

$^1$H-NMR ($CDCl_3$): δ= 0,58 (3H), 1,38-1,56 (2H), 1,71-1,88 (3H), 2,05 (1H), 2,07 (1H), 2,18-2,65 (9H), 2,72 (1H), 4,43 (3H), 4,54 (2H), 5,79 (1H), 7,16 (2H), 7,39 (2H) ppm.

Beispiel 14a

{3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[a]phenanthren-3,2'-[1,3]dioxan]-11-yl)-phenyl]-prop-2-ynyloxy}-acetonitril

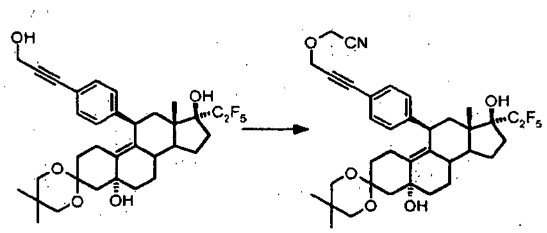

In Analogie zu Beispiel 8 setzte man 250 mg (0,40 mmol) der nach Beispiel 1a dargestellten Verbindung unter Verwendung von Bromacetonitril um und isolierte nach Aufarbeitung und Reinigung 186 mg (70%) der Titelverbindung als farblosen Schaum.

**Beispiel 15**

4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester

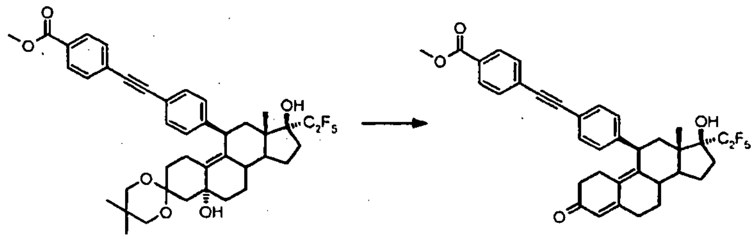

In Analogie zu Beispiel 1 setzte man 14 mg (19 μmol) der nach Beispiel 15a dargestellten Verbindung um und isolierte nach Aufarbeitung und Reinigung 7,7 mg (63%) der Titelverbindung als farblosen Schaum.

$^1$H-NMR ($CDCl_3$): δ=0,61 (3H), 1,38-1,57 (2H), 1,73-1,90 (3H), 2,08 (1H), 2,14 (1H), 2,20-2,68 (9H), 2,74 (1 H), 3,92 (3H), 4,47 (1H), 5,80 (1 H), 7,19 (2H), 7,46 (2H), 7,57 (2H), 8,01 (2H) ppm.

Beispiel 15a

4-[4-((5R,8S,11R,13S,14S,17S)-5,17-Dihydroxy-5',5',13-trimethyl-17-pentafluocethyl-2,3,4,5,6,7,8,11,12,13,14,15,16,17-tetradecahydro-1*H*-spiro[cyclopenta[a]phenanthren-3,2'-[1,3]dioxan]-11-yl)-phenylethynyl]-benzoesäuremethylester

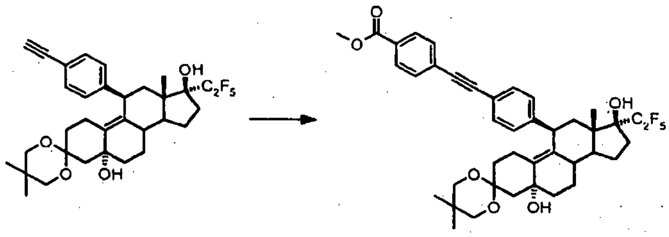

Die Lösung von 50 mg (84 μmol) der nach Beispiel 15b dargestellten Verbindung in 8 ml Tetrahydrofuran versetzte man mit 14,4 mg 4-Chlorbenzoesäuremethylester, 3,4 ml Triethylamin, Tetrakis(triphenylphosphin)palladium(0), 1,3 mg Kupfer(I)iodid, 2,4 mg und rührte 3 Stunden bei 23°C. Man engte ein und reinigte den Rückstand durch Chromatographie. Isoliert wurden 25 mg (41%) der Titelverbindung als farbloser Schaum.

Beispiel 15b

(5R,8S,11R,13S,14S,17S)-11-(4-Ethinyl-phenyl)-5',5',13-trimethyl-17-pentafluorethyl-1,2,3,4,6,7,8,11,12,13,14,15,16,17-tetradecahydro-1*H*-spiro[cyclopenta[a]phenanthren-3,2'-[1,3]dioxan]-5,17-diol

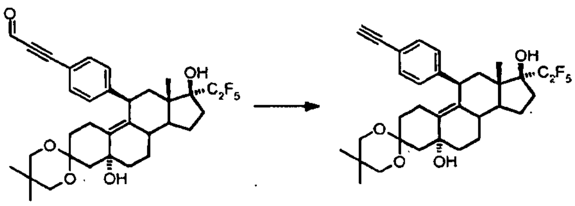

Die Lösung von 1,64 g (2,63 mmol) der nach Beispiel 2a dargestellten Verbindung in 30 ml Tetrahydrofuran versetzte man mit 2 ml einer 50%igen Kaliumhydroxidlösung und rührte 2 Stunden bei 60°C. Man verdünnte mit Wasser, säuerte durch Zugabe einer 1 molaren Salzsäure an und extrahierte mehrfach mit Ethylacetat. Die vereinigten organischen Extrakte wusch man mit gesättigter Natriumhydrogencarbonatlösung und gesättigter Natriumchloridlösung und trocknete über Natriumsulfat. Den nach Filtration und Lösungsmittelabzug erhaltenen Rückstand reinigte man durch Chromatographie. Isoliert wurden 1,34 g (86%) der Titelverbindung als farblosen Schaum.

**Beispiel 16**

4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure

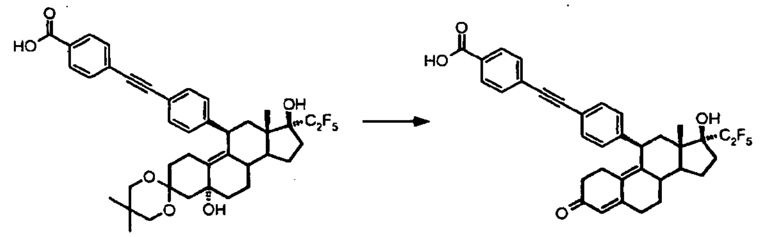

In Analogie zu Beispiel 1 setzte man 28 mg (38 μmol) der nach Beispiel 16a dargestellten Verbindung um und isolierte nach Aufarbeitung und Reinigung 17 mg (74%) der Titelverbindung als farblosen Schaum.

$^1H$-NMR ($CD_3OD$): δ=0,60 (3H), 1,37-1,56 (2H), 1,71-1,84 (3H), 2,10 (1H), 2,20-2,48 (5H), 2,56-2,73 (4H), 2, 82 (1H), 4,55 (1H), 5,74 (1H), 7,27 (2H), 7,45 (2H), 7,47 (2H), 7,91 (2H) ppm.

Beispiel 16a

4-(4-((5R,8S,11R,13S,14S,17S)-5,17-Dihydroxy-5',5',13-trimethyl-17-pentafluorethyl-2,3,4,5,6,7,8,11,12,13,14,15,16, 17-tetradecahydro-1*H*-spiro[cyclopenta[a]phenanthren-3,2'-[1,3]dioxan]-11-yl)-phenylethynyl)-benzoesäure

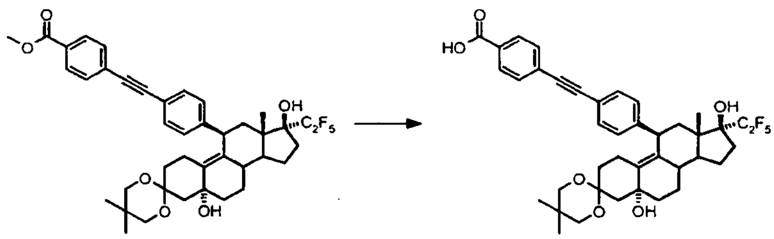

In Analogie zu Beispiel 13 setzte man 28 mg (38 μmol) der nach Beispiel 15a dargestellten Verbindung um und isolierte nach Aufarbeitung und Reinigung 26 mg (95%) der Titelverbindung als farblosen Schaum.

**Beispiel 17**

**3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenylethinyl]-benzoesäuremethylester**

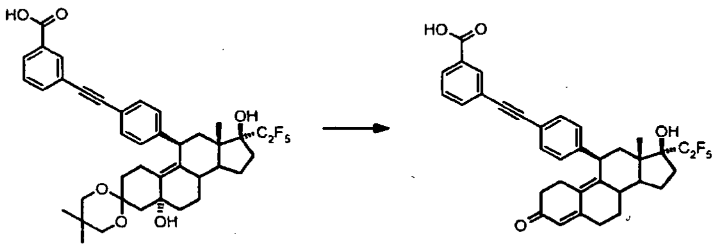

In Analogie zu Beispiel 1 setzt man 459 mg (690 μmol) der nach Beispiel 17a dargestellten Verbindung um und isoliert nach Aufarbeitung und Reinigung 307 mg (73%) der Titelverbindung als farblosen Schaum.

$^1H$-NMR ($CD_3OD$): δ=0,60 (3H), 1,35-1,59 (2H), 1,70-1,84 (3H), 2,10 (1H), 2,18-2,49 (5H), 2,55-2,75 (4H), 2, 82 (1H), 4,55 (1H), 5,74 (1H), 7,26 (2H), 7,36-7,49 (3H), 7,59 (1 H), 7,93 (1H), 8,07 (1H) ppm.

Beispiel 17a

3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[a]phenanthren-3,2'-[1,3]dioxan]-11-yl)-phenylethynyl]-benzoesäure

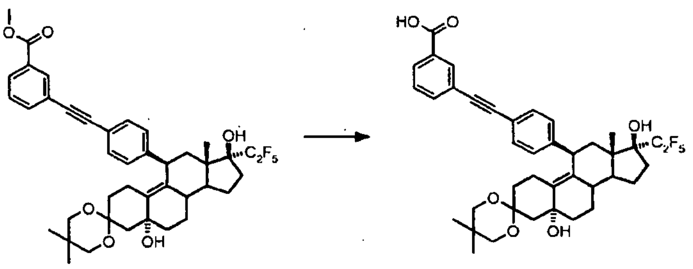

In Analogie zu Beispiel 13 setzte man 19 mg (26 μmol) der nach Beispiel 17b dargestellten Verbindung um und isolierte nach Aufarbeitung und Reinigung 18 mg (97%) der Titelverbindung als farblosen Schaum.

Beispiel 17b

3-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[a]phenanthren-3,2'-[1,3]dioxan]-11-yl)-phenylethynyl]-benzoesäuremethylester

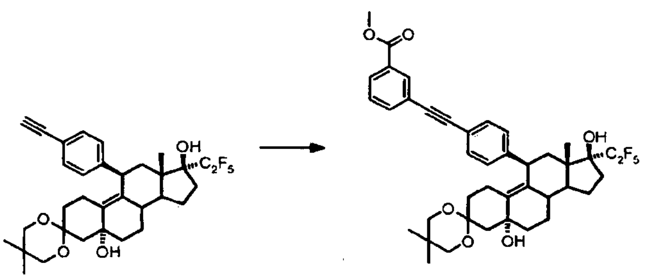

Die Mischung aus 2,3 mg Bis-acetonitril-palladium(II)chlorid, 6 mg 2-Dicyclohexylphosphino-2',4',6'-triisopropylphenyl und 82,5 mg Cäsiumcarbonat in 0,75 ml Acetonitril versetzte man bei 23°C mit 17 μl 3-Chlorbenzoesäuremethylester und nach 30 Minuten mit der Lösung von 75 mg (0,13 mmol) der nach Beispiel 15b dargestellten Verbindung in 0,75 ml Acetonitril. Man ließ 2 Stunden bei 70°C reagieren, versetzte mit Wasser und extrahierte mehrfach mit Ethylacetat. Die vereinigten organischen Extrakte trocknete man über Natriumsulfat und reinigt den nach Filtration und Lösungsmittelabzug erhaltenen Rückstand durch Chromatographie. Isoliert wurden 19 mg (21%) der Titelverbindung als farbloser Schaum.

**Beispiel 18**

**(8S,11 R,13S,14S,17S)-17-Hydroxy-13-methyl-17-pentafluorethyl-11-{4-[3-(1*H*-tetrazol-5-ylmethoxy)-prop-1-in-1-yl]-phenyl}-1,2,6,7,8,11,1,2,13,14,15,16,17-dodecahydro-cyclopenta[a]phenanthren-3-on**

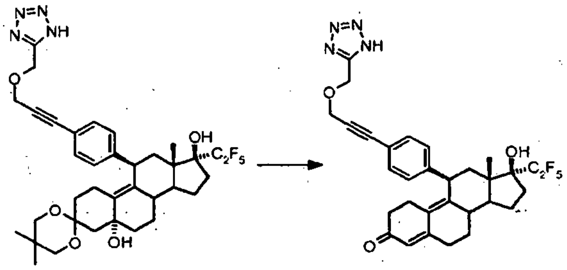

In Analogie zu Beispiel 1 setzte man 426 mg (200 µmol) der nach Beispiel 18a dargestellten Verbindung um und isolierte nach Aufarbeitung und Reinigung 44 mg (37%) der Titelverbindung als farblosen Schaum.

$^1$H-NMR ($CD_3OD$): δ=0,57 (3H), 1,36-1,56 (2H), 1,69-1,83 (3H), 2,09 (1H), 2,16-2,47 (5H), 2,54-2,72 (4H), 2, 80 (1H), 4,50-4,56 (3H), 4,99 (2H), 5,74 (1H), 7,22 (2H), 7,33 (2H) ppm.

Beispiel 18a

(5R,8S,11R,13S,14S,17S)-5',5',13-trimethyl-17-pentafluorethyl-11-{4-[3-(1H-tetrazol-5-ylmethoxy)-prop-1-ynyl]-phenyl}-1,2,3,4,6,7,8,11,12,13,14,15,16,17-tetradecahydro-1*H*-spiro[cyclopenta[a]phenanthren-3,2'-[1,3]dioxan]-5,17-diol

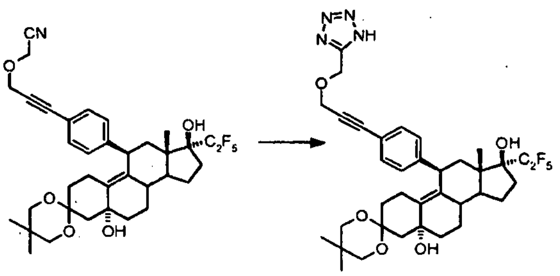

Die Lösung von 20 mg (30 µmol) der nach Beispiel 14a dargestellten Verbindung in 0,4 ml Dimethylformamid versetzte man mit 10 mg Natriumazid, 1 mg Ammoniumchlorid und erhitzte 16 Stunden auf 120°C. Man goss in Wasser, extrahierte mehrfach mit Ethylacetat, wusch die vereinigten organischen Extrakte mit Wasser und gesättigter Natriumchloridlösung und trocknete über Natriumsulfat. Den nach Filtration und Lösungsmittelabzug erhaltenen Rückstand reinigt man durch Chromatographie. Isoliert wurden 11,5 mg (54%) der Titelverbindung als farbloser Schaum.

**Beispiel 19**

**4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure**

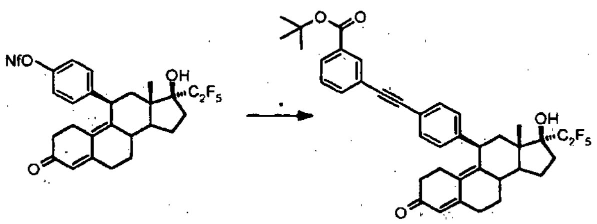

Die Lösung von 1 g (1,31 mmol) der nach Beispiel 19a dargestellten Verbindung in 7 ml Acetonitril versetzte man mit 350 mg 4-Ethinyl-benzoesäure-tert.-butylester, 364 µl Triethylamin, 11 mg Palladiumdichlorid, 34 mg Triphenylphosphin, 6 mg Kupfer(I)iodid und erhitzte 2 Stunden auf 80°C. Man goss in eine gesättigte Ammoniumchloridlösung, extrahierte mehrfach mit Ethylacetat, wusch die vereinigten organischen Extrakte mit gesättigter Natriumchloridlösung und trocknete über Natriumsulfat. Den nach Filtration und Lösungsmittelabzug erhaltenen Rückstand reinigte man durch

Chromatographie. Isoliert wurden 318 mg (36%) der Titelverbindung als farbloser Schaum.

$^1$H-NMR ($CDCl_3$): δ= 0,60 (3H), 1,36-1,55 (2H), 1,60 (9H), 1,74-1,87 (3H), 2,01-2,14 (2H), 2,22-2,66 (9H), 2, 74 (1 H), 4,46 (1H), 5,80 (1H), 7,18 (2H), 7,47 (2H), 7,55 (2H), 7,96 (2H) ppm.

Beispiel 19a

1,1,2,2,3,3,4,4,4-Nonafluor-butan-1-sulfonsäure 4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl ester

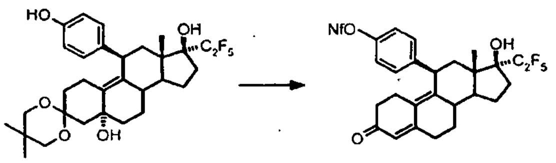

Die Lösung von 17,1 g (27,2 mmol) (5R,8S,11R,13S,14S,17S)-11-(4-Hydroxy-phenyl)-5',5',13-trimethyl-17-pentafluorethyl-2,3,4,6,7,8,11,12,13,14,15,16,17-tetradecahydro-1*H*-spiro[cyclopenta[a]phenanthren-3,2'-[1,3]dioxan]5,17-diol in 380 ml Tetrahydrofuran kühlt man auf -3°C, versetzte mit 25,5 ml einer 1,6 molaren Lösung von Butyllithium in Hexan und nach 30 Minuten mit 9,78 ml Nonafluorbutansulfonylfluorid. Man rührte 1,5 Stunden bei 0°C, goss auf gesättigte Natriumhydrogencarbonatlösung, extrahierte mehrfach mit Ethylacetat, wusch die vereinigten organischen Extrakte mit Wasser und gesättigter Natriumchloridlösung und trocknete über Natriumsulfat. Den nach Filtration und Lösungsmittelabzug erhaltenen Rückstand reinigte man durch Kristallisation Isoliert wurden 18,6 g (89%) der Titelverbindung als farbloser Feststoff.

**Beispiel 20**

3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure

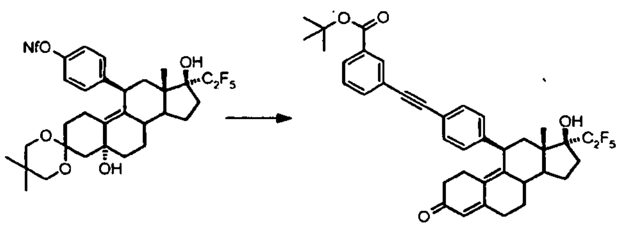

In Analogie zu Beispiel 19 setzte man 2 g (2,62 mmol) der nach Beispiel 19a dargestellten Verbindung unter Verwendung von 3-Ethinyl-benzoesäure-tert.-butylester um und isolierte nach Aufarbeitung und Reinigung 478 mg (27%) der Titelverbindung als farblosen Schaum.

$^1$H-NMR ($CDCl_3$): δ= 0,61 (3H), 1,40-1,65 (2H), 1,60 (9H), 1,74-1,88 (3H), 2,02-2,13 (2H), 2,22-2,67 (9H), 2, 74 (1H), 4,47 (1H), 5,80 (1H), 7,18 (2H), 7,40 (1H), 7,46 (2H), 7,66 (1H), 7,94 (1H), 8,12 (1H) ppm.

**Beispiel 21**

**(8S,11R,13S,14S,17S)-17-Hydroxy-11-[4-(3-hydroxy-3-methyl-but-1-inyl)-phenyl]-13-methyl-17-pentafluorethyl-1,2,6,7,8,11,12,13,14,15,16,17-dodecahydro-cyclopenta[*a*]phenanthren-3-on**

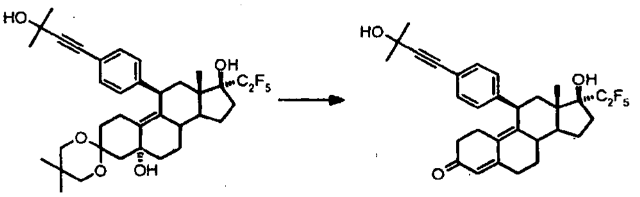

In Analogie zu Beispiel 1 setzte man 103 mg (160 μmol) der nach Beispiel 21a dargestellten Verbindung um und isolierte nach Aufarbeitung und Reinigung 57 mg (66%) der Titelverbindung als farblosen Schaum.

$^1$H-NMR ($CDCl_3$): δ= 0,57 (3H), 1,38-1,56 (2H), 1,61 (6H), 1,72-1,86 (3H), 2,03 (1H), 2,06 (1H), 2,08 (1H), 2, 19-2,65 (9H), 2,71 (1H), 4,43 (1H), 5,79 (1H), 7,11 (2H), 7,34 (2H) ppm.

Beispiel 21 a

(5R,8S,11R,13S,14S,17S)-11-[4-(3-Hydroxy-3-methyl-but-1-ynyl)-phenyl]-5',5',13-trimethyl-17-pentafluorethyl-2,3,4,6, 7,8,11,12,13,14,15,16,17-tetradecahydro-1*H*-spiro[cyclopenta[a]phenanthren-3,2'-[1,3]dioxan-5,17-diol

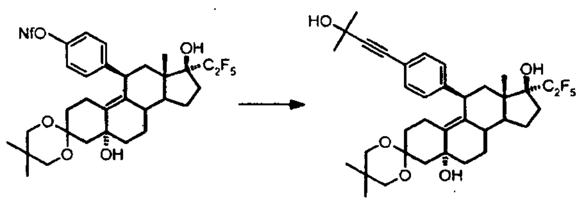

In Analogie zu Beispiel 1a setzte man 500 mg (0,58 mmol) 1,1,2,2,3,3,4,4,4-Nonafluor-butan-1-sulfonsäure 4-((5R,8S,11R,13S,14S,17S)-5,17-dihydroxy-17-pentafluorethyl-5',5',13-trimethyl-2,3,4,6,7,8,11,12,13,14,15,16,17-tetradecahydro-1*H*-spiro[cyclopenta[a]phenanthren-3,2'-[1,3]dioxan-11-yl)-phenyl ester unter Verwendung von 2-Methyl-but-3-in-2-ol um und isolierte nach Aufarbeitung und Reinigung 103 mg (27%) der Titelverbindung als farblosen Schaum.

**Beispiel 22**

(8S,11R,13S,14S,17S)-11-(4-Ethinyl-phenyl)-17-hydroxy-13-methyl-17-pentafluorethyl-1,2,6,7,8,11,12,13,14,15,16, 17-dodecahydro-cyclopenta[a]phenanthren-3-on

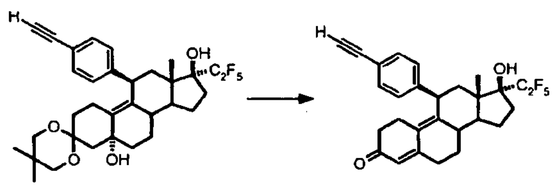

In Analogie zu Beispiel 1 setzte man 13 mg (22 μmol) der nach Beispiel 22a dargestellten Verbindung um und isolierte nach Aufarbeitung und Reinigung 8 mg (74%) der Titelverbindung als farblosen Schaum.

$^1$H-NMR ($CDCl_3$): δ= 0,58 (3H), 1,41-1,55 (2H), 1,74-1,86 (3H), 2,07 (1H), 2,11 (1H), 2,21-2,64 (9H), 2,72 (1 H), 3,06 1(H), 4,44 (1H), 5,79 (1H), 7,14 (2H), 7,41 (2H) ppm.

Beispiel 22a

(5R,8S,11R,13S,14S,17S)-11-[4-Ethinyl-phenyl]-5',5',13-trimethyl-17-pentafluorethyl-2,3,4,6,7,8,11,12,13,14,15,16,17-tetradecahydro-1*H*-spiro[cyclopenta[a]phenanthren-3,2'-[1,3]dioxan-5,17-diol

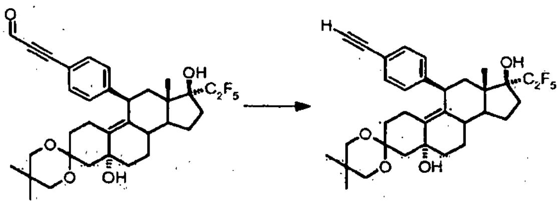

Die Lösung von 1,64 g (2,63 mmol) der nach Beispiel 2a dargestellten Verbindung in 30 ml Tetrahydrofuran versetzte man mit 2 ml einer 50%igen wäßrigen Kaliumhydroxidlösung und rührte 2 Stunden bei 60°C. Man säuerte durch Zugabe einer 1 molaren Salzsäure an, extrahierte mehrfach mit Ethylacetat, wusch die vereinigten organischen Extrakte mit gesättigter Natriumhydrogencarbonat- und Natriumchloridlösung und trocknete über Natriumsulfat. Den nach Filtration und Lösungsmittelabzug erhaltenen Rückstand reinigte man durch Chromatographie. Isoliert wurden 1, 34 g (86%) der Titelverbindung als farbloser Schaum.

**Beispiel 23**

**(8S,11R,13S,14S,17S)-11-[4-(3-Azido-prop-1-in-1-yl)-phenyl]-17-hydroxy-13-methyl-17-pentafluorethyl-1,2,6,7,8,11,12,13,14,15,16,17-dodecahydro-cyclopenta[a]phenanthren-3-on**

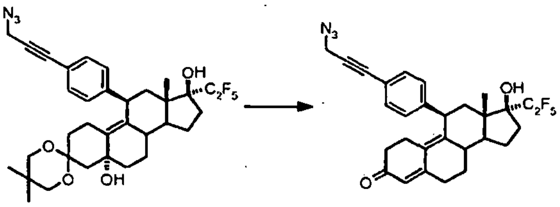

In Analogie zu Beispiel 1 setzte man 21,7 mg (33 μmol) der nach Beispiel 23a dargestellten Verbindung um und isolierte nach Aufarbeitung und Reinigung 14,5 mg (80%) der Titelverbindung als farblosen Schaum.

$^1$H-NMR ($CDCl_3$): $\delta$= 0,59 (3H), 1,38-1,56 (2H), 1,71-1,88 (3H), 2,07 (1H), 2,17-2,64 (10H), 2,72 (1H), 4,14 (2H), 4.44 (1H), 5,79 (1H), 7,15 (2H), 7,39 (2H) ppm.

Beispiel 23a

(5R,8S,11R,13S,14S,17S)-11-[4-(3-Azido-prop-1-in-1-yl)-phenyl]-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan-5,17=diol

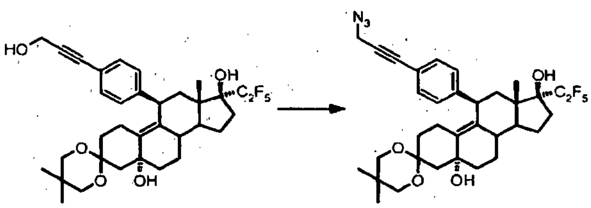

Die Lösung von 150 mg (0,24 mmol) der nach Beispiel 1 a dargestellten Verbindung in 3,6 ml Tetrahydrofuran

versetzte man bei 3°C mit 0,1 ml Diphenylphosphorylazid, 42 µl 1,8-Diazabicyclo[5.4.0]undec-7-en und ließ 2 Stunden bei 23°C reagieren. Man versetzte mit Wasser, extrahierte mehrfach mit Ethylacetat, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 114 g (73%) der Titelverbindung als farbloser Schaum.

**Beispiel 24 (A+B)**

**(A) (E)-[4-((8S,11 R,13S,14S,17S)-17-Hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-propinal *O*-benzyl-oxim und**

(B) (Z)-[4-((8S,11 R,13S,14S,17S)-17-Hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-propinal *O*-benzyl-oxim

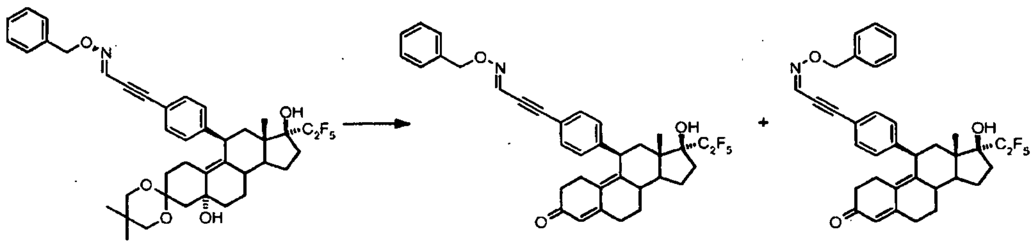

Die Lösung von 52 mg (71 mmol) der nach Beispiel 24a dargestellten Verbindungen in 1,2 ml Aceton versetzte man mit 0,11 ml einer 4N Salzsäure und rührte 20 Minuten bei 23°C. Man versetzte mit 59 µl Triethylamin, engte ein und reinigte den nach Filtration und Lösungsmittelabzug erhaltenen Rückstand durch Chromatographie. Isoliert wurden 14 mg (31%) der Titelverbindung A sowie 14,5 mg (33%) der Titelverbindung B jeweils als farbloser Schaum.

$^{1}$H-NMR ($CDCl_3$) von A: δ= 0,57 (3H), 1,38-1,56 (2H), 1,70-1,88 (3H), 2,08 (1H), 2,12 (1H), 2,19-2,65 (9H), 2, 71 (1H), 4,44 (1H), 5,20 (2H), 5,79 (1H), 7,16 (2H), 7,30-7,47 (7H), 7,60 (1H) ppm.

$^{1}$H-NMR ($CDCl_3$) von B: δ= 0,57 (3H), 1,38-1,57 (2H), 1,72-1,88 (3H), 2,07 (1 H), 2,17-2,64 (10H), 2,71 (1H), 4,44 (1H), 5,26 (2H), 5,79 (1H), 6,93 (1H), 7,17 (2H), 7,28-7,48 (7H) ppm.

Beispiel 24a

(E/Z)-[4-((5R,8S,11R,13S,14S,17S)-5,17-Dihydroxy-5',5',13-trimethyl-17-pentafluorethyl-2,3,4,5,6,7,8,11,12,13,14,15, 16,17-tetradecahydro-1*H*-spiro[cyclopenta[*a*]phenanthren-3,2'-[1,3]dioxan]-11-yl)-phenyl]-propinal *O*-benzyl-oxim

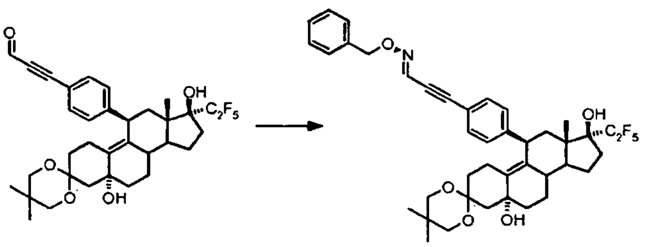

Die Lösung von 10,8 mg *O*-Benzylhydroxylamin in 1 ml Ethanol versetzte man mit 50 mg (80 µmol) der nach Beispiel 2a dargestellten Verbindung, rührte 2 Stunden bei 23°C, engte ein und reinigte den Rückstand durch Chromatographie. Isoliert wurden 52 mg (89%) der Titelverbindungen als farbloser Schaum.

**Beispiel 25 (A+B)**

**(A) (E)-[4-((8S,11R,13S,14S,17S)-17-Hydroxy-13-methyl-3-oxo-17-pentafluorethyl-2,3,6,7,8,11,12,13,14,15,**

**16,17-dodecahydro-1*H*-cyclopenta[*a*]phenanthren-11-yl)-phenyl]-propinal *O*-ethyl-oxim und**
**(B) (Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-ethyl-oxim**

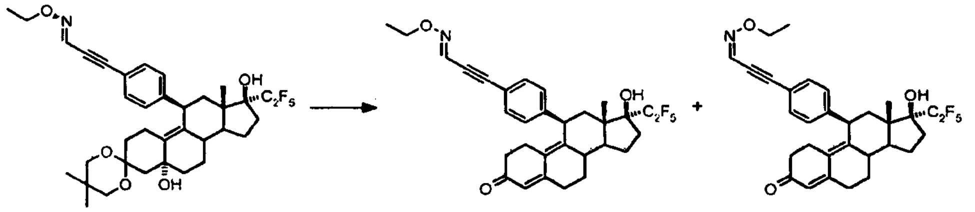

In Analogie zu Beispiel 24 setzte man 47 mg (71 μmol) der nach Beispiel 25a dargestellten Verbindungen um und isolierte nach Aufarbeitung und Reinigung 9 mg (23%) der Titelverbindung A sowie 12 mg (30%) der Titelverbindung B jeweils als farblosen Schaum.

$^{1}$H-NMR ($CDCl_3$) von A: δ= 0,57 (3H), 1,30 (3H), 1,39-1,56 (2H), 1,71-1,87 (3H), 2,08 (1 H), 2,12 (1 H), 2,19-2,65 (9H), 2,72 (1 H), 4,22 (2H), 4,44 (1 H), 5,79 (1H), 7,16 (2H), 7,43 (2H), 7,53 (1H) ppm.

$^{1}$H-NMR ($CDCl_3$) von B: δ= 0,57 (3H), 1,34 (3H), 1,39-1,57 (2H), 1,71-1,87 (3H), 2,07 (1H), 2,17 (1H), 2,19-2,65 (9H), 2,72 (1H), 4,27 (2H), 4,44 (1 H), 5,79 (1H), 6,89 (1H), 7,17 (2H), 7,44 (2H) ppm.

Beispiel 25a

(E/Z)-[4-((5R,8S,11R,13S,14S,17S)-5,17-Dihydroxy-5',5',13-trimethy)-17-pentafluorethyl-2,3,4,5,6,7,8,11,12,13,14,15,16,17-tetradecahydro-1*H*-spiro[cyclopenta[a]phenanthren-3,2'-[1,3]dioxan]-11-yl)-phenyl]-propinal *O*-ethyl-oxim

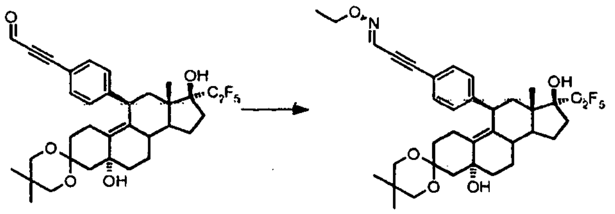

In Analogie zu Beispiel 24a setzte man 50 mg (80 μmol) der nach Beispiel 2a dargestellten Verbindung unter Verwendung von O-Ethylhydroxylamin um und isolierte nach Aufarbeitung und Reinigung 47 mg (88%) der Titelverbindungen als farblosen Schaum.

**Beispiel 26**

**(E/Z)-[4-((8S,11 R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-isobutyl-oxim**

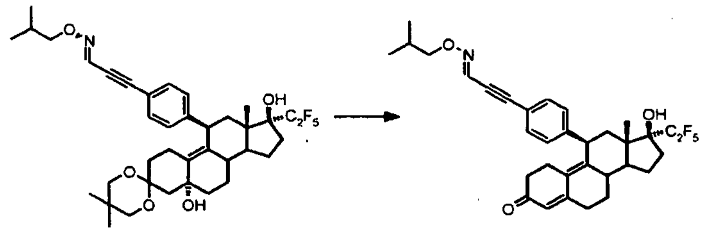

In Analogie zu Beispiel 1 setzte man 60 mg (86 μmol) der nach Beispiel 25a dargestellten Verbindungen um und isolierte nach Aufarbeitung und Reinigung 9,3 mg (18%) der Titelverbindungen als farblosen Schaum.

$^{1}$H-NMR ($CDCl_3$): δ=0,57 (3H), 0,93 (6H), 1,39-1,56 (2H), 1,71-1,88 (3H), 1,95-2,11 (2H), 2,13 (1H), 2,20-2,66 (9H), 2,72 (1H), 3,93 (2H), 4,44 (1H), 5,79 (1H), 7,16 (2H), 7,42 (2H), 7,55+6,86 (1H) ppm.

Beispiel 26a

(E/Z)-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[*a*]phenanthren-3,2'-[1,3]dioxan]-11-yl)-phenyl]-propinal *O*-isobutyl-oxim

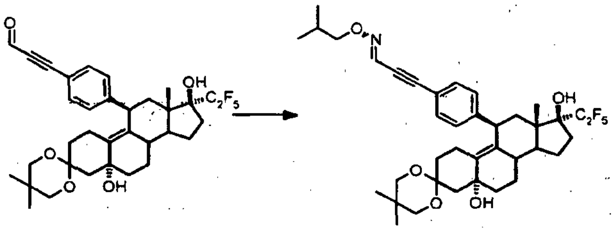

In Analogie zu Beispiel 24a setzte man 50 mg (80 µmol) der nach Beispiel 2a dargestellten Verbindung unter Verwendung von *O*-isobutylhydroxylamin um und isolierte nach Aufarbeitung und Reinigung 47 mg (84%) der Titelverbindungen als farblosen Schaum.

**Beispiel 27**

**(E/Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-(3,4-dichlor-benzyl)-oxim**

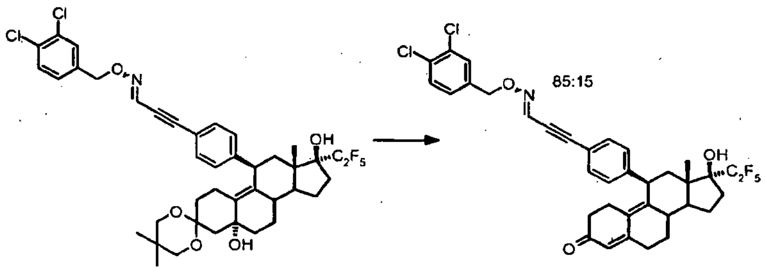


In Analogie zu Beispiel 1 setzte man 71 mg (80 µmol) der nach Beispiel 27a dargestellten Verbindungen um und isolierte nach Aufarbeitung und Reinigung 26 mg (47%) der Titelverbindungen als farblosen Schaum.
$^{1}$H-NMR ($CDCl_3$): δ=0,57 (3H), 1,39-1,56 (2H), 1,71-1,88 (3H), 2,05 (1H), 2,07 (1H), 2,18-2,66 (9H), 2,72 (1H), 4,44 (1H), 5,12+5,18 (2H), 5,79 (1H), 6,94+7,61 (1H), 7,12-7,28 (3H), 7,37-7,54 (4H) ppm.

Beispiel 27a

(E/Z)-[4-((5R,8S,11R,13S,14S,17S)-5,17-Dihydroxy-5',5',13-trimethyl-17-pentafluorethyl-2,3,4,5,6,7,8,11,12,13,14,15,16,17-tetradecahydro-1*H*-spiro[cyclopenta[*a*]phenanthren-3,2'-[1,3]dioxan]-11-yl)-phenyl]-propinal *O*-(3,4-dichlor-benzyl)-oxim

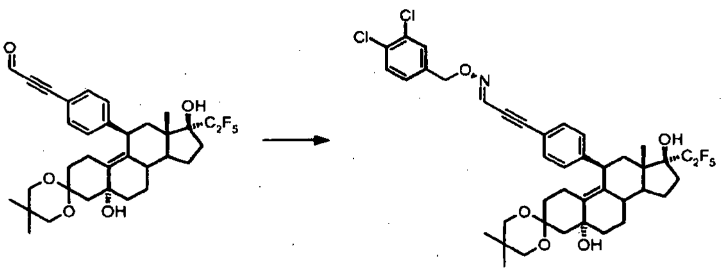

In Analogie zu Beispiel 24a setzte man 50 mg (80 μmol) der nach Beispiel 2a dargestellten Verbindung unter Verwendung von *O*-(3,4-Dichlor-benzyl)-hydroxylamin um und isolierte nach Aufarbeitung 71 mg der Titelverbindung als Rohprodukt.

**Beispiel 28** (Vergleichbeispiel)

1-(3,5-Dimethyl-isoxazol-4-yl)-3-{3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff

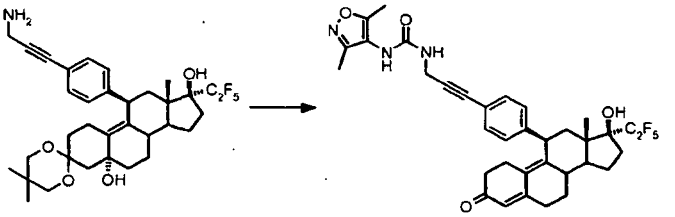

Die Lösung aus 25,6 mg (38 μmol) der nach Beispiel 28a dargestellten Verbindung in 1 ml Dichlormethan versetzte man mit 5,8 mg 3,5-Dimethylisoxazol-4-ylisocyanat und rührte 1 Stunde bei 23°C. Man versetzte mit 57 μl einer 4 molaren Lösung von Chlorwasserstoff in Dioxan und nach 10 Minuten mit 31,7 μl Triethylamin. Man engte ein und reinigte den Rückstand durch Chromatographie. Isolierte wurden 15,2 mg (61%) der Titelverbindung als farbloser Schaum.

$^{1}H$-NMR ($CDCl_3$): δ=0,56 (3H), 1,38-1,53 (2H), 1,70-1,87 (2H), 1,98-2,10 (2H), 2,12-2,63 (9H), 2,16 (3H), 2,31 (3H), 2,71 (1H), 3,09 (1H), 4,20 (2H), 4,41 (1H), 5,23 (1H), 5,77 (1H), 6,17 (1H), 7,10 (2H), 7,27 (2H) ppm.

Beispiel 28a

(5R,8S,11R,13S,14S,17S)-11-[4-(3-Amino-prop-1-in-1-yl)-phenyl]-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-5,17-diol

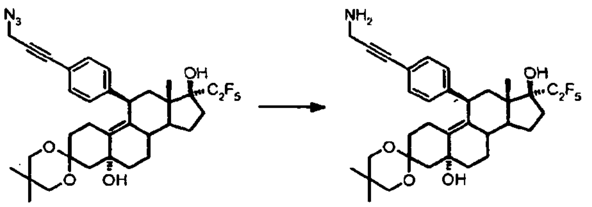

Die Lösung von 267 mg (0,41 mmol) der nach Beispiel 23a dargestellten Verbindung in 10 ml Tetrahydrofuran versetzte man mit 2 ml Wasser, 1 ml Trimethylphosphin und rührte 4 Stunden bei 23°C. Man versetzte mit 1 ml einer 25%igen Ammoniaklösung, rührte weitere 16 Stunden bei 23°C und engte ein. Die als Rohprodukt erhaltene Titelverbindung setzte man ohne Reinigung weiter um.

**Beispiel 29**

**1-(3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-isopropyl-harnstoff**

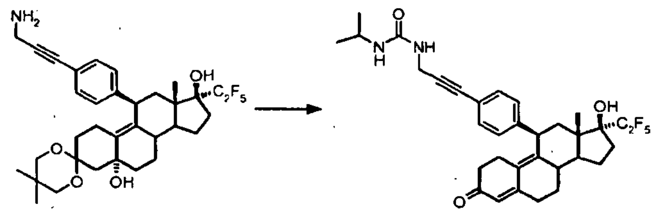

In Analogie zu Beispiel 28 setzte man 35 mg (52 μmol) der nach Beispiel 28a dargestellten Verbindung unter Verwendung von Isopropylisocyanat um und isolierte nach Aufarbeitung und Reinigung 19,5 mg (62%) der Titelverbindung als farblosen Schaum.

$^1$H-NMR ($CDCl_3$): δ= 0,57 (3H), 1,11 (6H), 1,39-1,54 (2H), 1,72-1,87 (3H), 2,04 (1H), 2,18-2,64 (9H), 2,70 (1H), 3,40 (1H), 3,85 (1H), 4,14 (2H), 4,40 (1H), 4,74 (1H), 5,03 (1H), 5,77 (1H), 7,10 (2H), 7,30 (2H) ppm.

**Beispiel 30**

3-(3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-propionsäureethylester

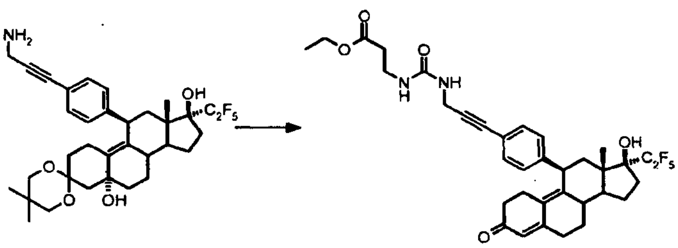

In Analogie zu Beispiel 28 setzte man 25 mg (37 μmol) der nach Beispiel 28a dargestellten Verbindung unter Verwendung von 3-isocyanato-propionsäureethylester um und isolierte nach Aufarbeitung und Reinigung 16,7 mg (68%) der Titelverbindung als farblosen Schaum.

$^1$H-NMR ($CDCl_3$): δ= 0,57 (3H), 1,23 (3H), 1,37-1,54 (2H), 1,71-1,87 (3H), 2,05 (1H), 2,17-2,63 (11H), 2,71 (1H), 3,09 (1H), 3,45 (2H), 4,07-4,19 (4H), 4,41 (1H), 4,98 (1H), 5,29 (1 H), 5,77 (1 H), 7,11 (2H), 7,31 (2H) ppm.

**Beispiel 31**

1-Ethyl-3-{3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff

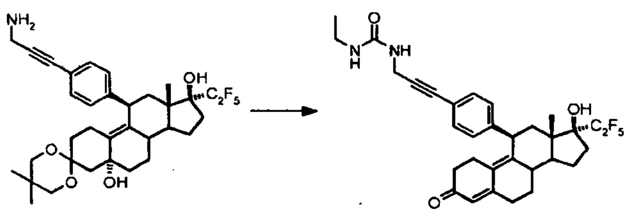

In Analogie zu Beispiel 28 setzte man 88 mg (0,11 mmol) der nach Beispiel 28a dargestellten Verbindung unter Verwendung von Ethylisocyanat um und isolierte nach Aufarbeitung und Reinigung 35 mg (43%) der Titelverbindung als farblosen Schaum.

$^1$H-NMR ($CDCl_3$): δ= 0,57 (3H), 1,12 (3H), 1,37-1,57 (2H), 1,71-1,89 (3H), 2,05 (1H), 2,16-2,65 (9H), 2,70 (1H), 2,82 (1H), 3,22 (2H), 4,17 (2H), 4,42 (1H), 4,60 (1 H), 4,76 (1 H), 5,78 (1 H), 7,11 (2H), 7,32 (2H) ppm.

Beispiel 32

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-methoxy-phenyl)-harnstoff**

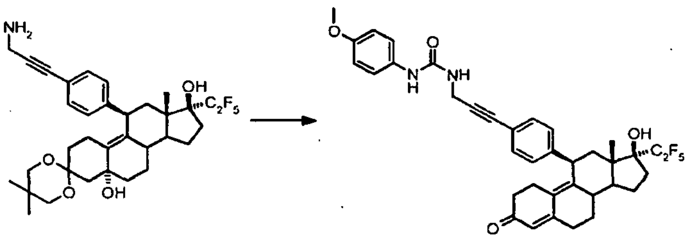

In Analogie zu Beispiel 28 setzte man 25 mg (37 µmol) der nach Beispiel 28a dargestellten Verbindung unter Verwendung von 4-Methoxyphenylisocyanat um und isolierte nach Aufarbeitung und Reinigung 12 mg (49%) der Titelverbindung als farblosen Schaum.
$^1$H-NMR ($CDCl_3$): δ= 0,55 (3H), 1,38-1,52 (2H), 1,71-1,86 (3H), 2,04 (1H), 2,17-2,62 (9H), 2,69 (1H), 3,00 (1 H), 3,75 (3H), 4,20 (2H), 4,39 (1H), 5,42 (1H), 5,77 (1 H), 6,81 (2H), 6,88 (1 H), 7,07 (2H), 7,18 (2H), 7,28 (2H) ppm.

**Beispiel 33**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-*tert*-butyl-phenyl)-harnstoff**

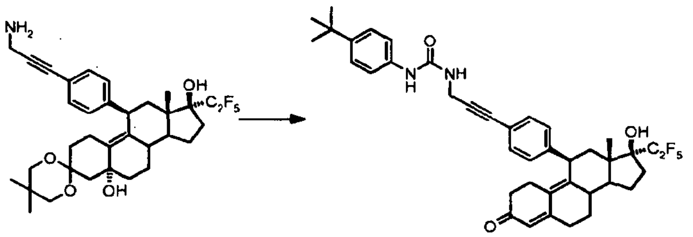

In Analogie zu Beispiel 28 setzte man 25,6 mg (38 µmol) der nach Beispiel 28a dargestellten Verbindung unter Verwendung von 4-*tert*-Butylphenylisocyanat um und isolierte nach Aufarbeitung und Reinigung 11,8 mg (44%) der Titelverbindung als farblosen Schaum.
$^1$H-NMR ($CDCl_3$): δ= 0,55 (3H), 1,27 (9H), 1,38-1,52 (2H), 1,72-1,86 (3H), 2,04 (1H), 2,17-2,63 (9H), 2,69 (1H), 2,88 (1H), 4,22 (2H), 4,39 (1H), 5,52 (1H), 5,77 (1H), 6,95 (1 H), 7,08 (2H), 7,20 (2H), 7,28 (2H), 7,30 (2H) ppm.

**Beispiel 34**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-phenyl-harnstoff**

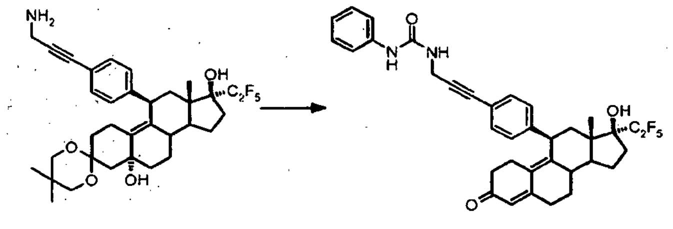

In Analogie zu Beispiel 28 setzte man 25,6 mg (38 μmol) der nach Beispiel 28a dargestellten Verbindung unter Verwendung von Phenylisocyanat um und isotierte nach Aufarbeitung und Reinigung 12 mg (49%) der Titelverbindung als farblosen Schaum.

$^1$H-NMR ($CDCl_3$): δ= 0,55 (3H), 1,37-1,52 (2H), 1,72-1,86 (3H), 2,03 (1H), 2,16-2,62 (9H), 2,68 (1H), 2,94 (1 H), 4,21 (2H), 4,38 (1 H), 5,67 (1H), 5,77 (1H), 7,08 (1 H), 7,07 (2H), 7,19 (1H), 7,21-7,32 (6H) ppm.

**Beispiel 35**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-chlor-phenyl)-harnstoff**

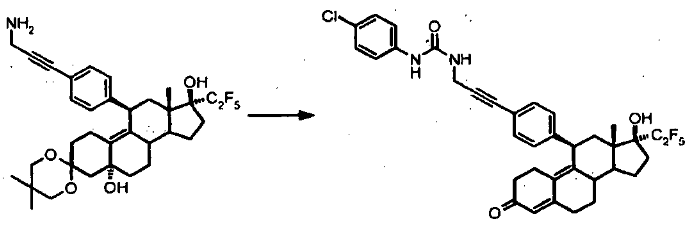

In Analogie zu Beispiel 28 setzte man 25 mg (37 μmol) der nach Beispiel 28a dargestellten Verbindung unter Verwendung von 4-Chlörphenylisocyanat um und isolierte nach Aufarbeitung und Reinigung 8,9 mg (35%) der Titelverbindung als farblosen Schaum.

$^1$H-NMR ($CDCl_3$): δ=0,55 (3H), 1,39-1,52 (2H), 1,72-1,89 (3H), 2,04 (1H), 2,16-2,62 (9H), 2,69 (1H), 2,87 (1H), 4,21 (2H), 4,39 (1H), 5,73 (1H), 5,77 (1H), 7,07 (2H), 7,17 (2H), 7,24 (2H), 7,26 (2H), 7,36 (1H) ppm.

**Beispiel 36**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-fluor-phenyl)-harnstoff**

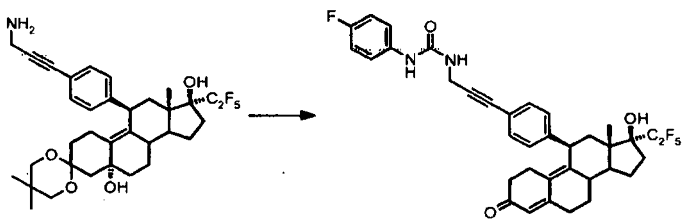

In Analogie zu Beispiel 28 setzte man 25,6 mg (38 μmol) der nach Beispiel 28a dargestellten Verbindung unter Verwendung von 4-Fluorphenylisocyanat um und isolierte nach Aufarbeitung und Reinigung 13,3 mg (53%) der Titelverbindung als farblosen Schaum.

$^1$H-NMR ($CDCl_3$): δ= 0,55 (3H), 1,38-1,53 (2H), 1,72-1,87 (3H), 2,03 (1H), 2,16-2,62 (9H), 2,69 (1 H), 3,02 (1H), 4,19 (2H), 4,38 (1H), 5,71 (1H), 5,77 (1H), 6,90 (2H), 7,06 (2H), 7,20-7,27 (4H), 7,32 (1H) ppm.

**Beispiel 37**

**4-(3-{3-[4-((8S,11R,13S,14S,17S)-17-Hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-prop-2-inyl}-ureido)-benzoesäureethylester**

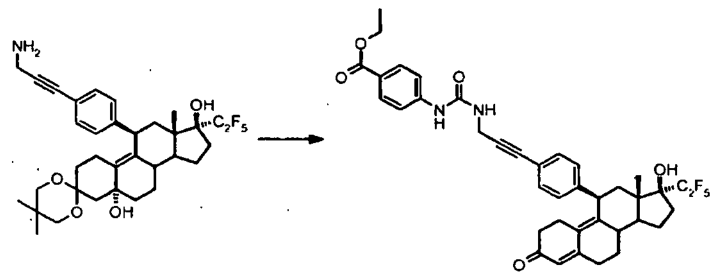

In Analogie zu Beispiel 28 setzte man 25 mg (37 μmol) der nach Beispiel 28a dargestellten Verbindung unter Verwendung von 4-Isocyanato-benzoesäureethylester um und isolierte nach Aufarbeitung und Reinigung 6,0 mg (23%) der Titelverbindung als farblosen Schaum.

$^1$H-NMR ($CDCl_3$): δ= 0,58 (3H), 1,36 (3H), 1,39-1,52 (2H), 1,72-1,87 (3H), 2,03 (1H), 2,17-2,63 (9H), 2,69 (1H), 3,30 (1H), 4,19-4,35 (4H), 4,38 (1H), 5,78 (1H), 6,06 (1H), 7,04 (2H), 7,21 (2H), 7,44 (2H), 7,83 (1H), 7,87 (2H) ppm.

**Beispiel 38**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-methyl-phenyl)-harnstoff**

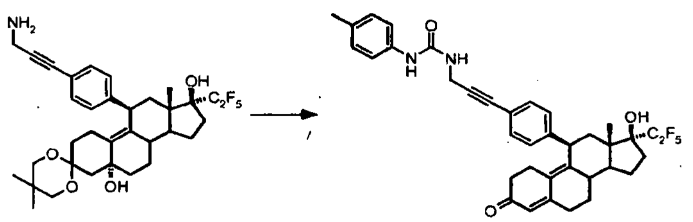

In Analogie zu Beispiel 28 setzte man 25 mg (37 μmol) der nach Beispiel 28a dargestellten Verbindung unter Verwendung von 4-Methylphenylisocyanat um und isolierte nach Aufarbeitung und Reinigung 10,8 mg (44%) der Titelverbindung als farblosen Schaum.

$^1$H-NMR ($CDCl_3$): δ= 0,55 (3H), 1,37-1,54 (2H), 1,70-1,82 (3H), 2,04 (1H), 2,15-2,63 (9H), 2,28 (3H), 2,69 (1H), 2,90 (1H), 4,20 (2H), 4,39 (1H), 5,47 (1H), 5,77 (1H), 6,92 (1H), 7,07 (4H), 7,16 (2H), 7,28 (2H) ppm.

**Beispiel 39**

1-Benzyl-3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff

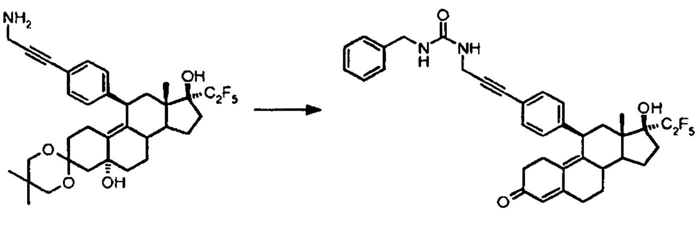

In Analogie zu Beispiel 28 setzte man 35 mg (52 μmol) der nach Beispiel 28a dargestellten Verbindung unter Verwendung von Benzylisocyanat um und isolierte nach Aufarbeitung und Reinigung 17,5 mg (51%) der Titelverbindung als farblosen Schaum.

$^1$H-NMR ($CDCl_3$): δ= 0,56 (3H), 1,36-1,53 (2H), 1,70-1,98 (3H), 2,04 (1H), 2,14-2,62 (9H), 2,67 (1H), 3,28 (1H), 4,11 (2H), 4,29 (2H), 4,38 (1H), 5,26 (1H), 5,37 (1H), 5,75 (1H), 7,08 (2H), 7,19-7,30 (7H) ppm.

**Beispiel 40**

**1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-*tert*-butyl-harnstoff**

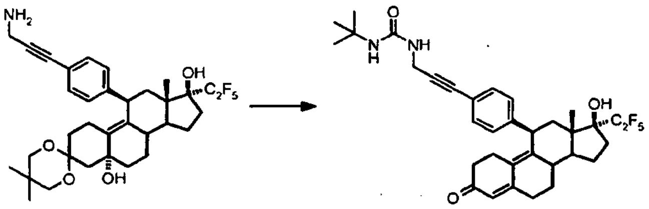

In Analogie zu Beispiel 28 setzte man 35 mg (52 μmol) der nach Beispiel 28a dargestellten Verbindung unter Verwendung von *tert*-Butylisocyanat um und isolierte nach Aufarbeitung und Reinigung 12,6 mg (39%) der Titelverbindung als farblosen Schaum.

$^1$H-NMR ($CDCl_3$): δ= 0,57 (3H), 1,33 (9H), 1,37-1,56 (2H), 1,72-1,88 (3H), 2,05 (1 H), 2,18-2,64 (9H), 2,64-2, 78 (2H), 4,13 (2H), 4,41 (1H), 4,53 (1H), 4,63 (1H), 5,78 (1H), 7,10 (2H), 7,31 (2H) ppm.

**Beispiel 41**

**1-Allyl-3-{3-[4-((8S,11 R,13S,14S,17S)-17-Hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-prop-2-inyl}-harnstoff**

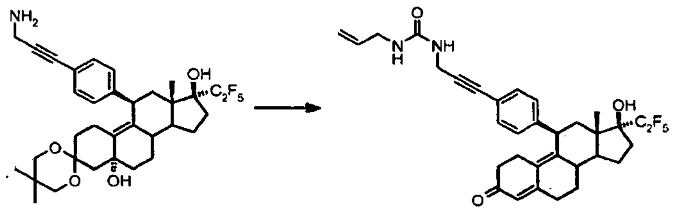

In Analogie zu Beispiel 28 setzte man 25 mg (37 μmol) der nach Beispiel 28a dargestellten Verbindung unter Verwendung von Allylisocyanat um und isolierte nach Aufarbeitung und Reinigung 6,3 mg (28%) der Titelverbindung als farblosen Schaum.

$^1$H-NMR ($CDCl_3$): δ=0,57 (3H), 1,38-1,56 (2H), 1,71-1,88 (3H), 2,05 (1H), 2,15-2,63 (9H), 2,70 (1H), 2,72 (1H), 3,81 (2H), 4,18 (2H), 4,41 (1H), 4,79 (1H), 4,88 (1H), 5,11 (1H), 5,20 (1H), 5,78 (1H), 5,84 (1H), 7,11 (2H), 7,32 (2H) ppm.

**Beispiel 42**

(3-{3-[4-((8S,11R,13S,14S,17S)-17-Hydroxy-13-methy)-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-essigsäureethylester

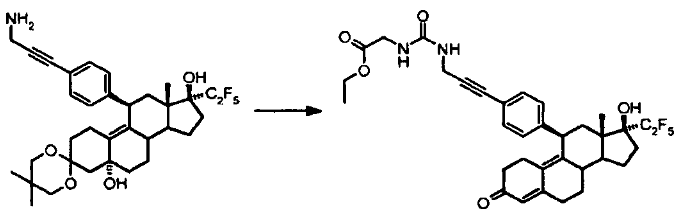

In Analogie zu Beispiel 28 setzte man 35 mg (52 μmol) der nach Beispiel 28a dargestellten Verbindung unter

Verwendung von Isocyanato-essigsäureethylester um und isolierte nach Aufarbeitung und Reinigung 17,1 mg (51%) der Titelverbindung als farblosen Schaum.

$^1$H-NMR ($CDCl_3$): δ=0,56 (3H), 1,24 (3H), 1,36-1,56 (2H), 1,39-1,88 (3H), 2,05 (1H), 2,16-2,63 (9H), 2,70 (1H), 3,15 (1H), 3,98 (2H), 4,11-4,23 (4H), 4,40 (1H), 5,34 (1H), 5,46 (1H), 5,77 (1H), 7,10 (2H), 7,31 (2H) ppm.

**Beispiel 43**

1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-piperidin-1-yl-phenyl)-**harnstoff**

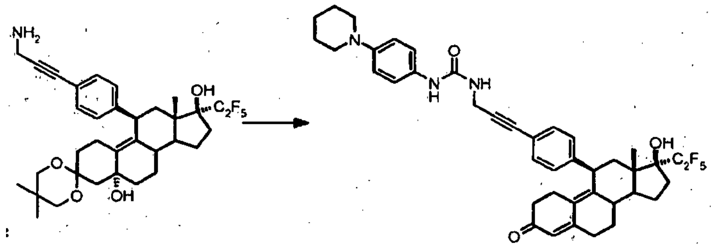

In Analogie zu Beispiel 28 setzte man 28 mg (38 μmol) der nach Beispiel 28a dargestellten Verbindung unter Verwendung von 4-Piperidin-1-yl-phenylisocyanat um und isolierte nach Aufarbeitung und Reinigung 8,4 mg (31%) der Titelverbindung als farblosen Schaum.

$^1$H-NMR ($CDCl_3$): δ= 0,56 (3H), 1,38-1,87 (11H), 2,05 (1 H), 2,17-2,64 (9H), 2,68 (1H), 2,70 (1H), 3,12 (4H), 4,22 (2H), 4,40 (1H), 5,07 (1H), 5,78 (1 H), 6,40 (1H), 6,89 (2H), 7,09 (2H), 7,13 (2H), 7,31 (2H) ppm.

**Beispiel 44**

Allyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

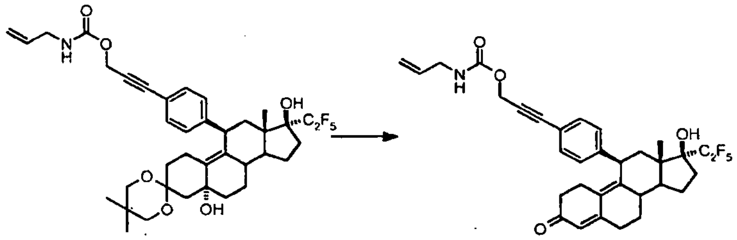

In Analogie zu Beispiel 1 setzte man 89 mg (80 μmol) der nach Beispiel 44a dargestellten Verbindung um und isolierte nach Aufarbeitung und Reinigung 25 mg (52%) der Titelverbindung als farblosen Schaum.

$^1$H-NMR ($CDCl_3$): δ= 0,57 (3H), 1,40-1,55 (2H), 1,73-1,86 (3H), 2,07 (1H), 2,11 (1H), 2,21-2,65 (9H), 2,72 (1 H), 3,84 (2H), 4,43 (1H), 4,87 (1 H), 4,91 (2H), 5,14 (1H), 5,21 (1H), 5,79 (1H), 5,85 (1H), 7,13 (2H), 7,38 (2H) ppm.

Beispiel 44a

Allyl-carbaminsäure 3-[4-((5R,8S,11R,13S,14S,17S)-5,17-dihydroxy-17-pentafluorethyl-5',5',13-trimethyl-2,3,4,5,6,7,8,11,12,13,14,15,16,17-tetradecahydro-1*H*-spiro[cyclopenta[*a*]phenanthren-3,2'-[1,3]dioxan]-11-yl)-phenyl]-prop-2-inyl ester

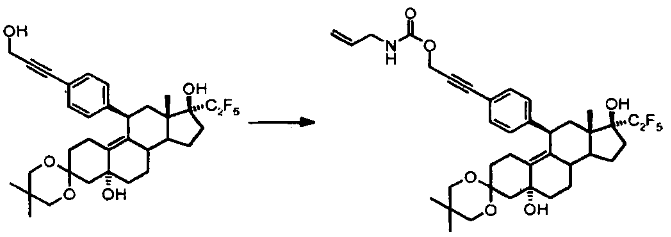

Die Lösung von 50 mg (80 µmol) der nach Beispiel 1a dargestellten Verbindung in 1,5 ml Dichlormethan versetzte man mit 424 µl Allylisocyanat und rührte 3,5 Tage bei 23°C. Man engte ein und setzte den Rückstand ohne Reinigung weiter um.

**Beispiel 45**

**Ethyl-carbaminsäure3-[4-((8S,11R,13S,14S,17S)-17-hydroxy-13-methyl-3-oxo-17-pentafluorethyl-2,3,6,7,8,11, 12,13,14,15,16,17-dodecahydro-1*H*-cyclopenta[*a*]phenanthren-11-yl)-phenyl]-prop-2-inyl ester**

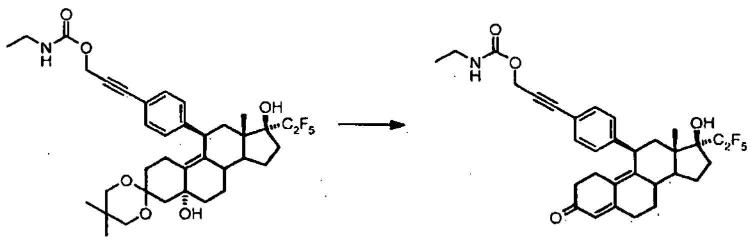

In Analogie zu Beispiel 1 setzte man 27 mg (39 µmol) der nach Beispiel 45a dargestellten Verbindung um und isolierte nach Aufarbeitung und Reinigung 12 mg (53%) der Titelverbindung als farblosen Schaum.

$^1$H-NMR ($CDCl_3$): δ= 0,57 (3H), 1,15 (3H), 1,38-1,57 (2H), 1,70-1,88 (3H), 2,06 (1H), 2,17-2,64 (10H), 2,71 (1H), 3,24 (2H), 4,43 (1H), 4,77 (1H), 4,88 (2H), 5,78 (1H), 7,13 (2H), 7,37 (2H) ppm.

Beispiel 45a

Ethyl-carbaminsäure 3-[4-((5R,8S,11R,13S,14S,17S)-5,17-dihydroxy-17-pentafluorethyl-5',5',13-trimethyl-2,3,4,5,6,7, 8,11,12,13,14,15,16,17-tetradecahydro-1*H*-spiro[cyclopenta[*a*]phenanthren-3,2'-[1,3]dioxan]-11-yl)-phenyl]-prop-2-inyl ester

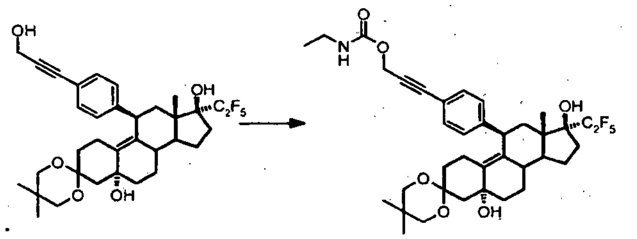

In Analogie zu Beispiel 44a setzte man 50 mg (80 µmol) der nach Beispiel 1a dargestellten Verbindung unter Verwendung von Ethylisocyanat bei 60°C um und isolierte nach Aufarbeitung und Reinigung 27 mg (48%) der Titelverbindung als farblosen Schaum.

**Beispiel 46**

**Phenyl-carbaminsäure 3-[4-((8S,11R,13S,14S,17S)-17-hydroxy-13-methyl-3-oxo-17-pentafluorethyl-2,3,6,7,8,**

**11,12,13,14,15,16,17-dodecahydro-1*H*-cyclopenta[*a*]phenanthren-11-yl)-phenyl]-prop-2-inyl ester**

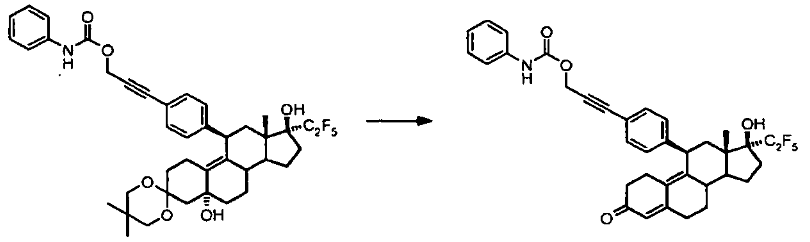

In Analogie zu Beispiel 1 setzte man 53,4 mg (72 µmol) der nach Beispiel 46a dargestellten Verbindung um und isolierte nach Aufarbeitung und Reinigung 17,4 mg (38%) der Titelverbindung als farblosen Schaum.

$^1$H-NMR ($CDCl_3$): δ= 0,57 (3H), 1,40-1,55 (2H), 1,73-1,85 (3H), 2,06 (1H), 2,18-2,64 (10H), 2,71 (1H), 4,43 (1H), 5,00 (2H), 5,79 (1H), 6,80 (1H), 7,08 (1 H), 7,13 (2H), 7,31 (2H), 7,37 (2H), 7,39 (2H) ppm.

Beispiel 46a

Phenyl-carbaminsäure 3-[4-((5R,8S,11R,13S, 14S, 17S)-5,17-dihydroxy-17-pentafluorethyl-5',5',13-trimethyl-2,3,4,5,6, 7,8,11,12,13,14,15,16,17-tetradecahydro-1*H*-spiro[cyclopenta[*a*]phenanthren-3,2'-[1,3]dioxan]-11-yl)-phenyl]-prop-2-inyl ester

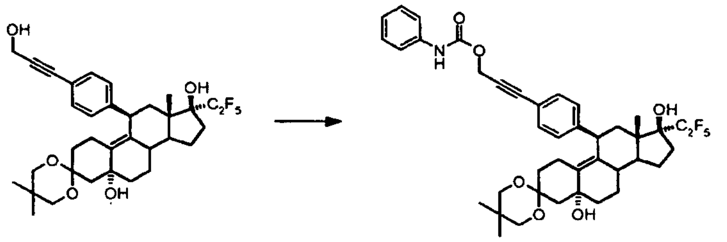

In Analogie zu Beispiel 44a setzte man 50 mg (80 µmol) der nach Beispiel 1 a dargestellten Verbindung unter Verwendung von Phenylisocyanat um und isolierte nach Aufarbeitung und Reinigung 53 mg (90%) der Titelverbindung als als farblosen Schaum.

**Beispiel 47**

4-Methylphenyl-carbaminsäure 3-[4-((8S,11 R,13S,14S,17S)-17-hydroxy-13-methyl-3-oxo-17-pentafluorethyl-2,3,6,7, 8,11,12,13,14,15,16,17-dodecahydro-*H*-cyclopenta[*a*]phenanthren-11-yl)-phenyl]-prop-2-inyl ester

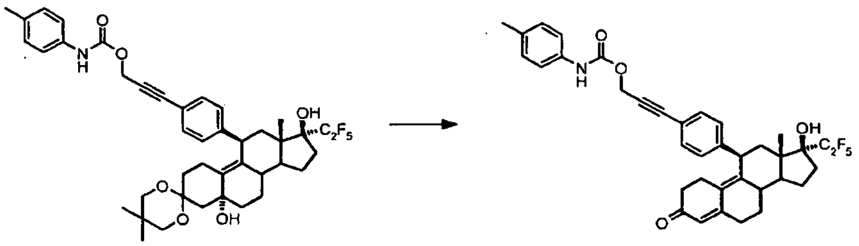

In Analogie zu Beispiel 1 setzte man 25 mg (33 μmol) der nach Beispiel 47a dargestellten Verbindung um und isolierte nach Aufarbeitung und Reinigung 8,2 mg (38%) der Titelverbindung als farblosen Schaum.
$^1$H-NMR ($CDCl_3$): δ= 0,57 (3H), 1,38-1,56 (2H), 1,71-1,86 (3H), 2,07 (1H), 2,11 (1H), 2,18-2,66 (9H), 2,31 (3H), 2,71 (1 H), 4,43 (1H), 4,99 (2H), 5,79 (1H), 6,66 (1H), 7,11 (2H), 7,14 (2H), 7,27 (2H), 7,38 (2H) ppm.

Beispiel 47a

4-Methylphenyl-carbaminsäure 3-[4-((5R,8S, 11 R, 13S, 14S, 17S)-5,17-dihydroxy-17-pentafluorethyl-5',5',13-trimethyl-2,3,4,5,6,7,8,11,12,13,14,15,16,17-tetradecahydro-1*H*-spiro[cyclopenta[a]phenanthren-3,2'-[1,3]dioxan]-11-yl)-phenyl]-prop-2-inyl ester

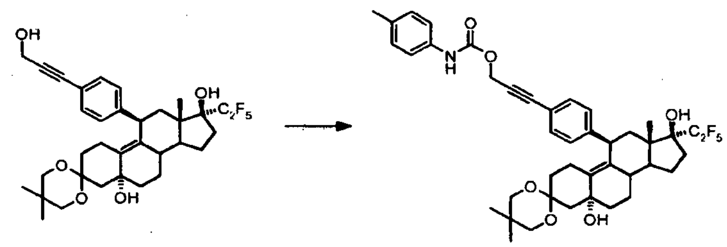

In Analogie zu Beispiel 44a setzte man 50 mg (80 μmol) der nach Beispiel 1a dargestellten Verbindung unter Verwendung von 4-Methylphenylisocyanat um und isolierte nach Aufarbeitung und Reinigung 25 mg (42%) der Titelverbindung als farblosen Schaum.

**Beispiel 48**

4-Fluorphenyl-carbaminsäure 3-[4-((8S,11R,13S,14S,17S)-17-hydroxy-13-methyl-3-oxo-17-pentafluorethyl-2,3,6,7,8, 11,12,13,14,15,16,17-dodecahydro-1*H*-cyclopenta[*a*]phenanthren-11-yl)-phenyl]-prop-2-inyl ester

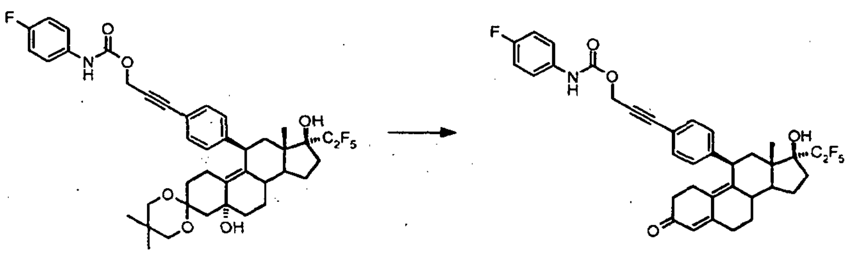

In Analogie zu Beispiel 1 setzte man 157 mg (80 μmol) der nach Beispiel 48a dargestellten Verbindung um und isolierte nach Aufarbeitung und Reinigung 9,2 mg (18%) der Titelverbindung als farblosen Schaum.

$^1H$-NMR ($CDCl_3$): δ= 0,57 (3H), 1,39-1,55 (2H), 1,74-1,85 (3H), 2,07 (1H), 2,10 (1H), 2,20-2,64 (9H), 2,71 (1H), 4,44 (1H), 5,00 (2H), 5,79 (1H), 6,72 (1 H), 7,01 (2H), 7,14 (2H), 7,35 (2H), 7,38 (2H) ppm.

Beispiel 48a

4-Fluorphenyl-carbaminsäure 3-[4-((5R,8S,11R,13S,14S,17S)-5,17-dihydroxy-17-pentafluorethyl-5',5',13-trimethyl-2,3,4,5,6,7,8,11,12,13,14,15,16,17-tetradecahydro-1H-spiro[cyclopenta[*a*]phenanthren-3,2'-[1,3]dioxan]-11-yl)-phenyl]-prop-2-inyl ester

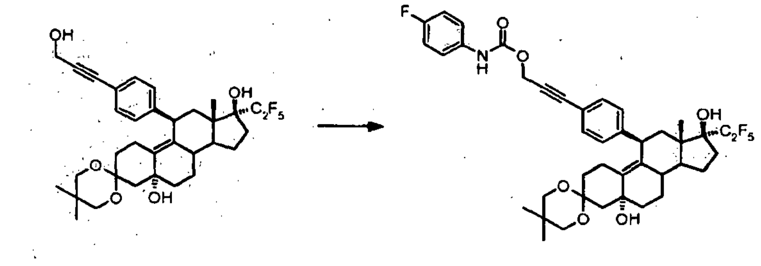

In Analogie zu Beispiel 44a setzte man 50 mg (80 μmol) der nach Beispiel 1a dargestellten Verbindung unter Verwendung von 4-Fluorphenylisocyanat um und isolierte die Titelverbindung als Rohprodukt, das ohne Reinigung weiter umgesetzte wird.

**Beispiel 49**

**Isopropyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester**

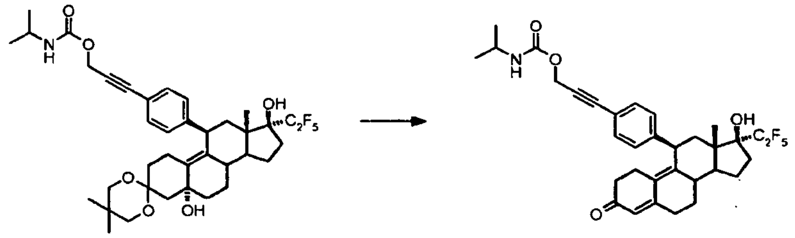

In Analogie zu Beispiel 1 setzte man 31,7 mg (45 μmol) der nach Beispiel 49a dargestellten Verbindung um und isolierte nach Aufarbeitung und Reinigung 15,5 mg (57%) der Titelverbindung als farblosen Schaum.

$^1H$-NMR ($CDCl_3$): δ= 0,57 (3H), 1,16 (6H), 1,38-1,57 (2H), 1,70-1,87 (3H), 2,06 (1H), 2,17-2,64 (10H), 2,71 (1H), 3,82 (1H), 4,42 (1H), 4,64 (1H), 4,87 (2H), 5,78 (1H), 7,12 (2H), 7,37 (2H) ppm.

Beispiel 49a

Isopropyl-carbaminsäure 3-[4-((5R,8S,11R,13S,14S,17S)-5,17-dihydroxy-17-pentafluorethyl-5',5',13-trimethyl-2,3,4,5,6,7,8,11,12,13,14,15,16,17-tetradecahydro-1*H*-spiro[cyclopenta[*a*]phenanthren-3,2'-[1,3]dioxan]-11-yl)-phenyl]-prop-2-inyl ester

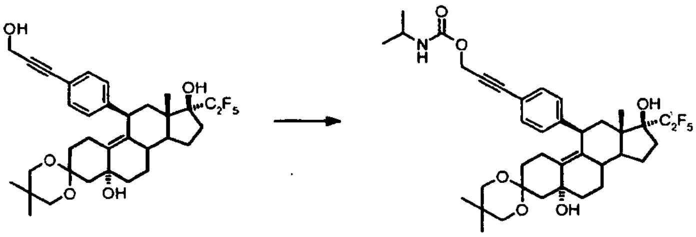

In Analogie zu Beispiel 44a setzte man 50 mg (80 μmol) der nach Beispiel 1a dargestellten Verbindung unter Verwendung von Isopropylisocyanat um und isolierte nach Aufarbeitung und Reinigung 31,7 mg (56%) der Titelverbindung als farblosen Schaum

**Beispiel 50**

**Benzyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester**

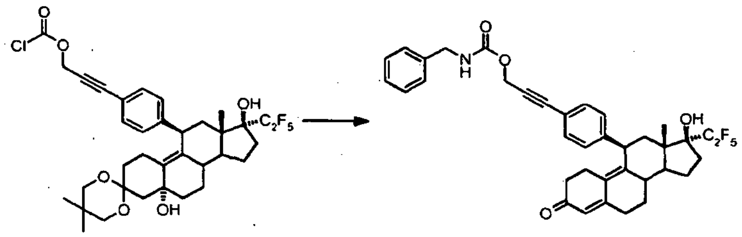

Die Lösung von 39 mg (57 μmol) der nach Beispiel 50a dargestellten Verbindung in 0,5 ml Dichlormethan versetzte man mit der Lösung von 12,4 μl Benzylamin in 0,5 ml Dichlormethan und rührte 1 Stunde bei 23°C. Man versetzte mit Wasser, extrahierte mehrfach mit Dichlormethan und trocknete die vereinigten organischen Extrakte über Natriumsulfat. Den nach Filtration und Lösungsmittelabzug erhaltenen Rückstand reinigte man durch Chromatographie. Isolierte wurden 5 mg (13%) der Titelverbindung als farbloser Schaum.

$^1$H-NMR ($CDCl_3$): δ=0,57 (3H), 1,42-1,55 (2H), 1,74-1,87 (3H), 2,03-2,11 (2H), 2,20-2,66 (9H), 2,72 (1H), 4,40 (2H), 4,43 (1H), 4,94 (2H), 5,11 (1H) 5,79 (1H), 7,13 (2H), 7,27-7,41 (7H) ppm.

Beispiel 50a

Chlorameisensäure 3-[4-((5R,8S,11R,13S,14S,17S)-5,17-dihydroxy-5',5',13-trimethyl-17-pentafluorethyl-2,3,4,5,6,7,8,11,12,13,14,15,16,17-tetradecahydro-1*H*-spiro[cyclopenta[*a*]phenanthren-3,2'-[1,3]dioxan]-11-yl)-phenyl]-prop-2-inyl ester

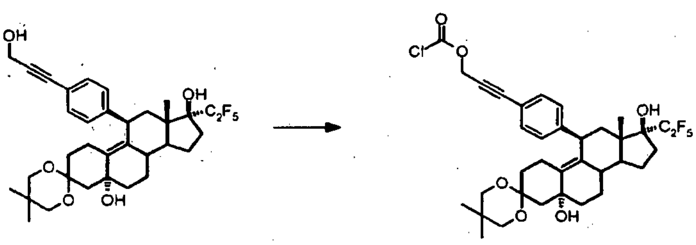

Die Lösung von 50 mg (80 μmol) der nach Beispiel 1a dargestellten Verbindung in 0,5 ml Dichlormethan versetzte man bei 0°C mit 12,9 μl Pyridin, 23,8 mg Triphosgen und rührte 1,5 Stunden. Man filtriert über wenig Kieselgel, engte das Filtrat ein und setzte die erhaltene Titelverbindung ohne Reinigung weiter um.

Beispiel 51

(8S,11 **R,13S,14S,17S)-17-Hydroxy-11-[4-(3-methanesulfonyl-phenylethinyl)-phenyl]-13-methyl-17-pentafluorethyl-1,2,6,7,8,11,12,13,14,15,16,17-dodecahydro-**cyclopenta[a]phenanthren-3-on

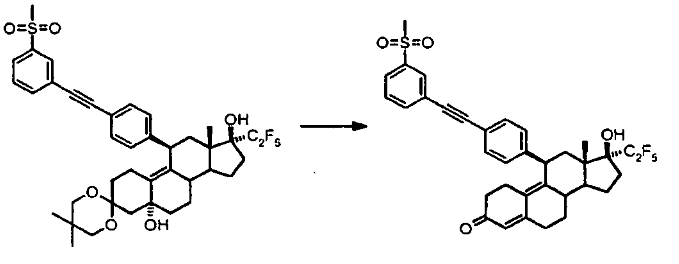

In Analogie zu Beispiel 1 setzte man 23 mg (31 μmol) der nach Beispiel 50a dargestellten Verbindung um und isolierte nach Aufarbeitung und Reinigung 10 mg (51%) der Titelverbindung als farblosen Schaum.

$^{1}$H-NMR ($CDCl_3$): δ= 0,61 (3H), 1,40-1,56 (2H), 1,74-1,88 (3H), 2,08 (1 H), 2,18 (1H), 2,22-2,67 (9H), 2,74 (1H), 3,08 (3H), 4,47 (1H), 5,80 (1H), 7,20 (2H), 7,46 (2H), 7,56 (1 H), 7,77 (1 H), 7,89 (1 H), 8,10 (1H) ppm.

Beispiel 51 a

(5R,8S,11R,13S,14S,17S)-11-[4-(3-Methanesulfonyl-phenylethinyl)-phenyl]-5',5',13-trimethyl-17-pentafluorethyl-1,2,3,4,6,7,8,11,12,13,14,15,16,17-tetradecahydro-spiro[cyclopenta[*a*]phenanthrene-3,2'-[1,3]dioxan]-5,17-diol

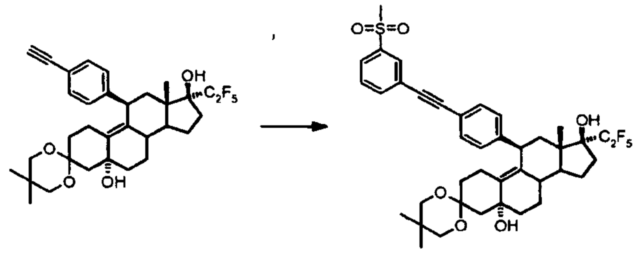

In Analogie zu Beispiel 17b setzte man 50 mg (84 μmol) der nach Beispiel 15b dargestellten Verbindung unter Verwendung von 3-Bromphenyl-methylsulfon um und isolierte nach Aufarbeitung und Reinigung 23 mg (37%) der Titelverbindung als farblosen Schaum.

**Beispiel** 52

4-Methoxyphenyl-carbaminsäure **3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl** ester

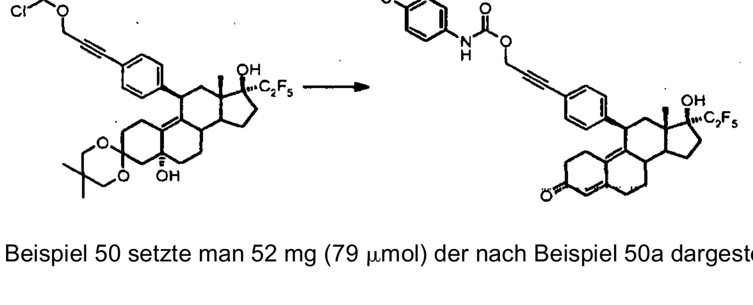

In Analogie zu Beispiel 50 setzte man 52 mg (79 μmol) der nach Beispiel 50a dargestellten Verbindung unter

Verwendung von p-Anisidin um und isolierte nach Aufarbeitung und Reinigung 13 mg (25%) der Titelverbindung als farblosen Schaum.

$^1$H-NMR ($CDCl_3$): δ= 0,56 (3H), 1,38-1,56 (2H), 1,72-1,86 (3H), 2,07 (1H), 2,17 (1H), 2,19-2,64 (9H), 2,71 (1 H), 3,78 (3H), 4,43 (1H), 4,99 (2H), 5,79 (1H), 6,63 (1H), 6,85 (2H), 7,13 (2H), 7,29 (2H), 7,38 (2H) ppm.

**Beispiel 53**

**4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxycarbonylamino}-benzoesäureethylester**

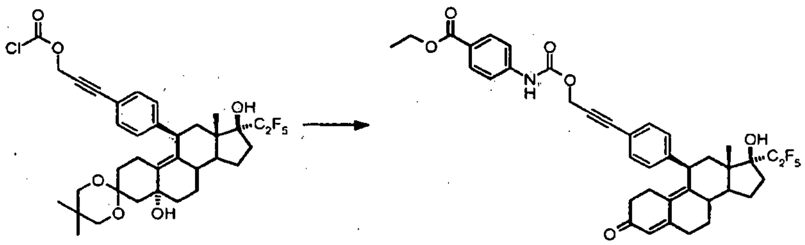

In Analogie zu Beispiel 50 setzte man 46,7 mg (80 μmol) der nach Beispiel 50a dargestellten Verbindung unter Verwendung von 4-Aminobezoesäureethylester um und isolierte nach Aufarbeitung und Reinigung 7,7 mg (14%) der Titelverbindung als farblosen Schaum.

$^1$H-NMR ($CDCl_3$): δ= 0,56 (3H), 1,38 (3H), 1,39-1,55 (2H), 1,72-1,86 (3H), 2,06 (1H), 2,18-2,64 (10H), 2,72 (1H), 4,35 (2H), 4,43 (1H), 5,02 (2H), 5,79 (1H), 7,03 (1H), 7,13 (2H), 7,37 (2H), 7,47 (2H), 8,00 (2H) ppm.

**Beispiel 54**

**(4-Piperidin-1-yl-phenyl)-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester**

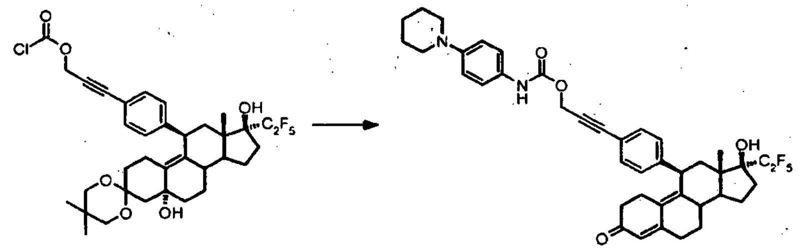

In Analogie zu Beispiel 50 setzte man 46,7 mg (80 μmol) der nach Beispiel 50a dargestellten Verbindung unter Verwendung von 4-Piperidin-1-yl-phenylamin um und isolierte nach Aufarbeitung und Reinigung 10,1 mg (17%) der Titelverbindung als farblosen Schaum.

$^1$H-NMR ($CDCl_3$): δ= 0,56 (3H), 1,38-1,87 (11 H), 2,06 (1H), 2,17 (1H), 2,20-2,64 (9H), 2,72 (1H), 3,08 (4H), 4,43 (1 H), 4,98 (2H), 5,79 (1H), 6,58 (1 H), 6,90 (2H), 7,13 (2H), 7,25 (2H), 7,38 (2H) ppm.

**Beispiel 55**

**4-Chlorphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester**

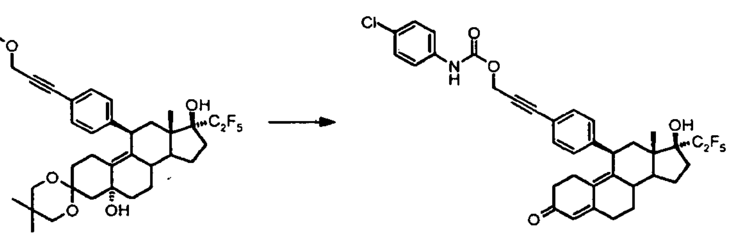

In Analogie zu Beispiel 50 setzte man 46,7 mg (80 μmol) der nach Beispiel 50a dargestellten Verbindung unter Verwendung von 4-Chlor-anilin um und isolierte nach Aufarbeitung und Reinigung 7,4 mg (14%) der Titelverbindung als farblosen Schaum.

$^1$H-NMR ($CDCl_3$): δ= 0,56 (3H), 1,38-1,55 (2H), 1,72-1,86 (3H), 2,06 (1H), 2,09 (1H), 2,18-2,64 (9H), 2,71 (1H), 4,43 (1H), 5,00 (2H), 5,79 (1H), 6,76 (1H), 7,13 (2H), 7,27 (2H), 7,35 (2H), 7,38 (2H) ppm.

**Beispiel 56**

**(8S,11 R,13S,14S,17S)-17-Hydroxy-11-[4-(4-methanesulfonyl-phenylethinyl)-phenyl]-13-methyl-17-pentafluorethyl-1,2,6,7,8,11,12,13,14,15,16,17-dodecahydro-cyclopenta[*a*]phenanthren-3-on**

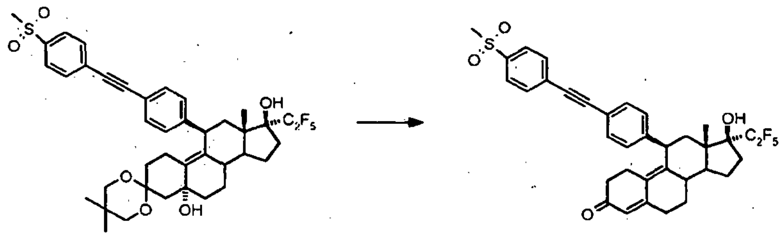

In Analogie zu Beispiel 1 setzte man 11,4 mg (15 μmol) der nach Beispiel 56a dargestellten Verbindung um und isolierte nach Aufarbeitung und Reinigung 5,4 mg (55%) der Titelverbindung als farblosen Schaum.

$^1$H-NMR ($CDCl_3$): δ= 0,60 (3H), 1,42-1,56 (2H), 1,75-1,88 (3H), 2,08 (2H), 2,23-2,67 (9H), 2,74 (1H), 3,07 (3H), 4,47 (1H), 5,80 (1H), 7,21 (2H), 7,48 (2H), 7,69 (2H), 7,92 (2H) ppm.

Beispiel 56a

(5R,8S,11R,13S,14S,17S)-11-[4-(4-Methanesulfonyl-phenylethinyl)-phenyl]-5',5',13-trimethyl-17-pentafluorethyl-1,2,3,4,6,7,8,11,12,13,14,15,16,17-tetradecahydro-spiro[cyclopenta[*a*]phenanthrene-3,2'-[1,3]dioxan]-5,17-diol

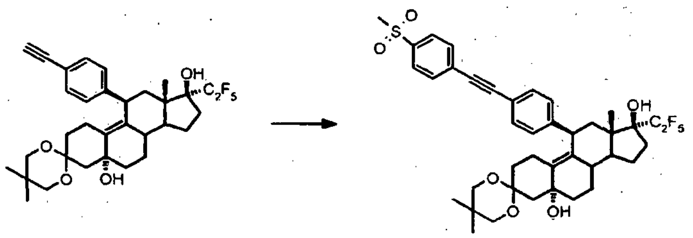

In Analogie zu Beispiel 17b setzte man 50 mg (84 μmol) der nach Beispiel 15b dargestellten Verbindung unter Verwendung von 4-Bromphenyl-methylsulfon um und isolierte nach Aufarbeitung und Reinigung 11,4 mg (18%) der Titelverbindung als farblosen Schaum.

**Beispiel 57**

**3-Pyridyl-carbaminsäure 3-[4-((8S,11R,13S,14S,17S)-17-hydroxy-13-methyl-3-oxo-17-pentafluorethyl-2,3,6,7,8, 11,12,13,14,15,16,17-dodecahydro-1*H*-cyclopenta[*a*]phenanthren-11-yl)-phenyl]-prop-2-inyl ester**

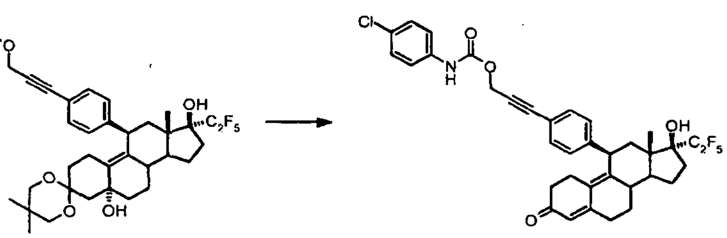

In Analogie zu Beispiel 50 setzte man 93,3 mg (0,16 mmol) der nach Beispiel 50a dargestellten Verbindung unter Verwendung von Pyridin-3-ylamin um und isolierte nach Aufarbeitung und Reinigung 16,3 mg (16%) der Titelverbindung als farblosen Schaum.

$^1$H-NMR ($CDCl_3$): δ= 0,57 (3H), 1,38-1,55 (2H), 1,72-1,87 (3H), 2,06 (1H), 2,17-2,63 (9H), 2,70 (1H), 3,10 (1H), 4,41 (1H), 5,02 (2H), 5,78 (1H), 7,10 (2H), 7,20 (1H), 7,28 (1 H), 7,35 (2H), 8,02 (1H), 8,31 (1 H), 8,49 (1H) ppm.

**Beispiel 58**

***tert*-Butyl-carbaminsäure 3-[4-((8S,11R,13S,14S,17S)-17-hydroxy-13-methyl-3-oxo-17-pentafluorethyl-2,3,6,7,8, 11,12,13,14,15,16,17-dodecahydro-1**H**-cyclopenta[*a*]phenanthren-11-yl)-phenyl]-prop-2-inyl** ester

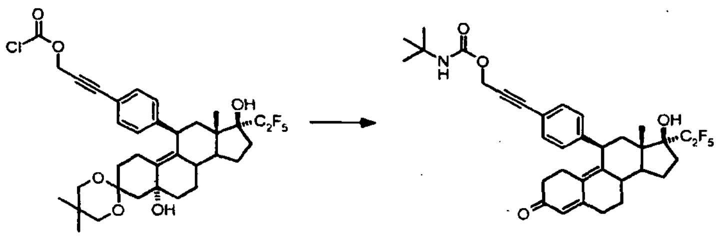

In Analogie zu Beispiel 50 setzte man 52 mg (79 μmol) der nach Beispiel 50a dargestellten Verbindung unter Verwendung von *tert*-Butylamin um und isolierte nach Aufarbeitung und Reinigung 16 mg (33%) der Titelverbindung als farblosen Schaum.

$^1$H-NMR ($CDCl_3$): δ= 0,57 (3H), 1,33 (9H), 1,40-1,54 (2H), 1,73-1,85 (3H), 2,07 (1H), 2,17 (1H), 2,20-2,64 (9H), 2,72 (1H), 4,43 (1H), 4,77 (1H), 4,85 (2H), 5,79 (1H), 7,13 (2H), 7,37 (2H) ppm.

**Beispiel 59**

***4-tert*-Butylphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-prop-2-inyl ester**

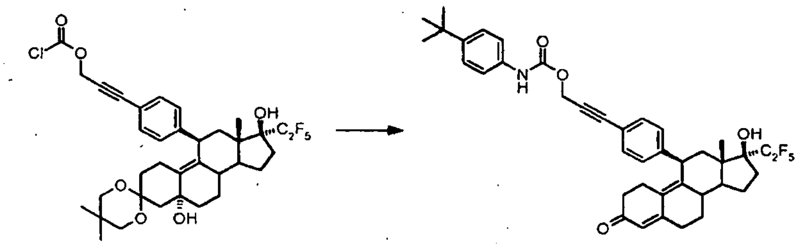

In Analogie zu Beispiel 50 setzte man 46,7 mg (80 μmol) der nach Beispiel 50a dargestellten Verbindung unter Verwendung von 4-*tert*-Butylanilin um und isolierte nach Aufarbeitung und Reinigung 11 mg (19%) der Titelverbindung als farblosen Schaum.

$^1$H-NMR ($CDCl_3$): δ= 0,57 (3H), 1,30 (9H), 1,40-1,54 (2H), 1,73-1,85 (3H), 2,02-2,12 (2H), 2,20-2,63 (9H), 2, 72 (1H), 4,44 (1H), 5,00 (2H), 5,79 (1H), 6,67 (1H), 7,13 (2H), 7,30 (2H), 7,34 (2H), 7,38 (2H) ppm.

**Beispiel 60**

**Bestimmung der Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MN-LUC Reporter Konstrukt**

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0,01 nmol/l, 0,1 nmol/l, 1 nmol/l, 10 nmol/l, 100 nmol/l und 1 pmol/l) inkubiert, um die agonistische Wirksamkeit zu bestimmen. Als Positivkontrolle der Reportergeninduktion wurden die Zellen mit dem synthetischen Gestagen Promegeston (0,01 nmol/l, 0,1 nmol/l, 1 nmol/l, 10 nmol/l, 100 nmol/l and 1 pmol/l) behandelt. Zur Bestimmung der antagonistischen Aktivitat wurden die Zellen mit 0,1 nmol/l Promegeston und zusätzlich mit steigenden Mengen der jeweiligen Testverbindung (0,01 nmol/l, 0,1 nmol/l, 1 nmol/l, 10 nmol/l, 100 nmol/l and 1 pmol/l) behandelt. Die Aktivitat des Reportergens LUC (LUC = Luciferase) wurde in den Zellysaten bestimmt und als RLU (relative light units) gemessen. Alle Meßwerte werden angegeben als % Wirksamkeit und als $EC_{50}$ bzw. $IC_{50}$ Konzentrationen.

a) agonistische Aktivitat: Keine der genannten Verbindungen zeigt eine agonistische Aktivitat.
b) antagonistische Aktivitat: Alle genannten Verbindungen zeigen eine 100%ige antagonistische Wirksamkeit. Die antagonistische Wirkstärke der Verbindungen ist in

Tabelle 1 zusammengefasst. Erfindungsgemäß bevorzugt sind Verbindungen deren PR-A oder PR-B Wert ≤ 0,2 nM ist.

| Bsp. | $IC_{50}$ [nM] | | Bsp. | $IC_{50}$ [nM] | | Bsp | $IC_{50}$ [nM] | |
|---|---|---|---|---|---|---|---|---|
| | PR-A | PR-B | | PR-A | PR-B | | PR-A | PR-B |
| 1 | 0,097 | 0,11 | 21 | 0,36 | 1,0 | 40 | 0,1 | 0,3 |
| 2 | 32 | 16 | 22 | 0,014 | 0,014 | 41 | 0,1 | 0,1 |
| 3 | 1,8 | 1,2 | 23 | 0,2 | 0,3 | 42 | 0,7 | 0,8 |
| 4 | 0,2 | 0,1 | 24A | 0,1 | 0,1 | 43 | 0,8 | 0,9 |
| 5 | 0,72 | 0,76 | 24B | 0,1 | 0,1 | 44 | 0,01 | 0,01 |
| 6 | 13 | 12 | 25A | 0,01 | 0,08 | 45 | 0,01 | 0,01 |
| 7 | 0,8 | 0,9 | 25B | 0,09 | 0,09 | 46 | 0,01 | 0,01 |
| 8 | 0,32 | 0,41 | 26 | 0,08 | 0,08 | 47 | 0,01 | 0,01 |
| 9 | 8,1 | 10 | 27 | 0,97 | 2,0 | 48 | 0,09 | 0,08 |
| 10 | 9,0 | 10 | 28 | 0,04 | 0,08 | 49 | 0,01 | 0,07 |
| 11 | 0,32 | 1,0 | 29 | 0,1 | 0,1 | 50 | 0,01 | 0,03 |

(fortgesetzt)

| Bsp. | $IC_{50}$ [nM] | | Bsp. | $IC_{50}$ [nM] | | Bsp | $IC_{50}$ [nM] | |
|---|---|---|---|---|---|---|---|---|
| | PR-A | PR-B | | PR-A | PR-B | | PR-A | PR-B |
| 12 | 1 | 2 | 30 | 0,1 | 0,4 | 51 | 0,08 | 0,1 |
| 13A | 0,1 | 0,2 | 31 | 0,08 | 0,1 | 52 | 0,01 | 0,07 |
| 13B | nd | nd | 32 | 0,2 | 0,1 | 53 | 0,16 | 0,19 |
| 14 | 0,1 | 0,1 | 33 | 0,9 | 0,8 | 54 | 0,10 | 0,09 |
| 15 | 0,7 | 1,0 | 34 | 0,1 | 0,1 | 55 | 0,26 | 0,2 |
| 16 | 0,1 | 0,05 | 35 | 0,4 | 0,4 | 56 | 0,01 | 0,08 |
| 17 | 0,2 | 0,2 | 36 | 0,1 | 0,2 | 57 | 0,01 | 0,08 |
| 18 | 8 | 5 | 37 | 0,1 | 0,4 | 58 | 0,09 | 0,01 |
| 19 | 3,3 | 4,3 | 38 | 0,2 | 0,3 | 59 | 0,7 | 1,2 |
| 20 | 2,3 | 7,0 | 39 | 0,1 | 0,1 | Ref. 1* | 0,4 | 0,3 |

Tabelle 1 nd = nicht ermittelt

* Ref. 1 Tabelle 1: Als Vergleichsverbindung (Referenzsubstanz) dient die in Beispiel 10 der WO 1998/34947 beschriebene Verbindung (8S,11R,13S,14S,17S)-17-Hydroxy-11-[4-(hydroxymethyl)-phenyl]-13-methyl-17-pentafluorethyl-1,2,6,7,8,11,12,13,14,15,16,17-dodecahydro-cyclopenta[a]phenanthren-3-on.

**Beispiel 61**

**Bestimmung der metabolischen Stabilität in humanen und Ratten-Lebermikrosomen**

Es wurden isolierte humane Lebermikrosomen (HLM) zur Beurteilung der metabolischen Stabilität von Verbindungen der allgemeinen Formel I eingesetzt.

Die Inkubationen wurden mit 2.4 ml HLM-lösung (0.5 mg/ml Proteingehalt), 30 $\mu$l der Testverbindung (finale Konzentration 1 $\mu$M) und 0.6 ml des Cofaktorgemisches (=NADPH-generierendem System aus 3 IU Glucose-6-phosphatdehydrogenase, 14.6 mg Glucose-6-phosphat, 1.2 mg NADP) bei 37°C in 100 mM Phosphatpuffer bei pH 7.4 durchgeführt. Es wurden zu 6 Zeitpunkten (2 - 60 min) Proben entnommen, mit gleichem Volumen Methanol gefällt und der Wiederfund der eingesetzten Testsubstanzen im Überstand mittels LC-MS/MS-Analytik ermittelt. Aus der daraus ermittelten Halbwertzeit des Substanzabbaus wurde die sogenannte intrinsische Clearance der Substanz im Lebermikrosomenansatz berechnet. Mit Hilfe dieser wurde unter Zuhilfenahme verschiedener physiologischer Kenngrößen (humaner Leberblutfluss: 1.3 L*kg/h; spezifisches Lebergewicht (pro kg Körpergewicht): 21g/kg; mikrosomaler Proteingehalt: 40mg/g Leber) nach dem well-stirred Modell eine (metabolische) *in vivo* Clearance in Bezug auf Phase I Reaktionen vorhersagt. Weiterhin wurde unter den Annahmen, dass (i) die Absorption der Prüfsubstanz 100% beträgt, und (ii) der First pass vollständig vom Lebermikrosomenmetabolismus abgebildet wird, eine maximale orale Bioverfügbarkeit (Fmax) errechnet.

Die getesteten Verbindungen weisen eine überraschend hohe metabolische Stabilität (kleine auf Basis der in vitro Daten vorhergesagte "Clearance" -Rate) und eine gute vorhergesagte maximale orale Bioverfügbarkeit $F_{max}$ auf (Tabelle 2).

Zusätzlich weisen einige Verbindungen eine für diese Wirkstoffklasse ungewöhnlich gute Löslichkeit in wässrigem Medium unter physiologischen Bedingungen auf (Tabelle 2).

Tabelle 2:

| Bsp. | vorhergesagte Clearance [l/h/kg] | | vorhergesagte $F_{max}$ [%] (maximale orale Bioverfügbarkeit) | | Löslichkeit bei pH 7,4 [mg/l] |
|---|---|---|---|---|---|
| | Human | Ratte | Human | Ratte | |
| Ref. 1* | 0,7 | 0,8 | 42 | 80 | 17 |
| 1 | 0,32 | 0,9 | 76 | 78 | 13 |
| 2 | 0,00 | 0,0 | 100 | 100 | 178 |

(fortgesetzt)

| Bsp. | vorhergesagte Clearance [l/h/kg] | | vorhergesagte $F_{max}$ [%] (maximale orale Bioverfügbarkeit) | | Löslichkeit bei pH 7,4 [mg/l] |
|---|---|---|---|---|---|
| | Human | Ratte | Human | Ratte | |
| 4 | 0,09 | 0,2 | 93 | 96 | 8 |
| 5 | 0,17 | 0,9 | 87 | 78 | 8 |
| 11 | 0,49 | 0,2 | 63 | 94 | 30 |
| 13A | 0,31 | 0,0 | 77 | 100 | 148 |
| 16 | 0,33 | 0,0 | 75 | 100 | 204 |
| 17 | 0,08 | 0,0 | 94 | 100 | 204 |
| 24A | 0,88 | 2,2 | 34 | 49 | 5 |
| 25A | 0,70 | 0,9 | 47 | 79 | 5 |
| 31 | 1,13 | 0,9 | 15 | 78 | 10 |
| 34 | 1,21 | 1,4 | 9 | 68 | 7 |
| 36 | 0,60 | 0,5 | 55 | 88 | 4 |
| * Ref. 1 Tabelle 2: Als Vergleichsverbindung (Referenzsubstanz) dient die in Beispiel 10 der WO 1998/34947 beschriebene Verbindung (8S,11R,13S,14S,17S)-17-Hydroxy-11-[4-(hydroxymethyl)-phenyl]-13-methyl-17-pentafluorethyl-1,2,6,7,8,11,12,13,14,15,16,17-dodecahydro-cyclopenta[a]phenanthren-3-on. | | | | | |

Die Vergleichsverbindung weist eine gute vorhergesagte maximale Bioverfügbarkeit in der Ratte, nicht aber im Menschen auf.

Besonders bevorzugt sind daher solche Verbindungen, die eine vorhergesagte maximale orale Bioverfügbarkeit bei unterschiedlichen Spezies (Ratte, Mensch) aufweisen, die jeweils größer 50% ist.

Ganz besonders bevorzugt sind solche Verbindungen, die eine vorhergesagte maximale orale Bioverfügbarkeit bei unterschiedlichen Spezies (Ratte, Mensch) aufweisen, die jeweils größer 70% ist. Beispielsweise genannt seien Verbindungen der Beispiele 1, 2, 4, 5, 13A, 16 und 17.

Ebenfalls besonders bevorzugt sind Verbindungen mit einer verbesserten Löslichkeit. Beispielsweise genannt seien Verbindungen der Beispiele 2, 11, 13A, 16 und 17.

**Beispiel 62**

**Bestimmung der Clearance und der Halbwertszeit nach intravenöser Applikation in Ratten**

Die Bestimmung der in vivo Clearance und Halbwertzeit von Prüfsubstanzen wurde in weiblichen Ratten mit einem Körpergewicht von ca. 200-250 g durchgeführt. Hierzu wurden die Prüfsubstanzen (bei Cassettenversuch bis zu 3 verschiedene Substanzen pro Tier) in gelöster Form bei einer Dosis von 0.3-0.5 mg/kg als bolus im Volumen von 2 ml/kg intravenös (i.v.) in die Schwanzvene appliziert, wobei verträgliche Lösungsvermittler wie PEG400 und/oder Ethanol in verträglicher Menge verwendet wurden. Aus einem Polyurethan-Katheter in der Vena jugularis wurden zu den Zeitpunkten 2min, 8min, 15min, 30min, 45min, 1h, 2h, 4h, 6h, 8h, 24h ca. 0.2 mL Blutproben entnommen. Die Blutproben wurden ohne Schütteln in Lithium-Heparinröhrchen (Monovetten® von Sarstedt) aufbewahrt und für 15min bei 3000Upm zentrifugiert. Ein Aliquot von 100µL wurde dem Überstand (Plasma) entnommen und durch Zugabe von 400µL kaltem Methanol gefällt. Die gefällten Proben wurden über Nacht bei -20°C ausgefroren, danach wiederum für 15min bei 3000UpM zentrifugiert bevor 150µL des klaren Überstandes zur Konzentrationsbestimmung abgenommen wurde. Die Analytik erfolgte durch ein Agilent 1200 HPLC-System mit angeschlossener LCMS/MS Detektion.

**Berechnung der PK Parameter (via nicht linearer Regression durch PK Berechnungssoftware):**

CLplasma: Gesamtplasmaclearance der Prüfsubstanz (in L*kg/h),
wobei CLplasma = Dosis / AUCinf;

AUCinf: extrapolierte Fläche unter der Plasmakonzentration-Zeit-Kurve (in µg*h/L);

CLblood: Gesamtblutclearance der Prüfsubstanz (in L*kg/h),

wobei (CLblood = CLplasma*Cp/Cb);

Cb/Cp: Verhältnis der Blut zu Plasma Konzentrationsverteilung der Prüfsubstanz;

T1/2: terminale Halbwertszeit der Prüfsubstanz (in h).

Tabelle 3

| Beispiel | $CL_{blood}$ [Uh/kg] | $t_{1/2}$ [h] |
|---|---|---|
| 1 | 0,9 ± 0,08 | 12,3 ± 0,3 |
| 4 | 3,3 ± 1,3 | 1,1 ± 0,7 |
| 16 | 2,1 ± 0,5 | 2,2 ± 0,4 |
| 17 | 0,7 ± 0,1 | 4,0 ± 0,8 |

Besonders bevorzugt sind Verbindungen, die eine terminale Halbwertszeit von mindestens 1 Stunde in der Ratte aufweisen. Beispielsweise genannt seien Verbindungen der Beispiele 1, 4, 16 und 17.

Ganz besonders bevorzugt sind Verbindungen, die eine terminale Halbwertszeit von mehr als 8 Stunden und gleichzeitig eine Blutclearance von weniger als 1,3 l/h/kg in der Ratte aufweisen. Beispielsweise genannt sei die Verbindung aus Beispiel 1.

**Beispiel 63**

**Abortivtest an weiblichen Ratten**

Die Wirkung von Progesteron und des Progesteron Rezeptors sind für eine erfolgreiche Schwangerschaft bzw. Trächtigkeit bei Säugetieren grundlegende Voraussetzung. Die Progesteron-antagonistische Wirkung der erfindungsgemäßen Verbindungen wurde an trächtigen Ratten (6 Ratten pro Gruppe) an Tag 5 bis 7 post coitum unter herkömmlichen Haltungs- und Fütterungsbedingungen getestet.

Nach erfolgreicher Anpaarung, wurden die trächtigen Tiere (Vorhandensein von Spermien im Vaginalabstrich an Tag 1 der Schwangerschaft = d1 p.c.) randomisiert und auf die Behandlungs- und Kontrollgruppe aufgeteilt. Die Tiere erhielten dann subkutan oder oral je 0,15; 0,5; 1,5 oder 5 mg/kg der Testverbindung oder 1,0 ml/kg Vehikel (Benzylbenzoat/Rhizinusöl: 1+4 [v/v]) täglich von Tag 5 bis Tag 7 (d5 - d7 p.c.).

Die Autopsie wurde an Tag 9 (d9 p.c.) durchgeführt. Als Kenngröße der Progesteronrezeptor-antagonistischen Wirkung wurde der Uterus auf das Vorhandensein von Nidationsstellen untersucht. Dabei wurde das völlige Fehlen, aber auch das Vorhandensein pathologischer, hämorrhagischer oder sonst abnormer Nidationsstellen an Tag 9 (d9 p.c.) als Abort gewertet. Die Ergebnisse der Tests sind in Tabelle 4 dargestellt.

Tabelle 4:

| Testverbindung | Tagesdosis [mg/kg] p.o. | Abortrate [%] |
|---|---|---|
| Vehikel | | 0 |
| Beispiel 1 | 0,5 | 100 |
| | 1,5 | 100 |
| | 5,0 | 100 |
| Beispiel 16 | 0,5 | 0 |
| | 1,5 | 16,7 |
| | 5,0 | 100 |

**Patentansprüche**

1. 17-Hydroxy-17-pentafluorethyl-estra-4,9(10)-dien-11-ethinylphenyl-Derivate der Formel I

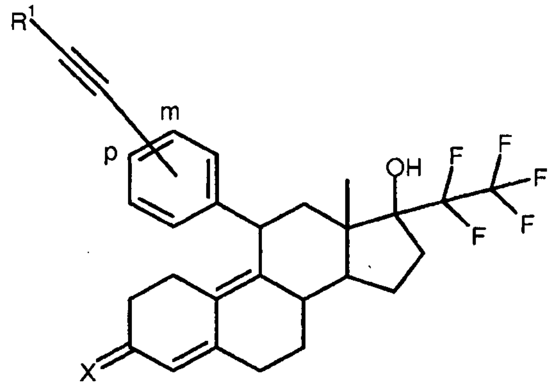

(I)

worin

$R^1$ über eine C-C-Dreifachbindung in Position m oder p am Phenylring angeknüpft ist und für einen Rest $-(CH=CH)_n-R^2$, $-(CH_2)_q-R^3$ oder $-CH=NOR^4$ steht,
$R^2$ Wasserstoff oder eine Aryl-, $C_1$-$C_{10}$-Alkyl-, $-CO_2R^6$ oder -CN Gruppe ist,
$R^3$ Wasserstoff, $NH_2$, $N_3$ oder eine $-NHCONHR^4$, $-OCONHR^4$, $-OR^5$ Gruppe ist,
$R^4$ ausgewählt ist aus der Gruppe, umfassend Wasserstoff, $C_1$-$C_{10}$-Alkyl, $C_2$-$C_{10}$-Alkenyl, Aryl, $C_7$-$C_{20}$-Aralkyl, $(CH_2)_s-R^6$, $CH_2-CO-OR^6$, worin

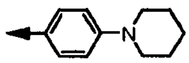 ,

$R^5$ ausgewählt ist aus der Gruppe, umfassend Wasserstoff, $C_7$-$C_{20}$-Aralkyl, $CH_2CO_2R^6$, $CH_2CN$, $CH_2CH_2OH$,

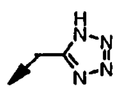 , 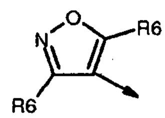

n für 0 bis 2,
q für 1 oder 2,
s für 1 oder 2,
$R^6$ für Wasserstoff, $C_1$-$C_{10}$-Alkyl, Aryl, $C_7$-$C_{20}$-Aralkyl,

X für Sauerstoff, $NOR^6$ oder eine $NNHSO_2R^6$ Gruppe, mit $R^6$ in der angegebenen Bedeutung, steht,

worin
Alkyl in R2, R4 und R6 sind gerad- oder verzweigtkettige Alkylgruppen mit der angegebenen Anzahl an Kohlenstoffatomen oder gegebenenfalls 1-10 Kohlenstoffatomen zu verstehen und beliebig substituiert sein können mit perhalogen, Hydroxygruppen, C1-C4-Alkoxygruppen, oder C6-C12-Arylgruppen (die wiederum durch 1-3 Halogenatome substituiert sein können),
Alkenyl in $R^4$ sind gerad- oder verzweigtkettige Alkenylgruppen mit 2-10 Kohlenstoffatomen zu verstehen und beliebig substituiert sein können mit 1-5 Halogenatome, Hydroxygruppen, $C_1$-$C_3$-Alkoxygruppen oder $C_6$-$C_{12}$-Arylgruppen (die wiederum durch 1-3 Halogenatome substituiert sein können), Aryl in $R^2$, $R^4$ und $R^6$ sind aromatische, mono- oder bicyclische Reste zu verstehen, wie zum Beispiel Phenyl oder Naphthyl und beliebig substituiert sein können mit einfach oder mehrfach Halogen, OH, $SO_2$-Alkyl, SO-Alkyl und S-Alkyl, O-Alkyl, $CO_2H$ $CO_2$-Alkyl, $NH_2$, $NO_2$, $N_3$, CN, $C_1$-$C_{10}$-Alkyl, $C_1$-$C_{10}$-Acyl oder $C_1$-$C_{10}$-Acyloxy-Gruppen,
Aralkyl in $R^4$, $R^5$ und $R^6$ sind Aralkylgruppen zu verstehen worin Aralkyl steht für Aralkylgruppen, die im Ring bis

zu 14 Kohlenstoffatome und in der Alkylkette 1-8, Kohlenstoffatome und beliebig substituiert sein können mit einfach oder mehrfach Halogen, OH, O-Alkyl, $CO_2H$, $CO_2$-Alkyl $NH_2$, $NH(C_1$-$C_{10}$-Alkyl), N(C,-$C_{10}$-Alkyl$)_2$, $NO_2$, $N_3$, CN, $C_1$-$C_{20}$-Alkyl, $C_1$-$C_{10}$-Perfluor-Alkyl, $C_1$-$C_{20}$-Acyl oder $C_1$-$C_{20}$-Acyloxy-Gruppen,
sowie ihre Salze, Solvate oder Solvate der Salze, einschließlich aller Kristallmodifikationen, die $\alpha$-, $\beta$- oder $\gamma$-Cyclodextrinclathrate, sowie die mit Liposomen verkapselten Verbindungen.

**2.** Verbindungen nach Anspruch 1 worin X für Sauerstoff steht.

**3.** Verbindungen nach Anspruch 1 worin die Gruppe

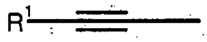

in para Position an den Phenylring geknüpft ist.

**4.** Verbindungen nach einem der Ansprüche 1 - 3 vorliegend als Solvat.

**5.** Verbindungen nach Anspruch 1 oder 2, worin $R^1$ für eine -$(CH=CH)_n$-$R^2$ oder eine - $(CH_2)_q$-$R^3$ Gruppe, mit n, q, $R^2$ und $R^3$ in den angegebenen Bedeutungen, steht.

**6.** Verbindungen nach Anspruch 1 oder 2, worin $R^2$ eine Gruppe COOH und $R^3$ eine Gruppe -$OR^5$ mit $R^5$ in der Bedeutung von $CH_2CO_2H$ oder $CH_2CH_2OH$ ist.

**7.** Verbindungen nach einem der Ansprüche 1 - 4 worin die Gruppe $R^1$ endständig eine Carbonsäuregruppe oder eine Alkoholgruppe trägt.

**8.** Verbindungen nach Anspruch 1, nämlich

- ➢ (8S,11R,13S,14S,17S)-17-Hydroxy-11-[4-(3-hydroxy-prop-1-in-1-yl)-phenyl]-13-methyl-17-pentafluorethyl-1,2,6,7,8,11,12,13,14,15,16,17-dodecahydro-cyclopenta[a]phenanthren-3-on
- ➢ (E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-pent-2-en-4-innitril
- ➢ (Z)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-pent-2-en-4-innitril
- ➢ (8S,11R,13S,14S,17S)-17-Hydroxy-11-{4-[3-(2-hydroxy-ethoxy)-prop-1-in-1-yl]-phenyl}-13-methyl-17-pentafluorethyl-1,2,6,7,8,11,12,13,14,15,16,17-dodecahydro-cyclopenta[a] phenanthren-3-on
- ➢ 3-{(E)-4-[4-((8S,11R,13S,14S,17S)-17-Hydroxy-13-methy)-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäure
- ➢ {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxy}-acetonitril
- ➢ 4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure 3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester
- ➢ (8S,11R,13S,14S,17S)-11-(4-Ethinyl-phenyl)-17-hydroxy-13-methyl-17-pentafluorethyl-1,2,6,7,8,11,12,13,14,15,16,17-dodecahydro-cyclopenta[*a*]phenanthren-3-on
- ➢ (8S,11R,13S,14S,17S)-11-[4-(3-Azido-prop-1-in-1-yl)-phenyl]-17-hydroxy-13-methyl-17-pentafluorethyl-1,2,6,7,8,11,12,13,14,15,16,17-dodecahydro-cyclopenta[a]phenanthren-3-on
- ➢ (E)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal O-benzyl-oxim und (Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-benzyl-oxim
- ➢ (E)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-propinal *O*-ethyl-oxim und (Z)-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phehanthren-11-yl)-phenyl]-propinal *O*-ethyl-oxim
- ➢ [4-((8S,11R,13S,14S,17S)-17-Hydroxy-13-methyl-3-oxo-17-pentafluorethyl-2,3,6,7,8,11,12,13,14,15,16,

17-dodecahydro-1*H*-cyclopehta[*a*]phenanthren-11-yl)-phenyl]-propinal *O*-isobutyl-oxim

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-isopropyl-harnstoff

➢ 3-(3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-ureido)-propionsäureethylester

➢ 1-Ethyl-3-{3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-methoxy-phenyl)-harnstoff

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-phenyl-harnstoff

➢ 1-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[*a*]phenanthren-11-yl)-phenyl]-prop-2-inyl}-3-(4-fluor-phenyl)-harnstoff

➢ 1-Benzyl-3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-harnstoff

➢ 1-Allyl-3-{3-[4-((8S,11R,13S,14S,17S)-17-Hydroxy-13-methyl-3-oxo-17-pentafluorethyl-2,3,6,7,8,11,12,13,14,15,16,17-dodeoahydro-1*H*-cyclopenta[*a*]phenanthren-11-yl)-phenyl]-prop-2-inyl}-harnstoff

➢ Allyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

➢ Ethyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[*a*]phenanthren-11-yl)-phenyl]-prop-2-inyl ester)

➢ Phenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

➢ 4-Methylphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*] phenanthren-11-yl)-phenyl]-prop-2-inyl ester

➢ 4-Fluorphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1 *H*-cyclopenta[*a*]phenanthren-11-yl)-phenyl]-prop-2-inyl ester

➢ Isopropyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

➢ Benzyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-prop-2-inyl ester

➢ (8S,11R,13S,14S,17S)-17-Hydroxy-11-[4-(3-methanesulfonyl-phenylethinyl)-phenyl]-13-methyl-17-pentafluorethyl-1,2,6,7,8,11,12,13,14,15,16,17-dodecahydro-cyclopenta[a]phenanthren-3-on

➢ 4-Methoxyphenyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenca[*a*] phenanthren-11-yl)-phenyl]-prop-2-inyl ester

➢ 4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyloxycarbonylamino}-benzoesäureethylester

➢ (4-Piperidin-1-yl-phenyl)-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

➢ (8S,11R,13S,14S,17S)-17-Hydroxy-11-[4-(4-methanesulfonyl-phenylethinyl)-phenyl]-13-methyl-17-pentafluorethyl-1,2,6,7,8,11,12,13,14,15,16,17-dodecahydro-cyclopenta[*a*]phenanthren-3-on

➢ 3-Pyridyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

➢ *tert*-Butyl-carbaminsäure 3-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl ester

**9.** Verfahren zur Herstellung der Verbindungen der allgemeinen Formel I, **dadurch gekennzeichnet, dass** man ein Phenylsulfonat der allgemeinen Formel II,

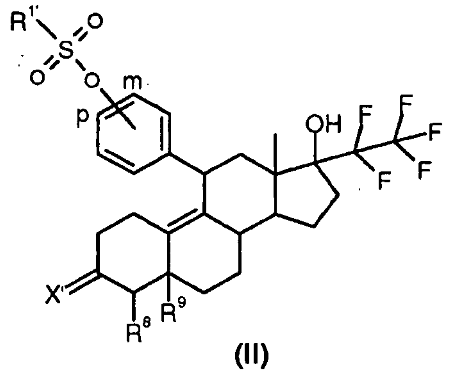

(II)

worin

$R^{1'}$ eine perfluorierte $C_1$-$C_{10}$-Alkylgruppe,
X' ein Sauerstoffatom, zwei Alkoxygruppen $OR^7$, eine $C_2$-$C_{10}$-Alkylen-$\alpha,\omega$-dioxygruppe, die geradkettig oder verzweigt sein kann,
$R^7$ $C_1$-$C_4$-Alkyl,
R8 Wasserstoff,
$R^9$ eine Hydroxylgruppe, oder
$R^8$, $R^9$ gemeinsam eine Bindung

darstellen durch Palladium katalysierte Kupplungsreaktionen in eine Verbindung der allgemeinen Formel I'

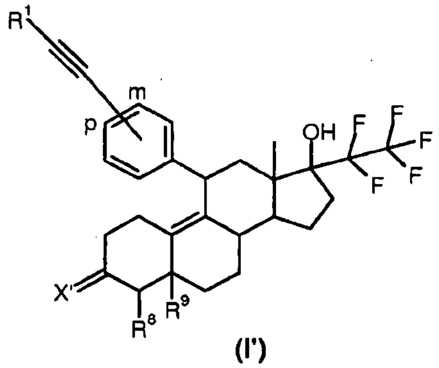

(I')

worin
$R^1$, X', $R^8$ und $R^9$ die oben genannten Bedeutungen haben, umsetzt und gegebenenfalls in $R^1$ vorhandene Funktionalitäten umwandelt und/oder weitere Folgereaktionen zu Verbindungen der allgemeinen Formel I' durchführt und anschließend aus der Gruppe X' eine Gruppe X mit X in der Bedeutung von Sauerstoff freisetzt und diese Carbonylgruppe (X = Sauerstoff) gegebenenfalls weiter funktionalisiert und zu einer Gruppe mit X in der Bedeutung von $NOR^6$ oder $NNHSO_2R^6$ umsetzt und/oder für den Fall das $R^8$ für Wasserstoff und $R^9$ für eine Hydroxylgruppe steht, durch Eliminierung von Wasser eine Doppelbindung erzeugt und $R^8$ und $R^9$ somit eine gemeinsam Bindung darstellen
worin
Alkyl sind gerad- oder verzweigtkettige Alkylgruppen mit der angegebenen Anzahl an Kohlenstoffatomen oder gegebenenfalls 1-10 Kohlenstoffatomen zu verstehen und beliebig substituiert sein können mit perhalogen, Hydroxygruppen, C1-C4-Alkoxygruppen, oder C6-C12-Arylgruppen (die wiederum durch 1-3 Halogenatome substituiert sein können),
Alkenyl sind gerad- oder verzweigtkettige Alkenylgruppen mit 2-10 Kohlenstoffatomen zu verstehen und beliebig substituiert sein.können mit 1-5 Halogenatome, Hydroxygruppen, $C_1$-$C_3$-Alkoxygruppen oder $C_6$-$C_{12}$-Arylgruppen (die wiederum durch 1-3 Halogenatome substituiert sein können), Aryl sind aromatische, mono- oder bicyclische Reste zu verstehen, wie zum Beispiel Phenyl oder Naphthyl und beliebig substituiert sein können mit einfach oder mehrfach Halogen, OH, $SO_2$-Alkyl, SO-Alkyl und S-Alkyl, O-Alkyl, $CO_2H$, $CO_2$-Alkyl, $NH_2$, $NO_2$, $N_3$, CN, $C_1$-$C_{10}$-Alkyl,

$C_1$-$C_{10}$-Acyl oder $C_1$-$C_{10}$-Acyloxy-Gruppen,
Aralkyl sind Aralkylgruppen zu verstehen worin Aralkyl steht für Aralkylgruppen, die im Ring bis zu 14 Kohlenstoffatome und in der Alkylkette 1-8, Kohlenstoffatome und beliebig substituiert sein können mit einfach oder mehrfach Halogen, OH, O-Alkyl, $CO_2H$, $CO_2$-Alkyl, $NH_2$, NH($C_1$-$C_{10}$-Alkyl), N($C_1$-$C_{10}$-Alkyl$)_2$, $NO_2$, $N_3$, CN, $C_1$-$C_{20}$-Alkyl, $C_1$-$C_{10}$-Perfluor-Alkyl, $C_1$-$C_{20}$-Acyl oder $C_1$-$C_{20}$-Acyloxy-Gruppen.

**10.** Verbindungen nach einem der Ansprüche 1-8, die eine Progesteronrezeptor-antagonistische Wirkung in stabilen Transfektanten humaner Neuroblastoma-Zellen zeigen.

**11.** Verbindungen nach Anspruch 1
(8S,11R,13S,14S,17S)-17-Hydroxy-11-[4-(3-hydroxy-prop-1-in-1-yl)-phenyl]-13-methyl-17-pentafluorethyl-1,2,6,7,8,11,12,13,14,15,16,17-dodecahydro-cyclopenta[a]phenanthren-3-on,
[4-((8S,11R,13S,14S,17S)-17-Hydroxy-13-methyl-3-oxo-17-pentafluorethyl-2,3,6,7,8,11,12,13,14,15,16,17-dodecahydro- 1*H*-cyclopenta[a]phenanthren-11-yl)-phenyl]-propinsäure,
(E)-5-[4-((8S,11 R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril,
(Z)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril,
(8S,11R,13S,14S,17S)-17-Hydroxy-11-{4-[3-(2-hydroxy-ethoxy)-prop-1-in-1-yl]-phenyl}-13-methyl-17-pentafluorethyl-1,2,6,7,8,11,12,13,14,15,16,17-dodecahydro-cyclopenta[a] phenanthren-3-on,
3-{(E)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäure,
4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure, 3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester,
1-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-prop-2-inyl}-3-(4-fluor-phenyl)-harnstoff
die bei unterschiedlichen Spezies (Ratte, Mensch) eine vorhergesagte maximale orale Bioverfügbarkeit aufweisen, die jeweils größer 50% ist.

**12.** Verbindungen nach Anspruch 1
(8S,11R,13S,14S,17S)-17-Hydroxy-11-[4-(3-hydroxy-prop-1-in-1-yl)-phenyl]-13-methyl-17-pentafluorethyl-1,2,6,7,8,11,12,13,14,15,16,17-dodecahydro-cyclopenta[a]phenanthren-3-on,
[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-phenyl]-propinsäure,
(E)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril,
(Z)-5-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-pent-2-en-4-innitril,
3-{(E)-4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-but-1-en-3-inyl}-benzoesäure,
4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäure, 3-(4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenylethinyl]-benzoesäuremethylester die bei unterschiedlichen Spezies (Ratte, Mensch) eine vorhergesagte maximale orale Bioverfügbarkeit aufweisen, die jeweils größer 70% ist.

**13.** Pharmazeutische Präparate enthaltend mindestens eine Verbindung nach einem der Ansprüche 1-8 oder 10-12 oder deren Gemische sowie pharmazeutisch verträgliche Träger.

**14.** Verwendung der Verbindungen gemäß Anspruch 1-8 oder 10-12 zur Herstellung eines Arzneimittels.

**15.** Verwendung der Verbindungen gemäß Anspruch 1 zur Herstellung eines Arzneimittels zur Behandlung und/oder Prophylaxe von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

## Claims

**1.** 17-Hydroxy-17-pentafluoroethylestra-4,9(10)-diene 11-ethynylphenyl derivatives of the formula I

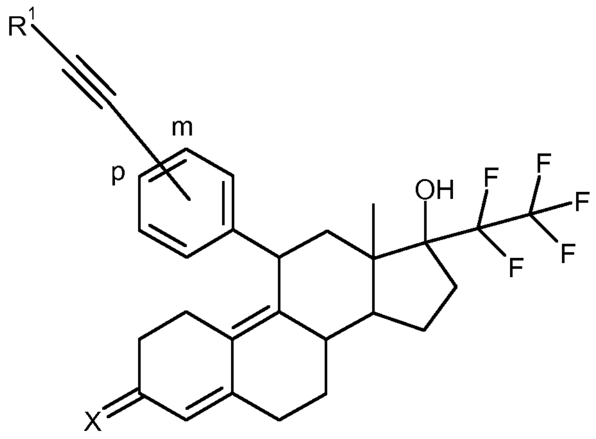

(I)

in which

$R^1$ is joined to the phenyl ring in the m or p position via a C-C triple bond and is a $-(CH{=}CH)_n-R^2$, $-(CH_2)_q-R^3$ or $-CH{=}NOR^4$ radical,
$R^2$ is hydrogen or an aryl, $C_1$-$C_{10}$-alkyl, $-CO_2R^6$ or -CN group,
$R^3$ is hydrogen, $NH_2$, $N_3$ or an $-NHCONHR^4$, $-OCONHR^4$, $-OR^5$ group,
$R^4$ is selected from the group comprising hydrogen, $C_1$-$C_{10}$-alkyl, $C_2$-$C_{10}$-alkenyl, aryl, $C_7$-$C_{20}$-aralkyl, $(CH_2)_s-R^6$, $CH_2-CO-OR^6$,

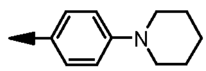

,

in which
$R^5$ is selected from the group comprising hydrogen, $C_7$-$C_{20}$-aralkyl, $CH_2CO_2R^6$, $CH_2CN$, $CH_2CH_2OH$,

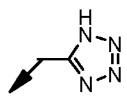

,

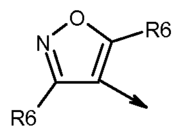

n is 0 to 2,
q is 1 or 2,
s is 1 or 2,
$R^6$ is hydrogen, $C_1$-$C_{10}$-alkyl, aryl, $C_7$-$C_{20}$-aralkyl,
X is oxygen, $NOR^6$ or an $NNHSO_2R^6$ group where $R^6$ is as defined above,
in which
alkyl in $R^2$, $R^4$ and $R^6$ are straight- or branched-chain alkyl groups with the specified number of carbon atoms or, as the case may be, 1-10 carbon atoms, which may have any substitution by perhalogen, hydroxyl groups, $C_1$-$C_4$-alkoxy groups or $C_6$-$C_{12}$-aryl groups (which may in turn be substituted by 1-3 halogen atoms),
alkenyl in $R^4$ are straight- or branched-chain alkenyl groups having 2-10 carbon atoms, which may have any substitution by 1-5 halogen atoms, hydroxyl groups, $C_1$-$C_3$-alkoxy groups or $C_6$-$C_{12}$-aryl groups (which may in turn be substituted by 1-3 halogen atoms),
aryl in $R^2$, $R^4$ and $R^6$ are aromatic mono- or bicyclic radicals, for example phenyl or naphthyl, which may have any mono- or polysubstitution by halogen, OH, $SO_2$-alkyl, SO-alkyl and S-alkyl, O-alkyl, $CO_2H$, $CO_2$-alkyl, $NH_2$, $NO_2$, $N_3$, CN, $C_1$-$C_{10}$-alkyl, $C_1$-$C_{10}$-acyl or $C_1$-$C_{10}$-acyloxy groups,
aralkyl in $R^4$, $R^5$ and $R^6$ are aralkyl groups in which aralkyl represents aralkyl groups which may contain up to

14 carbon atoms in the ring and 1-8 carbon atoms in the alkyl chain and may have any mono- or polysubstitution by halogen, OH, 0-alkyl, $CO_2H$, $CO_2$-alkyl, $NH_2$, NH ($C_1$-$C_{10}$-alkyl), N($C_1$-$C_{10}$-alkyl)$_2$, $NO_2$, $N_3$, CN, $C_1$-$C_{20}$-alkyl, $C_1$-$C_{10}$-perfluoroalkyl, $C_1$-$C_{20}$-acyl or $C_1$-$C_{20}$-acyloxy groups,
and the salts, solvates or solvates of the salts thereof, including all crystal polymorphs, the $\alpha$-, $\beta$- or $\gamma$-cyclodextrin clathrates, and the compounds encapsulated with liposomes.

**2.** Compounds according to Claim 1 in which X is oxygen.

**3.** Compounds according to Claim 1 in which the 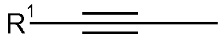 group is joined to the phenyl ring in the para position.

**4.** Compounds according to any of Claims 1 - 3 present in solvate form.

**5.** Compounds according to Claim 1 or 2, in which $R^1$ is a -(CH=CH)$_n$-$R^2$ or a -($CH_2$)$_q$ -$R^3$ group, where n, q, $R^2$ and $R^3$ are each as defined above.

**6.** Compounds according to Claim 1 or 2, in which $R^2$ is a COOH group and $R^3$ is a -$OR^5$ group where $R^5$ is defined as $CH_2CO_2H$ or $CH_2CH_2OH$.

**7.** Compounds according to any of Claims 1 - 4, in which the $R^1$ group bears a terminal carboxylic acid group or an alcohol group.

**8.** Compounds according to Claim 1, specifically

➢ (8S, 11R, 13S, 14S, 17S) -17-hydroxy-11- [4- (3-hydroxyprop-1-yn-1-yl)phenyl]-13-methyl-17-pentafluoroethyl-1,2,6,7,8,11,12,13, 14,15,16,17-dodecahydrocyclopenta[a]phenanthren-3-one
➢ (E)-5-[4-((8S,11R,135,145,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]pent-2-en-4-ynenitrile
➢ (Z)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]pent-2-en-4-ynenitrile
➢ (8S,11R,13S,14S,17S)-17-hydroxy-11-{4-[3-(2-hydroxyethoxy)prop-1-yn-1-yl]phenyl}-13-methyl-17-pentafluoroethyl-1,2,6,7,8,11,12,13,14,15,16,17-dodecahydrocyclopenta[a]phenanthren-3-one
➢ 3-{(E)-4-[4-((8S,11R,135,145,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]but-1-en-3-ynyl}benzoic acid
➢ {3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyloxy}acetonitrile
➢ 4-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenylethynyl]benzoic acid 3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)phenylethynyl]benzoic acid methyl ester
➢ (8S,11R,13S,14S,17S)-11-(4-ethynylphenyl)-17-hydroxy-13-methyl-17-pentafluoroethyl-1,2,6,7,8,11,12,13,14,15,16,17-dodecahydrocyclopenta[a]phenanthren-3-one
➢ (8S,11R,13S,14S,17S)-11-[4-(3-azidoprop-1-yn-1-yl)phenyl]-17-hydroxy-13-methyl-17-pentafluoroethyl-1,2,6,7,8,11,12,13,14, 15,16,17-dodecahydrocyclopenta[a]phenanthren-3-one
➢ (E)-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]propynal *O*-benzyl oxime and (Z)-[4-((8S,11R,135,145,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]propynal *O*-benzyl oxime
➢ (E)-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]propynal *O*-ethyl oxime and (Z)-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]propynal *O*-ethyl oxime
➢ [4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]propynal *O*-isobutyl oxime
➢ 1-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}-3-isopropylurea
➢ 3-(3-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}ureido)propionic acid ethyl ester

➢ 1-ethyl-3-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}urea

➢ 1-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}-3-(4-methoxyphenyl)urea

➢ 1-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}-3-phenylurea

➢ 1-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}-3-(4-fluorophenyl)urea

➢ 1-benzyl-3-{3-[4-((8S,11R,13S,14S,17S)-17-hydroxy-13-methyl-3-oxo-17-pentafluoroethyl-2,3,6,7,8,11,12,13,14,15,16, 17-dodecahydro-1*H*-cyclopenta[*a*]phenanthren-11-yl)phenyl]prop-2-ynyl}urea

➢ 1-allyl-3-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl}urea

➢ allylcarbamic acid 3-[4-((8S,11R,13S,14S,17S)-17-hydroxy-13-methyl-3-oxo-17-pentafluoroethyl-2,3,6,7,8,11,12,13,14,15, 16,17-dodecahydro-1*H*-cyclopenta[*a*]phenanthren-11-yl)phenyl]prop-2-ynyl ester

➢ ethylcarbamic acid 3-[4-((8S,11R,13S,14S,17S)-17-hydroxy-13-methyl-3-oxo-17-pentafluoroethyl-2,3,6,7,8,11,12,13,14,15, 16,17-dodecahydro-1*H*-cyclopenta[*a*]phenanthren-11-yl)phenyl]prop-2-ynyl ester

➢ phenylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

➢ 4-methylphenylcarbamic acid 3-[4-((8S,11R,13S,14S,17S)-17-hydroxy-13-methyl-3-oxo-17-pentafluoroethyl-2,3,6,7,8,11, 12,13,14,15,16,17-dodecahydro-1*H*-CyClopenta[*a*] phenanthren-11-yl)phenyl]prop-2-ynyl ester

➢ 4-fluorophenylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

➢ isopropylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

➢ benzylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

➢ (8S,11R,13S,14S,17S)-17-hydroxy-11-[4-(3-methanesulphonyl-phenylethynyl)phenyl]-13-methyl-17-pentafluoroethyl-1,2,6,7,8, 11,12,13,14,15,16,17-dodecahydrocyclopenta[a]phenanthren-3-one

➢ 4-methoxyphenylcarbamic acid 3-[4-((8S,11R,13S,14S,17S)-17-hydroxy-13-methyl-3-oxo-17-pentafluoroethyl-2,3,6,7,8,11, 12,13,14,15,16,17-dodecahydro-1*H*-cyclopenta[*a*] phenanthren-11-yl)phenyl]prop-2-ynyl ester

➢ 4-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyloxycarbonylamino}benzoic acid ethyl ester

➢ (4-piperidin-1-ylphenyl)carbamic acid 3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]prop-2-ynyl ester

➢ (8S,11R,13S,14S,17S)-17-hydroxy-11-[4-(4-methanesulphonylphenylethynyl)phenyl]-13-methyl-17-pentafluoroethyl-1,2,6,7,8,11,12,13,14,15,16,17-dodecahydrocyclopenta[a]phenanthren-3-one

➢ 3-pyridylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

➢ *tert*-butylcarbamic acid 3-[4-((8S,11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]prop-2-ynyl ester

**9.** Process for preparing the compounds of the general formula I, **characterized in that** a phenyl sulphonate of the general formula II

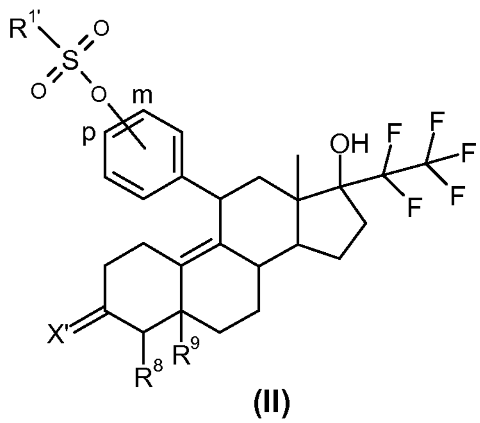

**(II)**

in which

$R^{1'}$ is a perfluorinated $C_1$-$C_{10}$-alkyl group, X' is an oxygen atom, two alkoxy groups $OR^7$, a $C_2$-$C_{10}$-alkylene-□, □-dioxy group which may be straight-chain or branched,
$R^7$ is $C_1$-$C_4$-alkyl,
$R^8$ is hydrogen,
$R^9$ is a hydroxyl group, or
$R^8$, $R^9$ together are a bond,
is converted by palladium-catalysed coupling reactions to a compound of the general formula I'

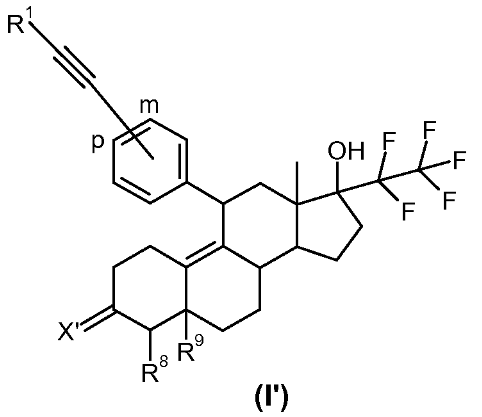

**(I')**

in which
$R^1$, X', $R^8$ and $R^9$ are each as defined above, and any functionalities present in $R^1$ are converted and/or further conversion reactions are conducted to give compounds of the general formula I', and then an X group where X is defined as oxygen is released from the X' group and this carbonyl group (X = oxygen) is optionally functionalized further and converted to a group where X is defined as $NOR^6$ or $NNHSO_2R^6$ , and/or, in the case that $R^8$ is hydrogen and $R^9$ is a hydroxyl group, a double bond is obtained by elimination of water and $R^8$ and $R^9$ thus constitute a common bond,
in which
alkyl are straight- or branched-chain alkyl groups with the specified number of carbon atoms or, as the case may be, 1-10 carbon atoms, which may have any substitution by perhalogen, hydroxyl groups, $C_1$-$C_4$-alkoxy groups or $C_6$-$C_{12}$-aryl groups (which may in turn be substituted by 1-3 halogen atoms), alkenyl are straight- or branched-chain alkenyl groups having 2-10 carbon atoms, which may have any substitution by 1-5 halogen atoms, hydroxyl groups, $C_1$-$C_3$-alkoxy groups or $C_6$-$C_{12}$-aryl groups (which may in turn be substituted by 1-3 halogen atoms),
aryl are aromatic mono- or bicyclic radicals, for example phenyl or naphthyl, which may have any mono- or polysubstitution by halogen, OH, $SO_2$-alkyl, SO-alkyl and S-alkyl, 0-alkyl, $CO_2H$, $CO_2$-alkyl, $NH_2$, $NO_2$, $N_3$, CN, $C_1$-$C_{10}$-alkyl, $C_1$-$C_{10}$-acyl or $C_1$-$C_{10}$-acyloxy groups.
aralkyl are aralkyl groups in which aralkyl represents aralkyl groups which may contain up to 14 carbon atoms

in the ring and 1-8 carbon atoms in the alkyl chain and may have any mono- or polysubstitution by halogen, OH, O-alkyl, $CO_2H$, $CO_2$-alkyl, $NH_2$, NH ($C_1$-$C_{10}$-alkyl), N ($C_1$-$C_{10}$-alkyl)$_2$, $NO_2$, $N_3$, CN, $C_1$-$C_{20}$-alkyl, $C_1$-$C_{10}$-perfluoroalkyl, $C_1$-$C_{20}$-acyl or $C_1$-$C_{20}$-acyloxy groups.

**10.** Compounds according to any of Claims 1-8 which exhibit progesterone receptor-antagonistic action in stable transfectants of human neuroblastoma cells.

**11.** Compounds according to Claim 1 (8S,11R,13S,14S,17S)-17-hydroxy-11-[4-(3-hydroxyprop-1-yn-1-yl)phenyl]-13-methyl-17-pentafluoroethyl-1,2,6,7,8,11,12,13, 14,15,16,17-dodecahydrocyclopenta[a]phenanthren-3-one, [4-((8S,11R,13S,14S,17S)-17-hydroxy-13-methyl-3-oxo-17-pentafluoroethyl-2,3,6,7,8,11,12,13,14,15,16,17-dodecahydro-*1H*-cyclopenta[a]phenanthren-11-yl)phenyl]propynoic acid, (E)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]pent-2-en-4-ynenitrile, (Z)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]pent-2-en-4-ynenitrile, (8S,11R,13S,14S,17S)-17-hydroxy-11-{4-[3-(2-hydroxyethoxy)prop-1-yn-1-yl]phenyl}-13-methyl-17-pentafluoroethyl-1,2,6,7,8,11,12,13,14,15,16,17-dodecahydrocyclopenta[a]phenanthren-3-one, 3-{(E)-4-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]but-1-en-3-ynyl}benzoic acid, 4-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenylethynyl]benzoic acid, 3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenylethynyl]benzoic acid methyl ester, 1-{3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)phenyl]prop-2-ynyl}-3-(4-fluorophenyl)urea, which, in different species (rats, humans), have a predicted maximum oral bioavailability which is in each case greater than 50%.

**12.** Compounds according to Claim 1 (8S,11R,13S,14S,17S)-17-hydroxy-11-[4-(3-hydroxyprop-1-yn-1-yl)phenyl]-13-methyl-17-pentafluoroethyl-1,2,6,7,8,11,12,13, 14,15,16,17-dodecahydrocyclopenta[a]phenanthren-3-one, [4-((8S,11R,13S,14S,17S)-17-hydroxy-13-methyl-3-oxo-17-pentafluoroethyl-2,3,6,7,8,11,12,13,14,15,16,17-dodecahydro-*1H*-cyclopenta[*a*]phenanthren-11-yl)phenyl]propynoic acid, (E)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)phenyl]pent-2-en-4-ynenitrile, (Z)-5-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]pent-2-en-4-ynenitrile, 3-{(E)-4-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[a]phenanthren-11-yl)phenyl]but-1-en-3-ynyl}benzoic acid, 4-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenylethynyl]benzoic acid, 3-[4-((8S,11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenylethynyl]benzoic acid methyl ester, which, in different species (rats, humans), have a predicted maximum oral bioavailability which is in each case greater than 70%.

**13.** Pharmaceutical formulations comprising at least one compound according to any of Claims 1-8 or 10-12 or mixtures thereof, and pharmaceutically suitable carriers.

**14.** Use of the compounds according to Claim 1-8 or 10-12 for production of a medicament.

**15.** Use of the compounds according to Claim 1 for production of a medicament for treatment and/or prophylaxis of fibroids of the uterus (myomas, uterine leiomyomas), endometriosis, heavy menstrual bleeds, meningiomas, hormone-dependent breast cancers and complaints associated with the menopause, or for fertility control and emergency contraception.

**Revendications**

**1.** Dérivés de 17-hydroxy-17-pentafluoroéthyl-estra-4,9(10)-dién-11-éthynylphényle de formule I

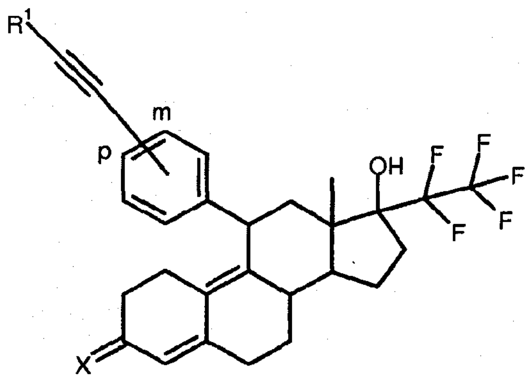

(I)

dans laquelle

$R^1$ est attaché via une triple liaison C-C en position m ou p au cycle phényle et représente un radical $-(CH=CH)_n-R^2$, $-(CH_2)_q-R^3$ ou $-CH=NOR^4$,
$R^2$ représente un atome d'hydrogène ou un groupe aryle, alkyle en $C_1$-$C_{10}$, $-CO_2R^6$ ou -CN,
$R^3$ représente un atome d'hydrogène, $NH_2$, $N_3$ ou un groupe $-NHCONHR^4$, $-OCONHR^4$, $-OR^5$,
$R^4$ est choisi dans le groupe comprenant un atome d'hydrogène, un groupe alkyle en $C_1$-$C_{10}$, alcényle en $C_2$-$C_{10}$, aryle, aralkyle en $C_7$-$C_{20}$, $(CH_2)_s-R^6$, $CH_2-CO-OR^6$,

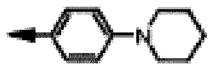,

où

$R^5$ est choisi dans le groupe comprenant un atome d'hydrogène, un groupe aralkyle en $C_7$-$C_{20}$, $CH_2CO_2R^6$, $CH_2CN$, $CH_2CH_2OH$,

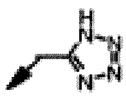, 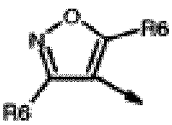

n représente 0 à 2,
q représente 1 ou 2,
s représente 1 ou 2,
$R^6$ représente un atome d'hydrogène, un groupe alkyle en $C_1$-$C_{10}$, aryle, aralkyle en $C_7$-$C_{20}$,

X représente un atome d'oxygène, $NOR^6$ ou un groupe $NNHSO_2R^6$, où $R^6$ a la signification indiquée,

où
par alkyle dans $R^2$, $R^4$ et $R^6$ il faut entendre des groupes alkyle à chaîne droite ou ramifiée ayant le nombre indiqué d'atomes de carbone ou le cas échéant de 1 à 10 atomes de carbone et qui peuvent être substitués de façon quelconque par perhalogéno, par des groupes hydroxy, des groupes alcoxy en $C_1$-$C_4$ ou des groupes aryle en $C_6$-$C_{12}$ (qui peuvent à leur tour être substitués par 1-3 atomes d'halogène),
par alcényle dans $R^4$ il faut entendre des groupes alcényle à chaîne droite ou ramifiée ayant de 2 à 10 atomes de carbone et qui peuvent être substitués de façon quelconque par 1-5 atomes d'halogène, groupes hydroxy, groupes alcoxy en $C_1$-$C_3$ ou groupes aryle en $C_6$-$C_{12}$ (qui peuvent à leur tour être substitués par 1-3 atomes d'halogène),
par aryle dans $R^2$, $R^4$ et $R^6$ il faut entendre des radicaux aromatiques, mono- ou bicycliques, comme par exemple phényle ou naphtyle et qui peuvent être substitués de façon quelconque une ou plusieurs fois par halogéno, OH, $SO_2$-alkyle, SO-alkyle et S-alkyle, O-alkyle, $CO_2H$, $CO_2$-alkyle, $NH_2$, $NO_2$, $N_3$, CN, alkyle en $C_1$-$C_{10}$, acyle en $C_1$-$C_{10}$ ou acyloxy en $C_1$-$C_{10}$,

par aralkyle dans $R^4$, $R^5$ et $R^6$ il faut entendre des groupes aralkyle, aralkyle signifiant des groupes aralkyle qui comportent dans le cycle jusqu'à 14 atomes de carbone et dans la chaîne alkyle de 1 à 8 atomes de carbone et peuvent être substitués de façon quelconque une ou plusieurs fois par halogéno, OH, O-alkyle, $CO_2H$, $CO_2$-alkyle, $NH_2$, NH(alkyle($C_1$-$C_{10}$)), N(alkyle($C_1$-$C_{10}$))$_2$, $NO_2$, $N_3$, CN, alkyle en $C_1$-$C_{20}$, perfluoroalkyle($C_1$-$C_{10}$), acyle en $C_1$-$C_{20}$ ou acyloxy($C_1$-$C_{20}$),
ainsi que leurs sels, produits de solvatation ou produits de solvatation des sels, y compris toutes les formes cristallines, les clathrates avec des $\alpha$-, $\beta$- ou $\gamma$-cyclodextrines, ainsi que les composés encapsulés avec des liposomes.

**2.** Composés selon la revendication 1, dans lesquels X représente un atome d'oxygène.

**3.** Composés selon la revendication 1, dans lesquels le groupe

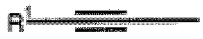

est attaché en position para au cycle phényle.

**4.** Composés selon l'une quelconque des revendications 1 à 3, présents sous forme de produit de solvatation.

**5.** Composés selon la revendication 1 ou 2, dans lesquels $R^1$ représente un groupe -(CH=CH)$_n$-$R^2$ ou un groupe ($CH_2$)$_q$-$R^3$, où n, q, $R^2$ et $R^3$ ont les significations indiquées.

**6.** Composés selon la revendication 1 ou 2, dans lesquels $R^2$ est un groupe COOH et $R^3$ est un groupe -$OR^5$ où $R^5$ a la signification de $CH_2CO_2H$ ou $CH_2CH_2OH$.

**7.** Composés selon l'une quelconque des revendications 1 à 4, dans lesquels le groupe $R^1$ porte en bout de chaîne un groupe acide carboxylique ou un groupe alcool.

**8.** Composés selon la revendication 1, à savoir

➢ (8S,11R,13S,14S,17S)-17-hydroxy-11-[4-(3-hydroxy-prop-1-yn-1-yl)-phényl]-13-méthyl-17-pentafluoroéthyl-1,2,6,7,8,11,12,13,14,15,16,17-dodécahydro-cyclopenta-[a]phénanthrén-3-one
➢ (E)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-pent-2-én-4-ynenitrile
➢ (Z)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-pent-2-én-4-ynenitrile
➢ (8S,11R,13S,14S,17S)-17-hydroxy-11-{4-[3-(2-hydroxy-éthoxy)-prop-1-yn-1-yl]-phényl}-13-méthyl-17-pentafluoroéthyl-1,2,6,7,8,11,12,13,14,15,16,17-dodécahydro-cyclopenta[*a*]phénanthrén-3-one
➢ acide 3-{(E)-4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-but-1-én-3-ynyl}-benzoïque
➢ {3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyloxy}-acétonitrile
➢ acide 4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoïque 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoate de méthyle
➢ (8S,11R,13S,14S,17S)-11-(4-éthynyl-phényl)-17-hydroxy-13-méthyl-17-pentafluoroéthyl-1,2,6,7,8,11,12,13,14,15,16,17-dodécahydro-cyclopenta[*a*]phénanthrén-3-one
➢ (8S,11R,13S,14S,17S)-11-[4-(3-azido-prop-1-yn-1-yl)-phényl]-17-hydroxy-13-méthyl-17-pentafluoroéthyl-1,2,6,7,8,11,12,13,14,15,16,17-dodécahydrocyclopenta[a]phénanthrén-3-one
➢ (E)-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-propynal O-benzyl-oxime et (Z)-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-propynal O-benzyl-oxime
➢ (E)-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-propynal O-éthyl-oxime et (Z)-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-propynal O-éthyl-oxime

➢ [4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-propynal O-isobutyl-oxime

➢ 1-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-3-isopropyl-urée

➢ 3-(3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-uréido)-propionate d'éthyle

➢ 1-éthyl-3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-urée

➢ 1-{3-[4-({8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-3-(4-méthoxy-phényl)-urée

➢ 1-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-3-phényl-urée

➢ 1-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-3-(4-fluoro-phényl)-urée

➢ 1-benzyl-3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-urée

➢ 1-allyl-3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-urée

➢ Allyl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ éthyl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ phényl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ 4-méthylphényl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ 4-fluorophényl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ isopropyl-carbamate 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ benzylcarbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ (8S,11R,13S,14S,17S)-17-hydroxy-11-[4-(3-méthane-sulfonyl-phényléthynyl)-phényl]-13-méthyl-17-pentafluoroéthyl-1,2,6,7,8,11,12,13,14,15,16,17-dodécahydro-cyclopenta[*a*]phénanthrén-3-one

➢ 4-méthoxyphényl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ 4-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl}-prop-2-ynyloxycarbonylamino}-benzoate d' éthyle

➢ (4-pipéridin-1-yl-phényl)-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ (8S,11R,13S,14S,17S)-17-hydroxy-11-[4-(4-méthane-sulfonyl-phényléthynyl)-phényl]-13-méthyl-17-pentafluoroéthyl-1,2,6,7,8,11,12,13,14,15,16,17-dodécahydro-cyclopenta[*a*]phénanthrén-3-one

➢ 3-pyridyl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle

➢ tert-butyl-carbamate de 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyle ;

**9.** Procédé pour la préparation des composés de formule générale I, **caractérisé en ce qu'**on convertit un phénylsulfonate de formule générale II,

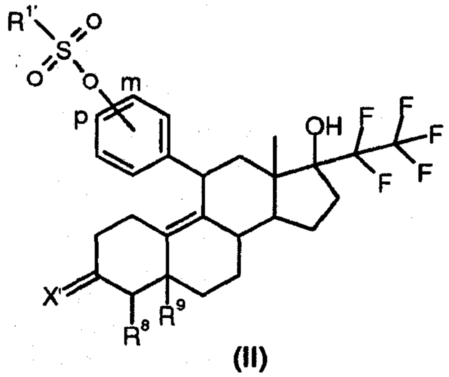

(II)

dans laquelle

$R^{1'}$ représente un groupe alkyle en $C_1$-$C_{10}$ perfluoré,
X' représente un atome d'oxygène, deux groupes alcoxy $OR^7$, un groupe alkylène($C_2$-$C_{10}$)-$\alpha$,$\omega$-dioxy qui peut être à chaîne droite ou ramifié,
$R^7$ représente un groupe alkyle en $C_1$-$C_4$,
$R^8$ représente un atome d'hydrogène,
$R^9$ représente un groupe hydroxy, ou
$R^8$, $R^9$ forment ensemble une liaison
par des réactions de couplage catalysées par le palladium, en un composé de formule générale I'

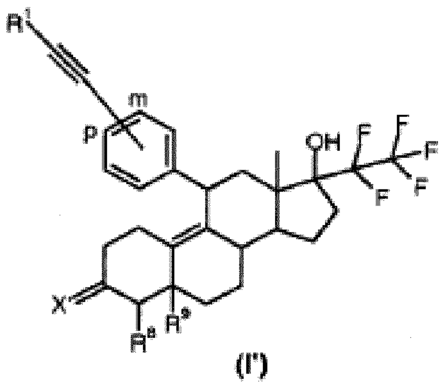

(I')

dans laquelle
$R^1$, X', $R^8$ et $R^9$ ont les significations indiquées plus haut et éventuellement on convertit des fonctionnalités présentes dans $R^1$ et/ou on effectue d'autres réactions ultérieures pour aboutir à des composés de formule générale I' et ensuite on libère à partir du groupe X' un groupe X où X a la signification d'un atome d'oxygène et éventuellement on fonctionnalise davantage ce groupe carbonyle (X = oxygène) et on le convertit en un groupe où X a la signification de $NOR^6$ ou $NNHSO_2R^6$ et/ou dans le cas où $R^8$ représente un atome d'hydrogène et $R^9$ représente un groupe hydroxy, on engendre une double liaison par élimination d'eau et $R^8$ et $R^9$ représentent donc une liaison commune
où
par alkyle il faut entendre des groupes alkyle à chaîne droite ou ramifiée ayant le nombre indiqué d'atomes de carbone ou le cas échéant de 1 à 10 atomes de carbone et qui peuvent être substitués de façon quelconque par perhalogéno, par des groupes hydroxy, des groupes alcoxy en $C_1$-$C_4$ ou des groupes aryle en $C_6$-$C_{12}$ (qui peuvent à leur tour être substitués par 1-3 atomes d'halogène),
par alcényle il faut entendre des groupes alcényle à chaîne droite ou ramifiée ayant de 2 à 10 atomes de carbone et qui peuvent être substitués de façon quelconque par 1-5 atomes d'halogène, groupes hydroxy, groupes alcoxy en $C_1$-$C_3$ ou groupes aryle en $C_6$-$C_{12}$ (qui peuvent à leur tour être substitués par 1-3 atomes d'halogène),
par aryle il faut entendre des radicaux aromatiques, mono- ou bicycliques, comme par exemple phényle ou naphtyle et qui peuvent être substitués de façon quelconque une ou plusieurs fois par halogéno, OH, $SO_2$-alkyle, SO-alkyle et S-alkyle, O-alkyle, $CO_2H$, $CO_2$-alkyle, $NH_2$, $NO_2$, $N_3$, CN, alkyle en $C_1$-$C_{10}$, acyle en $C_1$-$C_{10}$ ou

acyloxy en $C_1$-$C_{10}$,
par aralkyle il faut entendre des groupes aralkyle, aralkyle signifiant des groupes aralkyle qui comportent dans le cycle jusqu'à 14 atomes de carbone et dans la chaîne alkyle de 1 à 8 atomes de carbone et peuvent être substitués de façon quelconque une ou plusieurs fois par halogéno, OH, O-alkyle, $CO_2H$, $CO_2$-alkyle, $NH_2$, NH(alkyle($C_1$-$C_{10}$)), N (alkyle ($C_1$-$C_{10}$))$_2$, $NO_2$, $N_3$, CN, alkyle en $C_1$-$C_{20}$, perfluoroalkyle ($C_1$-$C_{10}$), acyle en $C_1$-$C_{20}$ ou acyloxy($C_1$-$C_{20}$),

**10.** Composés selon l'une quelconque des revendications 1 à 8, qui présentent une action antagoniste des récepteurs de progestérone dans des cellules transfectantes stables de neuroblastome humain.

**11.** Composés selon la revendication 1 (8S,11R,13S,14S,17S)-17-hydroxy-11-[4-(3-hydroxy-prop-1-yn-1-yl)-phényl]-13-méthyl-17-pentafluoroéthyl-1,2,6,7,8,11,12,13,14,15,16,17-dodécahydro-cyclopenta[a]phénanthrén-3-one, acide [4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-propynoïque, (E)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-pent-2-én-4-ynenitrile, (Z)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-pent-2-én-4-ynenitrile, (8S,11R,13S,14S,17S)-17-hydroxy-11-{4-[3-(2-hydroxy-éthoxy)-prop-1-yn-1-yl]-phényl)-13-méthyl-17-pentafluoroéthyl-1,2,6,7,8,11,12,13,14,15,16,17-dodécahydro-cyclopenta[a]phénanthrén-3-one, acide 3-{(E)-4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[a]phénanthrén-11-yl)-phényl]-but-1-én-3-ynyl}-benzoïque, acide 4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoïque, 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoate de méthyle, 1-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-prop-2-ynyl}-3-(4-fluoro-phényl)-urée qui présentent chez diverses espèces (rat, homme) une biodisponibilité orale maximale prévue qui est dans chaque cas supérieure à 50 %.

**12.** Composés selon la revendication 1 (8S,11R,13S,14S,17S)-17-hydroxy-11-[4-(3-hydroxy-prop-1-yn-1-yl)-phényl]-13-méthyl-17-pentafluoroéthyl-1,2,6,7,8,11,12,13,14,15,16,17-dodécahydro-cyclopenta[*a*]phénanthrén-3-one, acide [4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[*a*]phénanthrén-11-yl) phényl]-propynoïque, (E)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-pent-2-én-4-ynenitrile, (Z)-5-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[a]phénanthrén-11-yl)-phényl]-pent-2-én-4-ynenitrile, acide 3-{(E)-4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényl]-but-1-én-3-ynyl}-benzoïque, acide 4-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoïque, 3-[4-((8S,11R,13S,14S,17S)-17-hydroxy-13-méthyl-3-oxo-17-pentafluoroéthyl-2,3,6,7,8,11,12,13,14,15,16,17-dodécahydro-1*H*-cyclopenta[*a*]phénanthrén-11-yl)-phényléthynyl]-benzoate de méthyle qui présentent chez diverses espèces (rat, homme) une biodisponibilité orale maximale prévue qui est dans chaque cas supérieure à 70 %.

**13.** Préparations pharmaceutiques contenant au moins un composé selon l'une quelconque des revendications 1 à 8 ou 10 à 12 ou des mélanges de ceux-ci ainsi que des véhicules pharmaceutiquement acceptables.

**14.** Utilisation des composés selon l'une quelconque des revendications 1 à 8 ou 10 à 12, pour la fabrication d'un médicament.

**15.** Utilisation des composés selon la revendication 1, pour la fabrication d'un médicament destiné au traitement et/ou à la prophylaxie de fibroïdes utérins (myomes, léiomyomes utérins), de l'endométriose, de flux menstruels forts, de méningiomes, de carcinomes mammaires hormonodépendants et de troubles liés à la ménopause, ou à la régulation de la fécondité et la contraception d'urgence.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 057115 A
- WO 9834947 A
- WO 2008058767 A
- WO 9615794 A, Spicer
- WO 9603130 A, Stöckemann
- EP 2009003249 W, Möller
- WO 0147490 A
- WO 06010097 A
- WO 199834947 A

### In der Beschreibung aufgeführte Nicht-Patentliteratur

- **U. FUHRMANN et al.** *J. Med. Chem.,* 2000, vol. 43, 5010-5016
- Remington's Pharmaceutical Science. Mack Publishing Company, 1980
- *Tetrahedron Lett.,* 1985, vol. 26, 2069-2072